(19) Europäisches Patentamt
European Patent Office
Office européen des brevets

(11) **EP 3 629 023 A1**

(12) # EUROPEAN PATENT APPLICATION

(43) Date of publication:
**01.04.2020 Bulletin 2020/14**

(51) Int Cl.:
***G01N 33/569*** (2006.01)     ***C12Q 1/689*** (2018.01)
***G01N 33/574*** (2006.01)

(21) Application number: **18306282.7**

(22) Date of filing: **28.09.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Institut Gustave Roussy
94800 Villejuif (FR)**

(72) Inventors:
• **ZITVOGEL, Laurence
75006 PARIS (FR)**

• **IEBBA, Valerio
00148 ROME (IT)**

(74) Representative: **Santarelli
49, avenue des Champs-Elysées
75008 Paris (FR)**

Remarks:
The complete document including Reference Tables and the Sequence Listing can be downloaded from the EPO website

(54) **MICROBIOTA COMPOSITION, AS A MARKER OF RESPONSIVENESS TO ANTI-PD1/PD-L1/PD-L2 ANTIBODIES IN RENAL CELL CANCER**

(57)     The present invention relates to a method for *in vitro* determining if an individual having a renal cell cancer (RCC) is likely to respond to a treatment with an anti-PD1/PD-L1/PD-L2 Ab-based therapy, based on the analysis of the microbiota present in a stool sample from said individual. Twelve models useful to perform the above method are disclosed, as well as tools designed to easily perform this method.

EP 3 629 023 A1

**Description**

## FIELD OF THE INVENTION

The present invention relates to the field of anticancer treatment. In particular, the present invention concerns the role of the gut microbiota in the efficacy of treatments comprising administration of immune checkpoint inhibitors (ICI), in the treatment of renal cell cancer (RCC). The present invention provides "metagenomics-based gut oncomicrobiome signatures" (GOMS) at diagnosis prior to PD-1/PDL-1/PDL-2 blockade as novel predictors of response or resistance for the best clinical outcome and at 6 months of therapy of a renal cell cancer. The present invention also provides theranostic methods to identify patients in need of a bacterial compensation treatment before receiving an ICI and/or during the therapy with an ICI.

## BACKGROUND AND PRIOR ART

Major conceptual advances in cancer biology have been made over the past decade. The understanding that immune responses are routinely generated against tumor-specific neoantigens expressed by cancer-associated mutations and commonly dampened by the immunosuppressive tumor microenvironment (TME) has been seminal to the development of effective immunotherapies aimed at provoking immune control against tumor progression (Sharma and Allison, 2015a, 2015b). Progress in cancer immunotherapy has resulted in remarkable success in the treatment of a variety of hematological and solid metastatic malignancies such as melanoma, lung, bladder, kidney, Hodgkin's lymphoma, acute B cell leukemia, liver, Merkel-cell carcinoma and head and neck tumors, amongst others (Borghaei *et al.,* 2015; Nghiem *et al.,* 2016; Robert *et al.,* 2011; Rosenberg *et al.,* 2016). To date, therapies that block inhibitory signaling pathways expressed by T lymphocytes (so called "immune checkpoints") during the initial priming phase (in draining lymph nodes) or the effector phases (in tumor beds) of adaptive anticancer immune responses have demonstrated the greatest clinical benefit in overall survival (Ribas *et al.,* 2016; Topalian *et al.,* 2012). The prototypic example for this success has been the use of monoclonal antibodies (mAbs) targeting PD-1 (expressed by activated/exhausted T cells) or its ligand PD-L1 (commonly expressed by cancer cells or cells of the TME) (Pardoll, 2015). By releasing these molecular brakes, such mAbs reinstate the anticancer adaptive arm of the immune response.

While PD-1 blockade represents the most effective first line therapy in B-RAF wild type melanoma and the best option in second line unresectable lung carcinomas, approximately 60-70% of tumors exhibit primary resistance to this therapeutic strategy. Primary resistance has been attributed to low tumor mutational burden and poor intrinsic antigenicity of tumor cells (Riaz *et al.,* 2016; Rizvi et I., 2015), defective antigen presentation and priming phase (Spranger *et al.,* 2015), limited intratumoral infiltration related to exhausted T cell functions (Smyth *et al.,* 2016), and metabolic immunosuppressive pathways related to adenosine and indoleamine, 2, 3-dioxygenase (Koyama *et al.,* 2016; Smyth *et al.,* 2016). Similarly, primary resistance mechanisms to CTLA-4 blockade have also been elucidated. For example, melanoma presenting with loss of IFN-γ signaling lack response to ipilimumab (Gao *et al.,* 2016).

More recently, secondary resistance mechanisms to chronic inhibition of PD-1 receptors have been reported in approximately 25% of melanoma patients (Koyama *et al.,* 2016; Ribas *et al.,* 2016; Zaretsky *et al.,* 2016). Four out of 74 melanoma patients who progressed for a median follow up of 1.8 years despite continuous therapy with pembrolizumab developed lesions presenting with loss-of-function mutations in janus kinases JAK1 or JAK2, thus leading to decreased STAT1 phosphorylation and reduced sensitivity to the antiproliferative effects of IFNs or alternatively, a mutation within the gene encoding β-2 microglobulin, preventing folding and transport of MHC class I molecules to the cell surface for T cell recognition of tumor cells (Zaretsky *et al.,* 2016). In addition to these darwinian natural selection of genetic (or epigenetic) heritable traits, other acquired resistance mechanisms have been reported in mice. Predominately, adaptive immune resistance resulting from the IFN-γ-inducible expression of PD-L1 (Pardoll, 2012; Taube *et al.,* 2012), a primary ligand of PD-1, TNF-induced loss of antigenic variants (Landsberg *et al.,* 2012) as well as TCR-dependent upregulation of additional exhaustion markers on activated T lymphocytes such as Tim3/HAVCR2 (Koyama *et al.,* 2016; Restifo *et al.,* 2012; Smyth *et al.,* 2016), lymphocyte activation gene 3 (Lag3), T cell immunoreceptor with Ig and ITIM domains (TIGIT), B and T cell lymphocyte attenuator (BTLA), and V-domain Ig suppressor of T cell activation (VISTA).

The inventors' team just reported in Routy et al. Science Jan 5, 2018, in conjunction with two other reports (Gopalakrishnan V *et al* and Matson V *et al*), that primary resistance to PD-1/PDL-1-based immune checkpoint inhibitors (ICI) can be due to an abnormal gut microbiota. Antibiotics (ATB) inhibited the clinical benefit of ICI in advanced lung, kidney and bladder cancer patients. Fecal microbiota transplantation (FMT) from cancer patients who responded to ICI (but not from non-responding patients) into germ-free or ATB-treated mice ameliorated the antitumor effects of PD-1 blockade. Metagenomics of patient stools at diagnosis revealed correlations between clinical responses to ICI and dedicated microbial patterns, with relative increase of *Akkermansia muciniphila* when examining both lung and kidney cancer patients altogether. The diagnosis of a gut dysbiosis is important since it is amenable to a therapy that restores efficacy of ICI. Indeed, oral supplementation with *A. muciniphila* or *Alistipes indistinctus* post-FMT with non-responder feces restored the efficacy of PD-1 blockade in an IL-12-dependent manner, by increasing the recruitment of CCR9+CXCR3+CD4+ T lymphocytes into tumor beds.

The results disclosed in the present application show the predictive value of metagenomics-based gut oncomicrobiome signatures (GOMS), calculated at diagnosis from data of a larger cohort of 69 renal cell cancer patients (RCC) patients who received a second line therapy with nivolumab or pembrolizumab for a tyrosine kinase inhibitor or mTOR inhibitor-resistant advanced or metastatic RCC, for the clinical benefit of such a treatment. This study was performed using three RECIST criteria (best outcome, time to progression (TTP) at 3 months, TTP at 6 months), including or excluding 11 RCC patients who took antibiotics during or the 2 months preceeding the first administration of anti-PD1 Abs.

**SUMMARY OF THE INVENTION**

According to a first aspect, the present invention pertains to a method for *in vitro* determining if an individual having a renal cell cancer patients (RCC) is likely to respond to a treatment with an anti-PD1/PD-L1/PD-L2 Ab-based therapy, comprising the following steps:

(i) from a fecal sample (or ileal or colonic mucosal specimen) of said individual, obtaining an abundances pattern based on the relative abundances of a set of bacterial species comprising at least 8 bacterial species selected from the group of species disclosed in Table 1 below.
(ii) using one or several pre-defined equations each corresponding to a model obtained for at least 8 bacterial species from said set of bacterial species, calculating the probability that said individual responds to the treatment ($P_R$) or the probability that said individual resists to the treatment ($P_{NR}$) with an anti-PD1/PD-L1/PD-L2 Ab-based therapy.

The invention also pertains to 12 models useful to perform the above method, 6 of which were obtained from data of patients who did not take any antibiotics during the last two months, whereas the 6 others can be used in patients whom recent antibiotics uptake is unknown. These models rely on (partly) different subsets of the microbiota (listed in Tables 3, 6, 9 and 12 below) and can be used either alone or combined.

Tools designed to easily perform the above method are also part of the present invention, such as a nucleic acid microarray comprising nucleic acid probes specific for each of the microorganism species to be detected in step (i) of the method, and such as a set of primers comprising primer pairs for amplifying sequences specific for each of the microorganism species to be detected in step (i) of said method.

Theranostic methods for determining whether an individual needs a bacterial composition with a bacterial composition and/or by FMT before receiving an anti-PD1/PD-L1 Ab-based therapy are also part of the invention.

**BRIEF DESCRIPTION OF THE DRAWINGS**

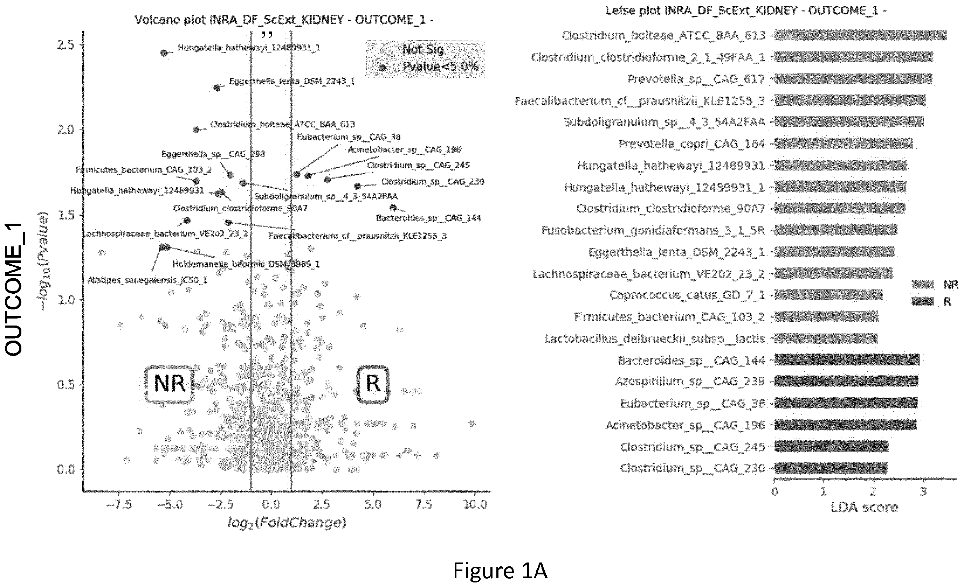

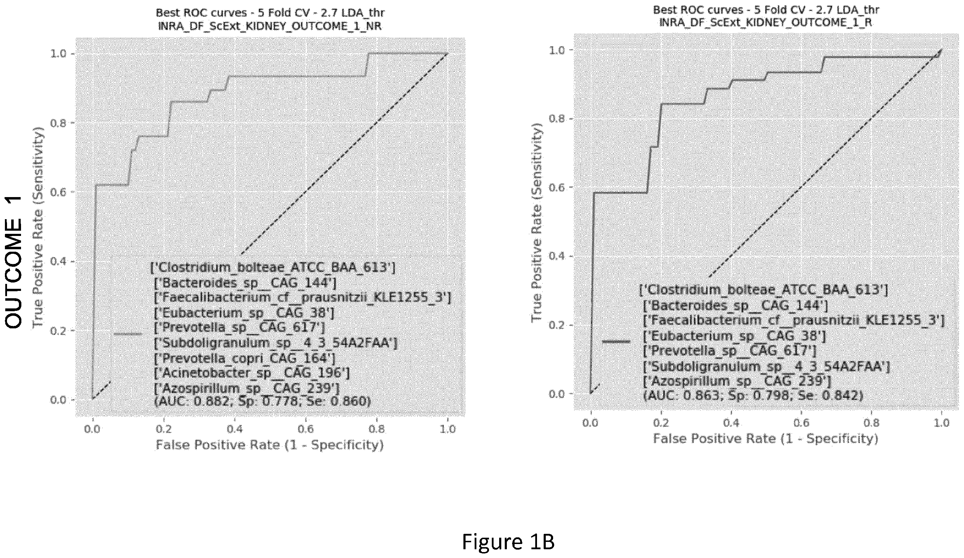

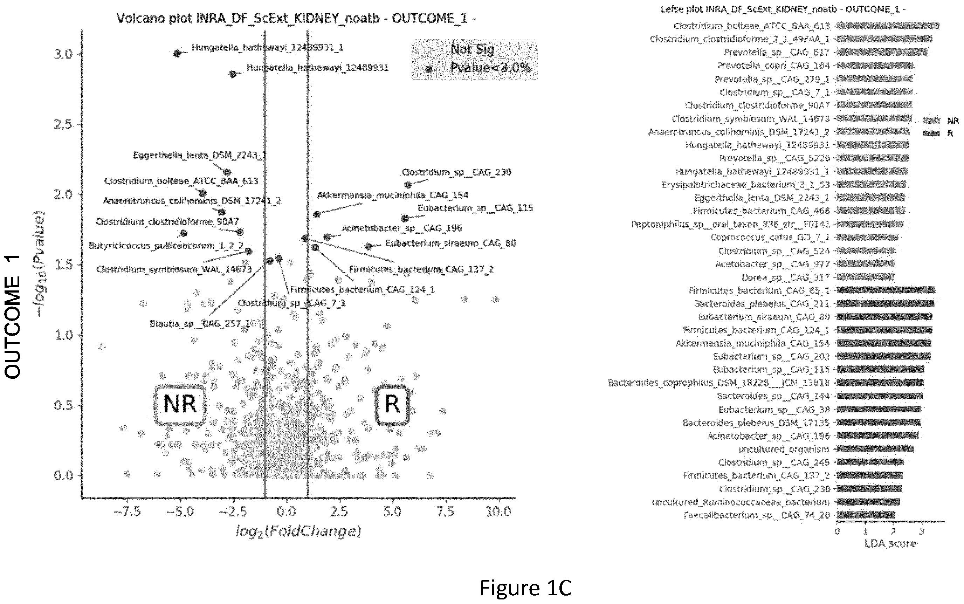

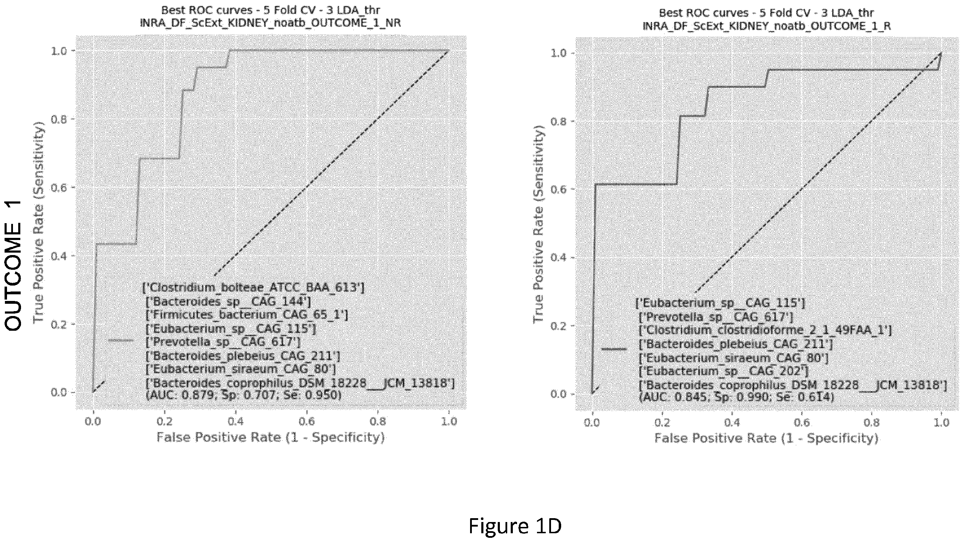

**Figure 1. Commensal species associated with best outcome.**
The RECIST 1.1 criterium taken into account as predictive for clinical benefit was "best clinical outcome".

A. Volcano plots (left) and Linear discriminant analysis effect size (LEfSe) (right) analysis to assess putative bacterial biomarkers for building metagenomics-based GOMS in RCC patients' stools regardless antibiotic usage. Volcano plots were generated computing for each bacterial species: i) the log2 of fold ratio (FR) among the mean relative abundances of R versus NR (*x* axis); ii) the co-log10 of P values deriving from Mann-Whitney U test calculated on relative abundances in absolute values (*y* axis). LEfSe plots were generated with Python 2.7 on output files derived from LEfSe pipeline, and all species with LDA score $\geq 2$ were considered for subsequent analysis.
B. ROC curves to assess predictability of metagenomic-based GOMS in RCC patients' stools regardless antibiotic usage. Patients were divided into NR and R according to their « resistant » or « responding » clinical phenotype upon ICI treatment. Combinations of selected bacterial species in panel A were performed with Python 2.7 and underwent ROC analysis. Upon 5-fold cross-validation (no noise added), the ROC curve corresponding to the bacterial species consortium having the best AUC was depicted. Specificity (*x* axis) and sensitivity (*y* axis) along with their best values were reported for each curve (inset). Diagonal line depicts the absence of predictability for the best clinical outcome.

C-D. Idem as A-B but considering only patients who did not take ATB.

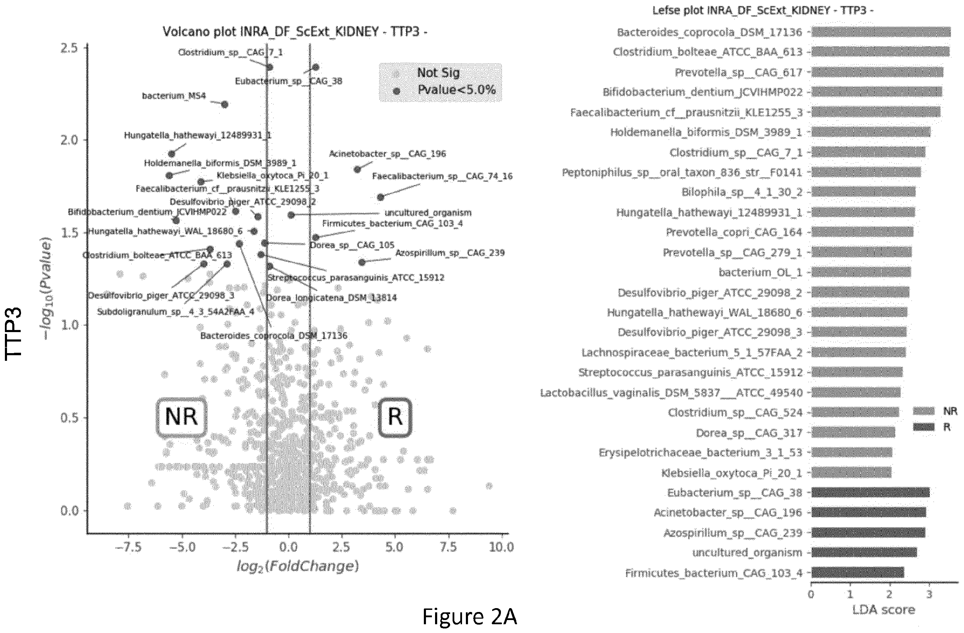

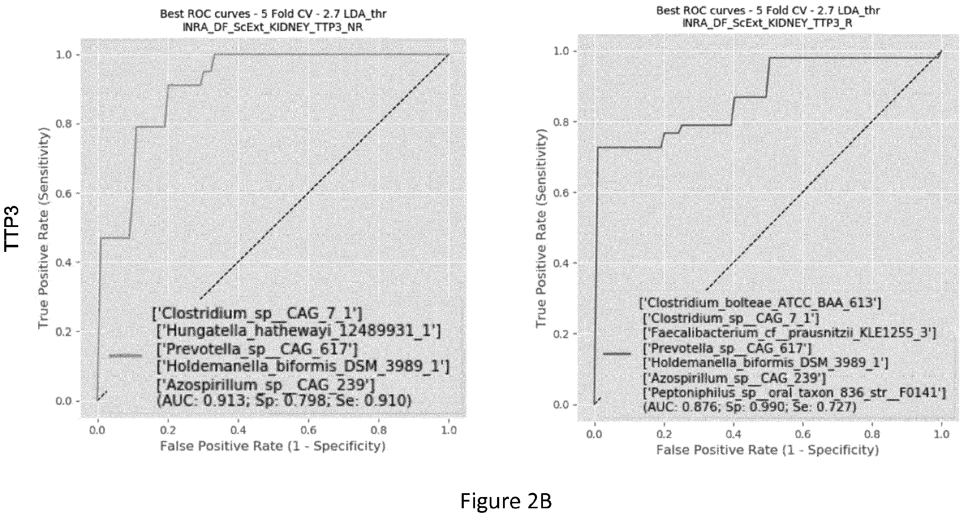

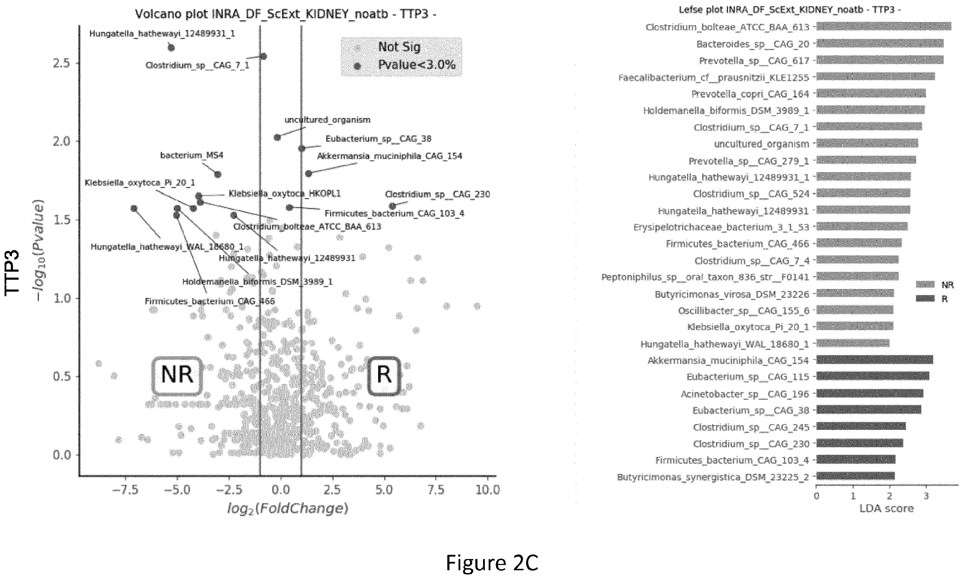

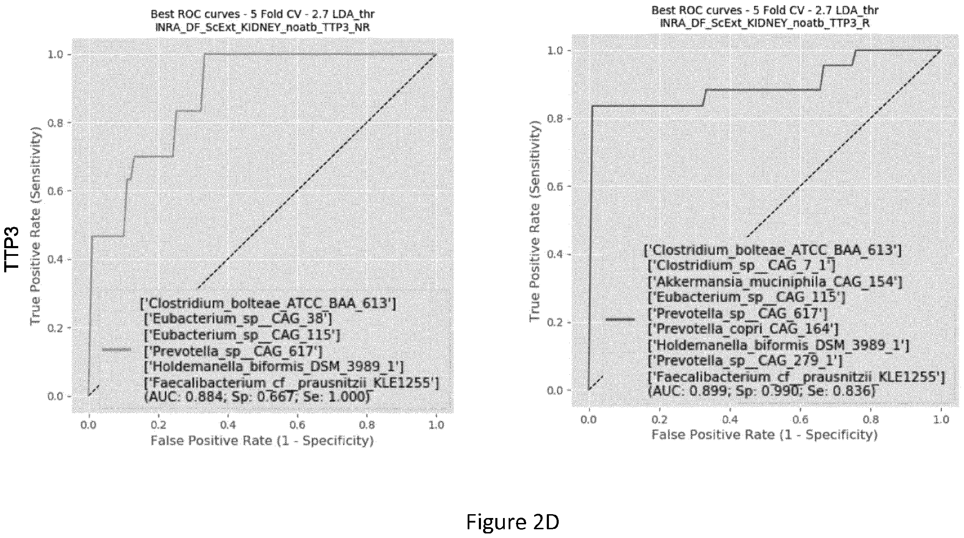

**Figure 2. Commensal species associated with TTP>3 months.**
The RECIST 1.1 criterium taken into account as predictive for clinical benefit was "TTP< or > 3 months".

A. Volcano plots (left) and LEfSe (right) analysis to assess putative bacterial biomarkers for building metagenomics-based GOMS in RCC patients' stools regardless antibiotic usage. Volcano plots were generated computing for each bacterial species: i) the log2 of fold ratio (FR) among the mean relative abundances of R versus NR (*x* axis); ii) the co-log10 of P values deriving from Mann-Whitney U test calculated on relative abundances in absolute values (*y* axis). LEfSe plots were generated with Python 2.7 on output files derived from LEfSe pipeline, and all species with LDA score $\geq$ 2 were considered for subsequent analysis.

B. ROC curves to assess predictability of metagenomic-based GOMS in RCC patients' stools regardless antibiotic usage. Patients were divided into NR and R according to their « resistant » or « responding » clinical phenotype upon ICI treatment. Combinations of selected bacterial species in panel A were performed with Python 2.7 and underwent ROC analysis. Upon 5-fold cross-validation (no noise added), the ROC curve corresponding to the bacterial species consortium having the best AUC was depicted. Specificity (*x* axis) and sensitivity (*y* axis) along with their best values were reported for each curve (inset). Diagonal line depicts the absence of predictability for the best clinical outcome.

C-D. Idem as A-B but considering only patients who did not take ATB.

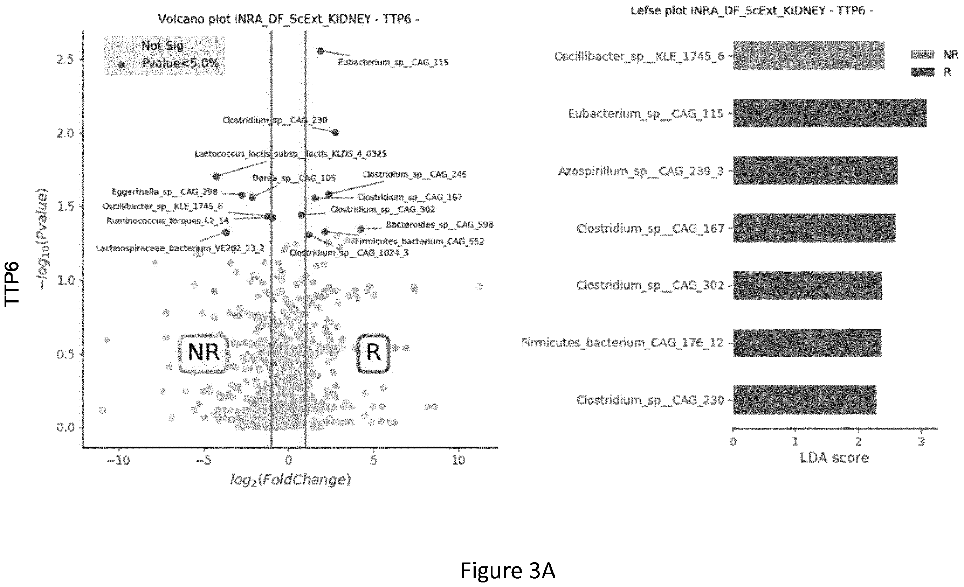

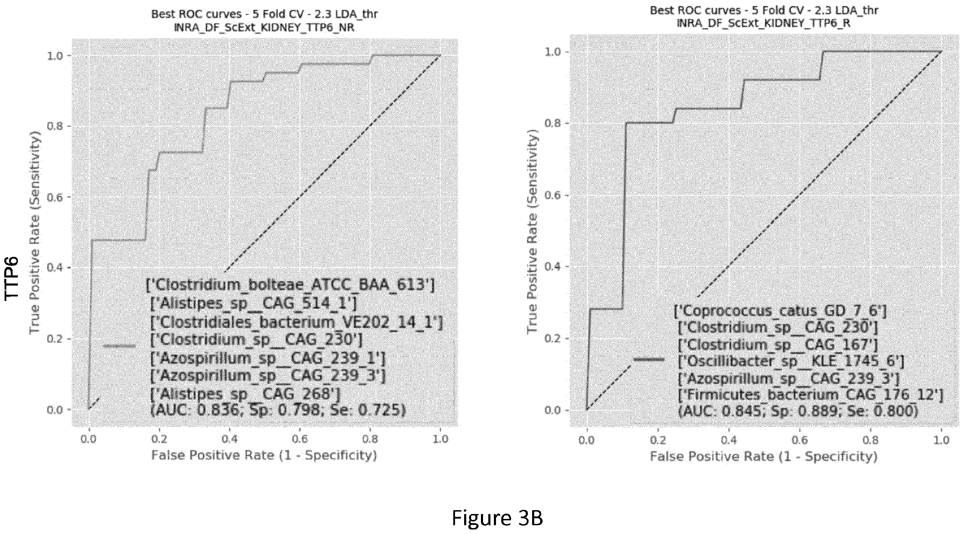

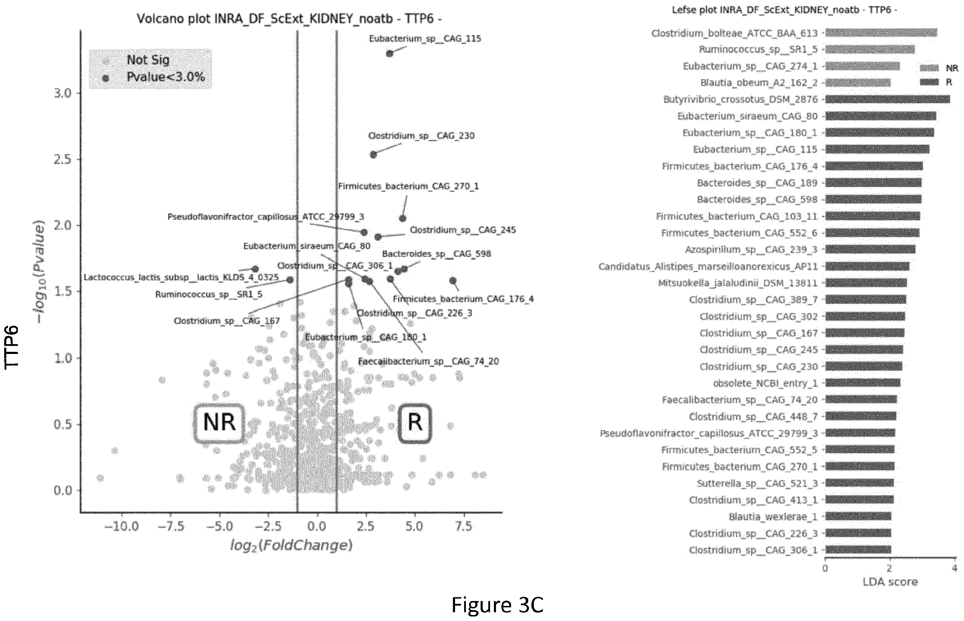

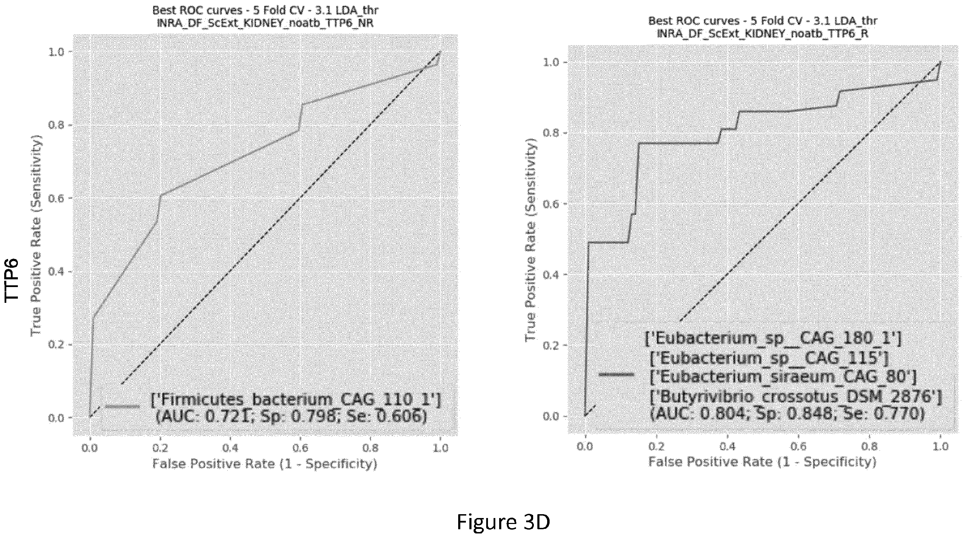

**Figure 3. Commensal species associated with TTP>6 months.**

The RECIST 1.1 criterium taken into account as predictive for clinical benefit was "TTP< or >6 months".

A. Volcano plots (left) and LEfSe (right) analysis to assess putative bacterial biomarkers for building metagenomics-based GOMS in RCC patients' stools regardless antibiotic usage. Volcano plots were generated computing for each bacterial species: i) the log2 of fold ratio (FR) among the mean relative abundances of R versus NR (*x* axis); ii) the co-log10 of P values deriving from Mann-Whitney U test calculated on relative abundances in absolute values (*y* axis). LEfSe plots were generated with Python 2.7 on output files derived from LEfSe pipeline, and all species with LDA score $\geq$ 2 were considered for subsequent analysis.

B. ROC curves to assess predictability of metagenomic-based GOMS in RCC patients' stools regardless antibiotic usage. Patients were divided into NR and R according to their « resistant » or « responding » clinical phenotype upon ICI treatment. Combinations of selected bacterial species in panel A were performed with Python 2.7 and underwent ROC analysis. Upon 5-fold cross-validation (no noise added), the ROC curve corresponding to the bacterial species consortium having the best AUC was depicted. Specificity (*x* axis) and sensitivity (*y* axis) along with their best values were reported for each curve (inset). Diagonal line depicts the absence of predictability for the best clinical outcome.

C-D. Idem as A-B but considering only patients who did not take ATB.

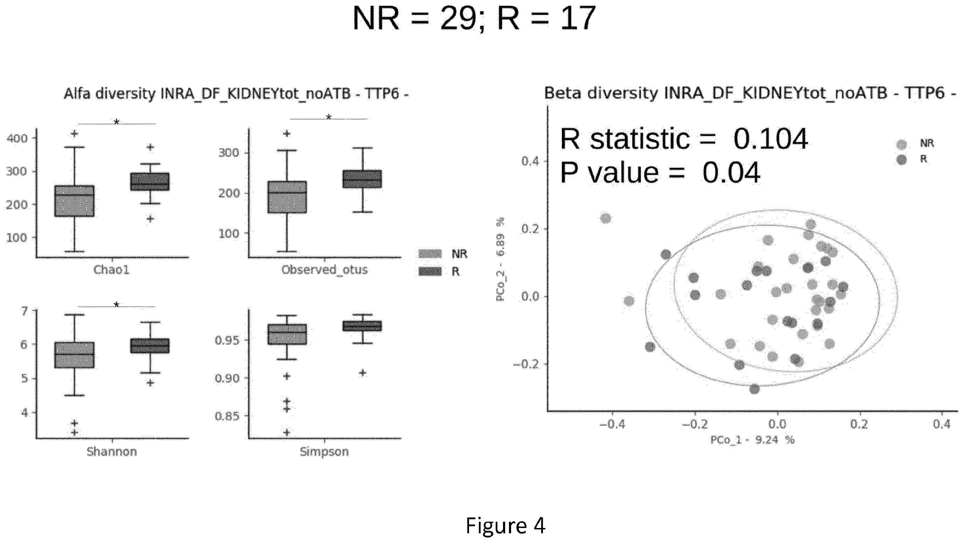

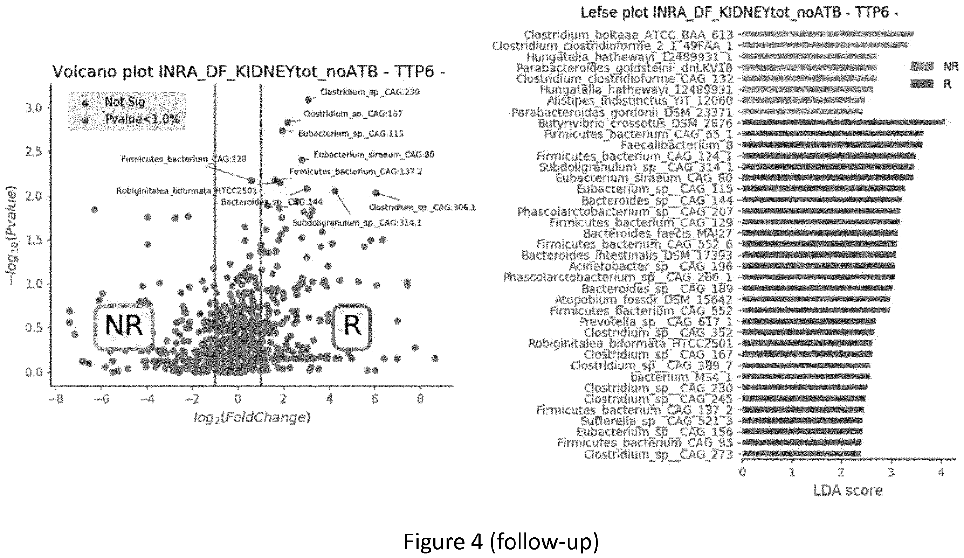

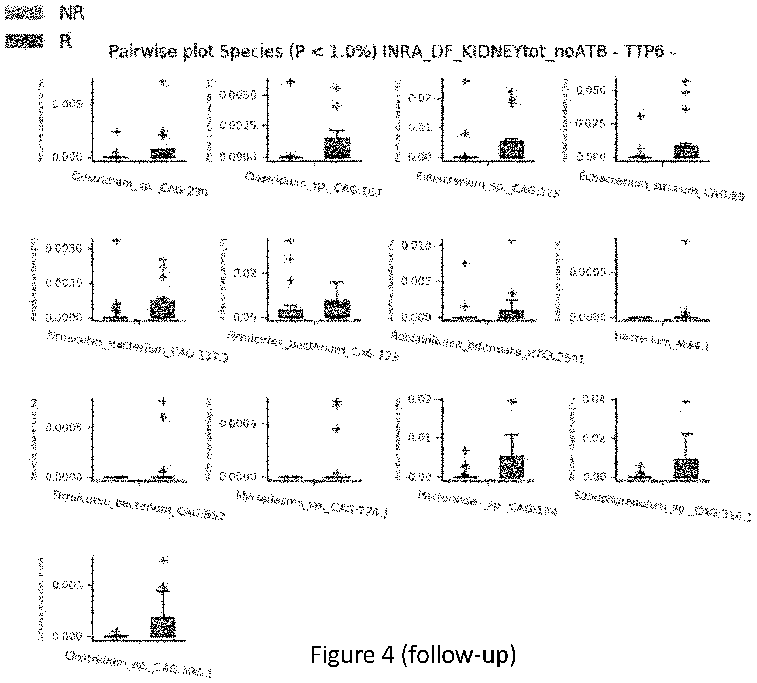

**Figure 4. TTP6 months allows to segregate R versus NR RCC patients who did not take ATB based on MG composition.**

All RCC patients who did not take ATB from the Routy et al. Science 2018 data base were considered unifying discovery and validation cohorts at TTP6. Alfa-diversity, beta-diversity, Volcano and LEfSe plots were generated as described in Figures 1, 2, 3 and 4. Pairwise analysis was performed on NR (orange) and R (blue) cohorts and reported are boxplots generated with Python v2.7 only for significant comparisons (Mann-Whitney U test, $P \leq 0.05$). Responders tend to have significantly higher alfa-diversity compared to NR, and are significantly divided from NR (beta-diversity, *P* values are indicated within the ordination plots). LEfSe, volcano and pairwise analysis plots highlights discriminant species within the two cohorts NR and R.

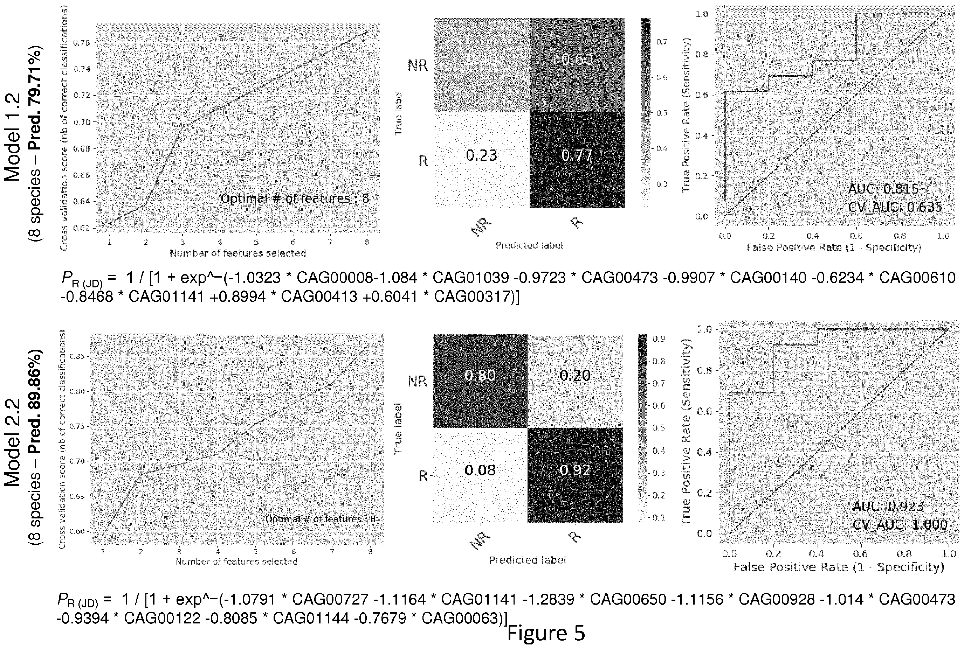

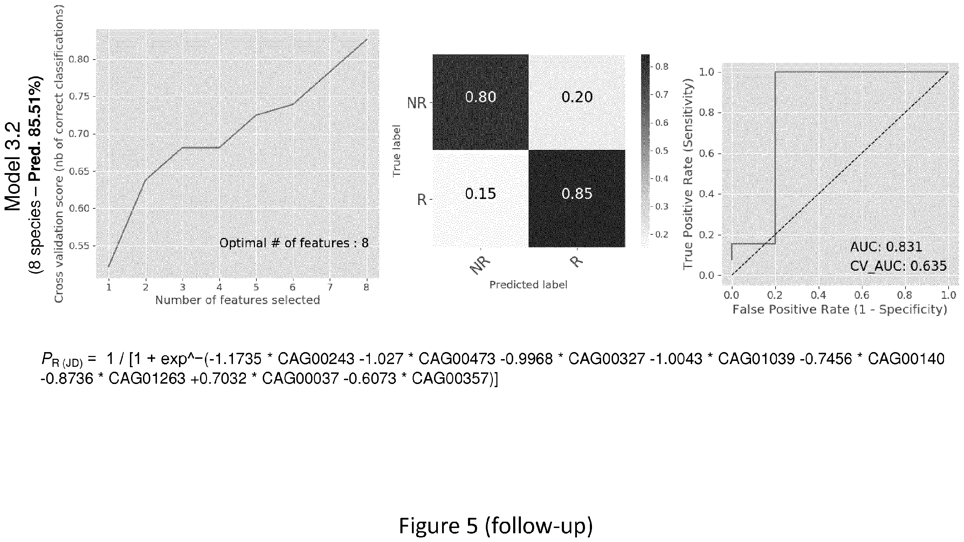

**Figure 5. Logistic regression models based on RCC patients regardless antibiotic usage and OUTCOME_1 criterion (OUTCOME_1 meaning considering all SD among responders).**

Logistic regression models were drawn from stool MG of RCC patients regardless antibiotic usage and OUTCOME_1 criterion. Each Model is depicted by three graphs: a feature selection (left), a confusion matrix (center) and a ROC curve (right). Confusion matrices and ROC curves were generated after 5-fold cross-validation. The higher the percentage within the crossing 'True label' and 'Predicted label' cells, the higher the model predictability. For the ROC curves, both AUC (area under curve) and CV_AUC (cross-validated area under curve) need to be taken into account. At the bottom of each triplet is reported the Model equation described by a logistic regression: *exp* is the exponent with natural base, *CAG* is the relative abundance of each CAG species expressed within the closed interval [0 : 1] and standardized (zero mean and unit variance).

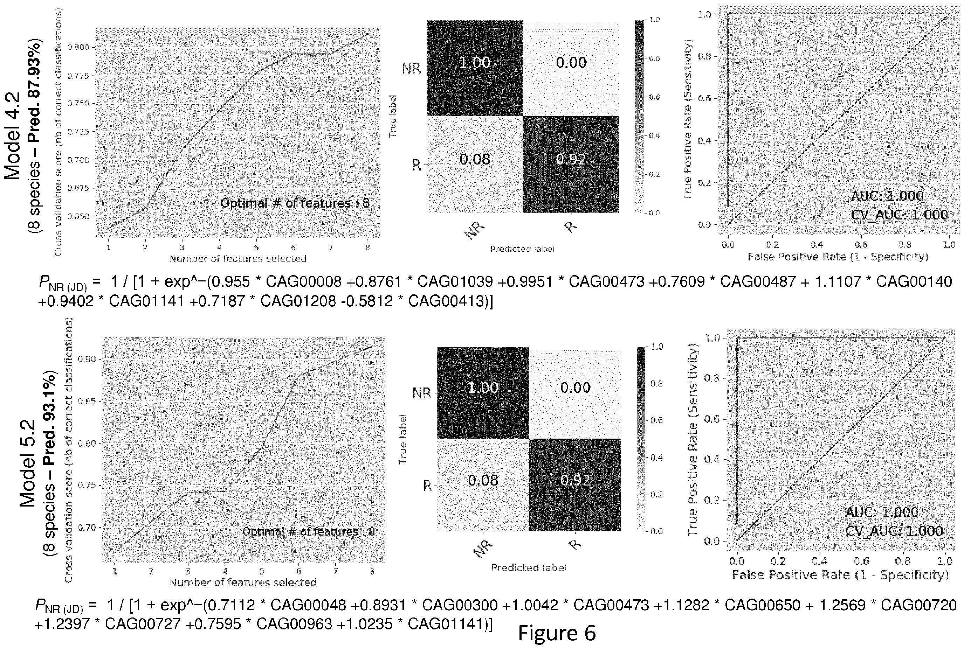

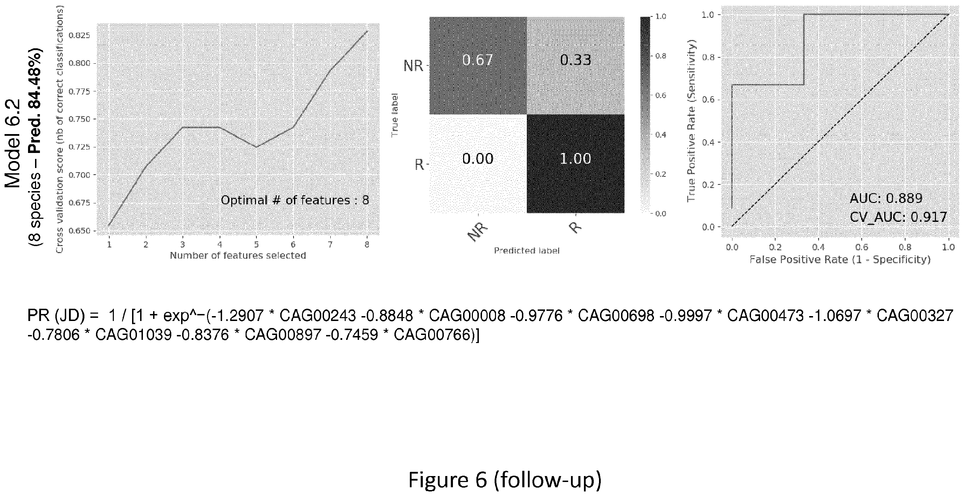

**Figure 6. Logistic regression models based on RCC patients without antibiotic treatment and OUTCOME_1 criterion (OUTCOME_1 meaning considering all SD among responders).**

Logistic regression models were drawn from stool MG of RCC patients with no antibiotic usage and OUTCOME_1 criterion. Triplet graph (feature selection, confusion matrix, ROC curve) description as in Figure 6.

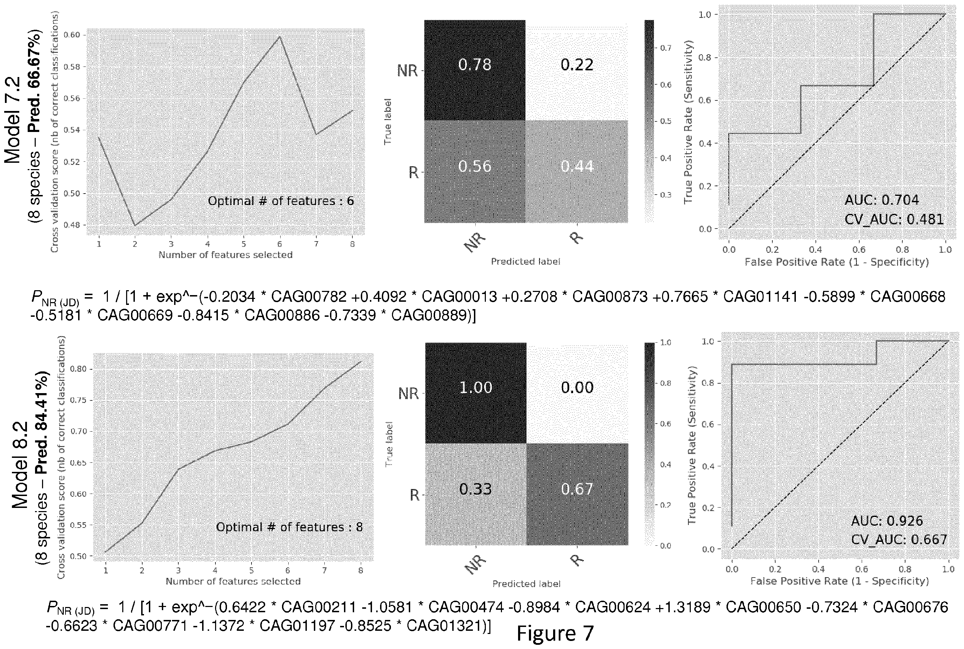

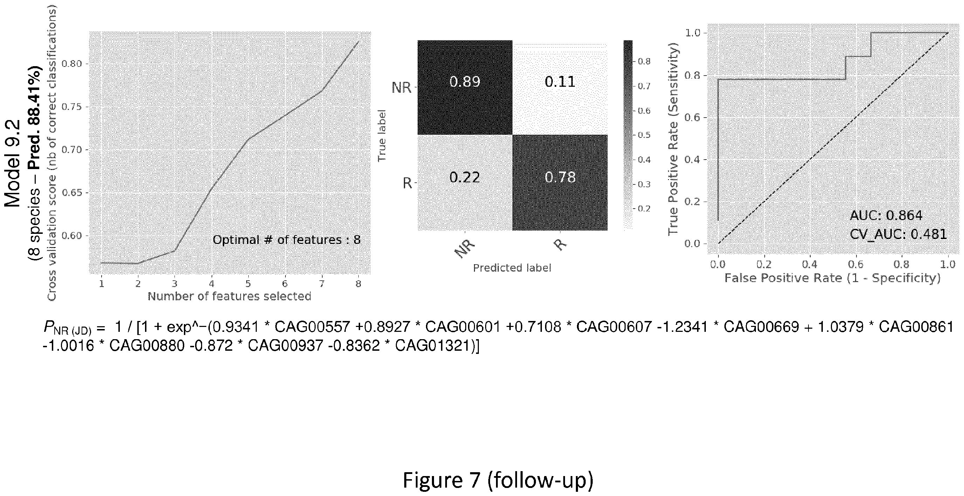

**Figure 7. Logistic regression models based on RCC patients regardless antibiotic usage and OUTCOME_2 criterion (OUTCOME_2 meaning considering only SD>6 months among responders).**

Logistic regression models were drawn from stool MG of RCC patients regardless antibiotic usage and OUTCOME_2 criterion. Triplet graph (feature selection, confusion matrix, ROC curve) description as in Figure 6.

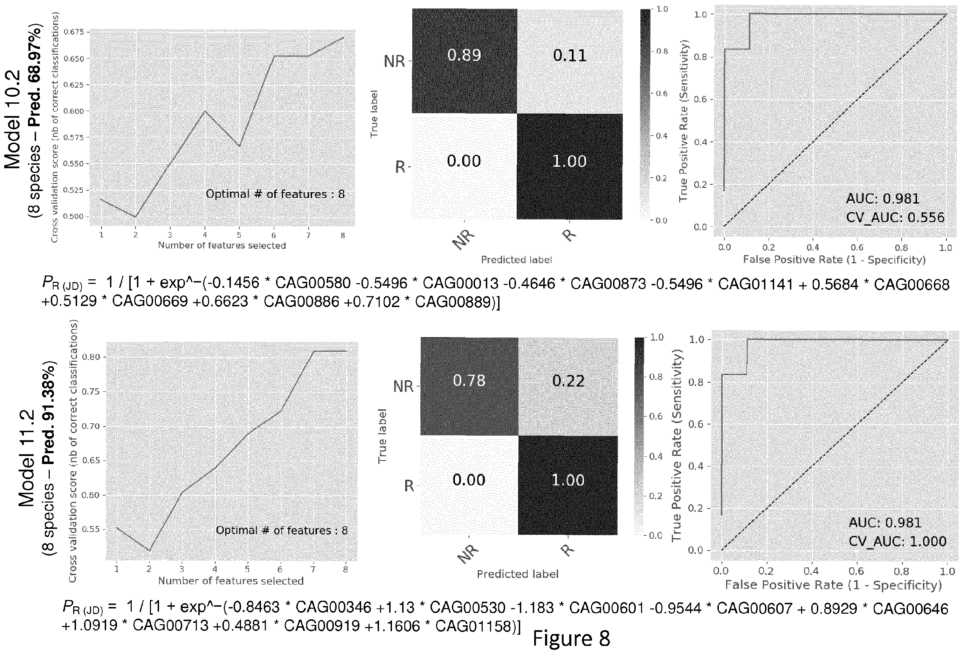

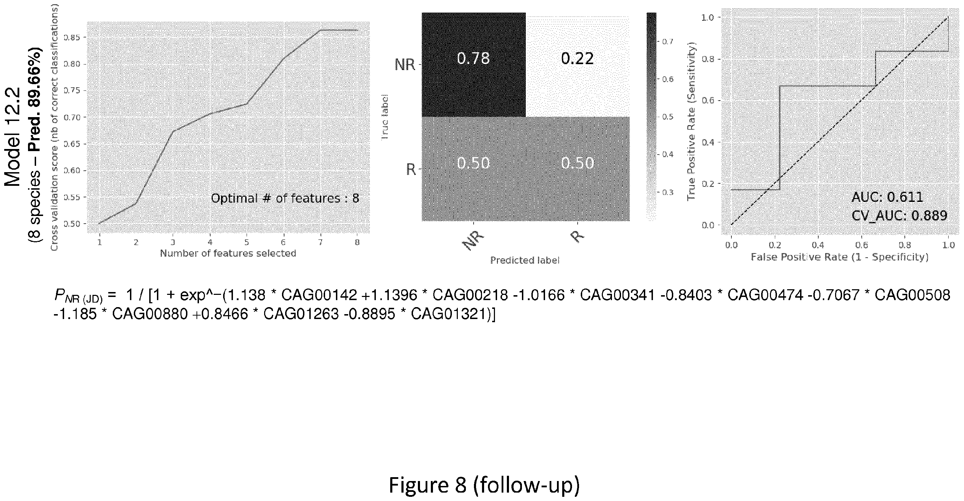

**Figure 8. Logistic regression models based on RCC patients without antibiotic treatment and OUTCOME_2 criterion (OUTCOME_2 meaning considering only SD>6 months among responders).**

Logistic regression models were drawn from stool MG of RCC patients with no antibiotic usage and OUTCOME_2 criterion. Triplet graph (feature selection, confusion matrix, ROC curve) description as in Figure 6.

## DETAILED DESCRIPTION OF THE PREFERRED EMBODIMENTS

In the present text, the following definitions are used:

- An "immune checkpoint inhibitor" (ICI) designates any drug, molecule or composition which blocks certain proteins made by some types of immune system cells, such as T cells, and some cancer cells. These proteins help keep immune responses in check and can keep T cells from killing cancer cells. When these proteins are blocked, the "brakes" on the immune system are released and T cells are able to kill cancer cells better. Examples of checkpoint proteins found on T cells or cancer cells include PD-1/PD-L1 and CTLA-4/B7-1/B7-2. In particular, ICIs encompass anti-PD1 antibodies (such as Nivolumab or Pembrolizumab), anti-PD-L1 antibodies (such as Atezolizumab or Durvalumab), anti-CTLA-4 antibodies and anti-PD-L2 antibodies. In the scientific literature, ICIs are also designated as "drugs blocking an immune checkpoint", or "immune checkpoint blockers" or "immune checkpoint blockade drugs".
- An "anti-PD1/PD-L1/PD-L2 Ab-based therapy" herein designates any therapy including the use of a drug that antagonizes PD1, PD-L1 or PD-L2. These include therapies mainly based on an ICI such as a drug antagonizing PD1 or PD-L1 or PD-L2, as well as combined therapies using several ICIs and/or additional anticancer drugs such as chemotherapeutic drugs. Non-limitative examples of combined therapies encompassed by the phrase "anti-PD1/PD-L1/PD-L2 Ab-based therapy" include anti-PD1 + anti-CTLA4, anti-PD1 + polychemotherapy (pemetrexed+ carboplatin), anti-Lag3 + anti-PD1, anti-NKG2A + anti-PD1, IDO inhibitor + anti-PD1 and anti-ICOS+anti-PD1. Although the currently used drugs antagonizing immune checkpoint proteins are monoclonal antibodies, other molecules specifically binding to PD1, PD-L1, PD-L2 or other proteins could be used for the development of future ICIs such as, for example, antibody fragments or specifically designed aptamers. Of course, the phrase "anti-PD1/PD-L1/PD-L2 Ab-based therapy" encompasses any therapy including an active molecule that antagonizes PD1 or PD-L1 or PD-L2.
- "NR" defines a non-responder status to PD-1/PDL-1/PDL-2 blockade
- "R" defines a responder status to PD-1/PDL-1/PDL-2 blockade
- "NGS" defines any Next Generation Sequencing platform available in the past, present or in the future.
- In the present text, each "bacterial species" is defined by a Co-Abundance gene Group ("CAG"), which is a group of bacterial genes from the gut microbiome (*i.e.,* the gene repertoire of the gut microbiota), which abundance level varies in the same proportion among different individual samples. In other words, a bacterial species according to the invention is defined by a cluster of bacterial gene sequences which abundance levels in samples from distinct subjects are statistically linked rather than being randomly distributed.

Most current approaches for analyzing metagenomic data rely on comparisons to reference genomes, but the human gut microbiota diversity extends beyond what is currently covered by reference databases. In the results disclosed herein, the inventors used a method based on binning co-abundant genes across a series of metagenomic samples, that enables comprehensive discovery of new microorganisms without the need for reference sequences. In what follows, part of the species identified as likely to play a role in the patients' response to therapies based on antibodies against PD1, PD-L1 or PD-L2 are newly-identified species, not yet precisely referenced in public databases. For each of the identified species (both newly-identified and species very close to already referenced species), the present application discloses a set of 50 bacterial genes which are non-redundant sequences and can be used, alone or in combination, as tracer genes to assess the presence and relative abundance to the corresponding species. Of course, once the species are identified, either by the set of non-redundant genes disclosed herein, or later on by their further identification and/or inclusion into a data base, the skilled in the art can assess their relative abundance by any appropriate means, such as, for example, by measuring the copy number of another non-redundant gene that co-varies with the 50 sequences disclosed in the present application, or even by identifying a signature of this species at the protein level rather than in a nucleic acids sample. Hence, the present invention is not limited to the use of the disclosed sequences to measure the relative abundance of the corresponding species.

- The "relative abundance" of a definite bacterial is defined as the fraction of the entire bacterial ecosystem belonging to this bacterial species. Throughout the present text, all relative abundances are expressed within the closed interval [0 : 1], where 1 stands for the maximum fraction available for a single bacterial species (*i.e.,* a bacterial species with a relative abundance equal to 1 means that 100% of the bacteria present in the sample are of the considered species). For NGS technique, the relative abundance of a bacterial species is considered as the number of reads of that selected species divided by the total number of reads representing the overall bacterial community. For the qPCR technique, the relative abundance of a bacterial species is considered as the ΔCt value of that species X (amplified by a pair of primers specific for X) divided by the ΔCt value of the total bacteria (amplified by an universal primers pair able to catch all the eubacteria present in a sample, the pair consisting of primers PRK341F and PRK806R or the pair consisting of primers 27F and 1492R).

- "predictability" represents the predictability of the overall model done with logistic regression, based on selected bacterial species. Predictability is expressed as a percentage value within the closed interval [0 : 100].
- "success rate" is the percentage of subjects recognized as NR or R after 5-fold cross-validation, and is different from model predictability.

When necessary, other definitions are provided later in the present text.

The present invention concerns a method for *in vitro* determining if a subject having a renal cell cancer is likely to benefit from a cancer treatment with an ICI and more specifically, from a treatment with antibodies (or other inhibiting molecules) directed against immune checkpoint blockers PD1, PD-L1 or PD-L2, alone or together with CTLA4 and/or other drugs as defined above. The responder (NR) or responder (R) status is established following these steps:

(i) from a fecal sample of the said subject (or from an ileal or colonic mucosal specimen), obtaining an "abundances pattern" based on the relative abundances of a set of bacterial species, expressed within the closed interval [0 : 1];
(ii) using the obtained abundances pattern to calculate, using one (or several) of the complementary models proposed in the present application, the probability that the subject will not respond (NR) or respond (R) to the treatment.

It is important to note here that, as already mentioned above, the present invention is not limited by the technique used to measure the relative abundances of the bacterial species, which can be obtained by NGS (through any past or future NGS platform, from the first generation to the last available on the market and those in development, using any NGS output file provided as fastq, BAM, SAM, or other kind of files extensions) or any other technique such as, for example, qPCR (quantitative polymerase chain reaction) and microarrays to express the relative abundances of selected bacterial species, using the sequences provided herein or any other co-variant sequence. When the relative abundances are assessed by genetic analysis, the data preferably derive from shotgun sequencing, and not 16S targeted sequencing, in order to comply with the bioinformatic pipeline described in Materials and Methods below.

According to a first aspect, the present invention pertains to a method for *in vitro* determining if an individual having a renal cell cancer (RCC) is likely to respond to a treatment with an ICI-based therapy such as an anti-PD1/PD-L1/PD-L2 Ab-based therapy, comprising the following steps:

(i) from a fecal sample of said individual, obtaining an abundances pattern based on the relative abundances of a set of bacterial species comprising at least 8 bacterial species selected from the group consisting of:

**Table 1:** Bacterial species used to assess the R or NR status of a RCC patient.

| CAG number | Bacterial species |
|---|---|
| CAG00008 | Clostridium_bolteae_ATCC_BAA_613 |
| CAG00013 | Clostridiales_bacterium_1_7_47FAA |
| CAG00037 | Bacteroides_faecis_MAJ27 |
| CAG00048_1 | Clostridium_sp__CAG_226 |
| CAG00063 | Barnesiella_viscericola_DSM_18177 |
| CAG00122 | Coprococcus_catus_GD_7 |
| CAG00140 | Subdoligranulum_sp__4_3_54A2FAA |
| CAG00142 | Bacteroides_stercoris_ATCC_43183 |
| CAG00211 | Firmicutes_bacterium_CAG_227 |
| CAG00218 | Bacteroides_sp__CAG_20 |
| CAG00243 | Lachnospiraceae_bacterium_1_1_57FAA |
| CAG00300 | Prevotella_sp__CAG_891 |

(continued)

| CAG number | Bacterial species |
|---|---|
| CAG00317 | Clostridium_sp__CAG_230 |
| CAG00327 | Faecalibacterium_sp__CAG_74 |
| CAG00341 | Eubacterium_sp__CAG_115 |
| CAG00346 | Eubacterium_rectale_M104_1 |
| CAG00357 | Bacteroides_ovatus_V975 |
| CAG00413 | Bacteroides_sp__CAG_144 |
| CAG00473 | Prevotella_sp__CAG_617 |
| CAG00474 | Sutterella_wadsworthensis_2_1_59BFAA |
| CAG00487 | Prevotella_sp__CAG_279 |
| CAG00508 | Alistipes_obesi |
| CAG00530 | Prevotella_sp__CAG_617 |
| CAG00557 | Ruminococcus_callidus_ATCC_27760 |
| CAG00580 | Clostridium_sp__CAG_62 |
| CAG00601 | uncultured_Faecalibacterium_sp_ |
| CAG00607 | Eubacterium_sp__CAG_251 |
| CAG00610 | Hungatella_hathewayi_12489931 |
| CAG00624 | Firmicutes_bacterium_CAG_552 |
| CAG00646 | Alistipes_sp__CAG_268 |
| CAG00650 | Dorea_formicigenerans_ATCC_27755 |
| CAG00668 | Azospirillum_sp__CAG_239 |
| CAG00669 | Firmicutes_bacterium_CAG_103 |
| CAG00676 | Firmicutes_bacterium_CAG_176 |
| CAG00698 | Ruminococcus_sp__CAG_177 |
| CAG00713 | Firmicutes_bacterium_CAG_270 |
| CAG00720 | Anaerotruncus_colihominis_DSM_17241 |
| CAG00727 | Eggerthella_lenta_DSM_2243 |
| CAG00766 | Firmicutes_bacterium_CAG_176 |
| CAG00771 | Clostridium_sp__CAG_413 |
| CAG00782 | Eubacterium_rectale_CAG_36 |
| CAG00861 | Oscillibacter_sp__CAG_241 |
| CAG00873 | Butyricimonas_virosa_DSM_23226 |
| CAG00880 | Subdoligranulum_sp__CAG_314 |
| CAG00886 | Sutterella_sp__CAG_351 |
| CAG00889 | Megasphaera_elsdenii_14_14 |
| CAG00897 | Firmicutes_bacterium_CAG_83 |
| CAG00919 | Clostridium_methylpentosum_DSM_5476 |
| CAG00928 | Acidiphilium_sp__CAG_727 |
| CAG00937 | Clostridium_sp__CAG_7 |

(continued)

| CAG number | Bacterial species |
|---|---|
| CAG00963 | Clostridium_sp__CAG_524 |
| CAG01039 | Faecalibacterium_cf__prausnitzii_KLE1255 |
| CAG01141 | Holdemanella_biformis_DSM_3989 |
| CAG01144 | Lactobacillus_vaginalis_DSM_5837__ATCC_49540 |
| CAG01158 | Pseudoflavonifractor_capillosus_ATCC_29799 |
| CAG01197 | Dialister_succinatiphilus_YIT_11850 |
| CAG01208 | Coprococcus_catus_GD_7 |
| CAG01263 | Clostridium_clostridioforme_2_1_49FAA |
| CAG01321 | Faecalibacterium_prausnitzii_SL3_3 |

(ii) using one or several pre-defined equations each corresponding to a model obtained for at least 8 bacterial species from said set of bacterial species, calculating the probability that said individual responds to the treatment ($P_R$) or the probability that said individual resists to the treatment ($P_{NR}$) with an ICI-based therapy.

In the above method, the "abundances pattern based on the relative abundances of a set of bacterial species" can be, for example, in the form of a vector of said relative abundances. This abundance pattern will be inserted into an executable program (Windows OS environment) by the person performing the method (e.g., a clinician) to obtain the NR or R probability percentage of a subject.

As described in the experimental part below, several models can be obtained from clinical data of a representative cohort to assess the probability, for an individual, to respond (or not to respond) to the treatment. The experimental part describes in detail 12 possible models which, although based on different strategies, all provide very good predictability and success rates.

In step (ii), several equations can be used, each corresponding to a model based on a subset of 8 bacteria from the set recited in step (i). The clinician will then combine the result obtained with each of these equations to more precisely predict the R or NR status of the patient.

According to a particular embodiment of the above method, at least one equation used in step (ii) corresponds to a model for a set of at least 8 bacterial species which are present in the fecal sample of said individual. However, a model based on 8 bacteria which are not all present in the sample can also be used, especially if it is combined to one or several models.

According to another particular embodiment of the above method, the model(s) used in step (ii) lead to an overall predictability of at least 65% and a success rate of at least 70%. The performance of a model will depend on the representativity of the cohort on which it has been obtained and on the relevance of the strategy used to build the model. A person skilled in the art of statistics can easily calculate the overall predictability and success rate of a model and determine whether it satisfies the required predictability and success rate criteria.

According to another particular embodiment of the above method, the Probability score obtained in step (ii) is interpreted as clinically meaningful if it is higher than 75% (fixed threshold) and/or if it is concordant among the different Models used, always taking into account the antibiotic usage (known or unknown).

According to another aspect of the present invention, the above method is combined with another method for determining, from a feces sample from a RCC patient, whether said patient is likely to be a good responder to a treatment with an ICI, based on an animal model. Such a method was already described in a previous application from the inventors's team (WO2016/063263) and comprises the steps of (i) performing a fecal microbial transplantation (FMT) of a feces sample from the patient into germ free (GF) model animals (e.g., GF mice); (ii) at least 7 to 14 days after step (i), inoculating said mice with a transplantable tumor model; (iii) treating the inoculated mice with the ICI; and (iv) measuring the tumor size in the treated animals. The results of step (iv) are illustrative of the response that can be expected for said patient to said treatment. In case the result obtained with the animal differs from the NR or R status predicted by the model(s) (with a probability of X%), the result with the animal model will prevail in the clinician's conclusion.

When the individual's antibiotic regimen exposure during the last two months is unknown, the method according to the invention is preferably performed using, in step (i), a set of bacterial species that comprises at least 8 bacterial species selected from the group consisting of:

**Table 2:** Bacterial species preferably used to assess the R or NR status (best outcome criterion) of a patient whose antibiotic regimen exposure during the last two months is unknown.

| CAG number | Bacterial species |
|---|---|
| CAG00008 | Clostridium_bolteae_ATCC_BAA_613 |
| CAG00037 | Bacteroides_faecis_MAJ27 |
| CAG00063 | Barnesiella_ viscericola_DSM_18177 |
| CAG00122 | Coprococcus_catus_GD_7 |
| CAG00140 | Subdoligranulum_sp__4_3_54A2FAA |
| CAG00243 | Lachnospiraceae_bacterium_1_1_57FAA |
| CAG00317 | Clostridium_sp__CAG_230 |
| CAG00327 | Faecalibacterium_sp__CAG_74 |
| CAG00357 | Bacteroides_ovatus_V975 |
| CAG00413 | Bacteroides_sp__CAG_144 |
| CAG00473 | Prevotella_sp__CAG_617 |
| CAG00610 | Hungatella_hathewayi_12489931 |
| CAG00650 | Dorea_formicigenerans_ATCC_27755 |
| CAG00727 | Eggerthella_lenta_DSM_2243 |
| CAG00928 | Acidiphilium_sp__CAG_727 |
| CAG01039 | Faecalibacterium_cf__prausnitzii_KLE1255 |
| CAG01141 | Holdemanella_biformis_DSM_3989 |
| CAG01144 | Lactobacillus_vaginalis_DSM_5837__ATCC_49540 |
| CAG01263 | Clostridium_clostridioforme_2_1_49FAA |

According to a preferred embodiment of the method for assessing the R or NR status (best outcome criterion) of a patient whose antibiotic regimen exposure during the last two months is unknown, one, two or three equations are used in step (ii), which correspond to models obtained for the following sets of bacterial species, identified by their CAG numbers:

**Table 3:** sets of bacteria used in Models 1.2, 2.2 and 3.2.

| set for model 1.2 | set for model 2.2 | set for model 3.2 |
|---|---|---|
| CAG00008 | CAG00727 | CAG00243 |
| CAG01039 | CAG01141 | CAG00473 |
| CAG00473 | CAG00650 | CAG00327 |
| CAG00140 | CAG00928 | CAG01039 |
| CAG00610 | CAG00473 | CAG00140 |
| CAG01141 | CAG00122 | CAG01263 |
| CAG00413 | CAG01144 | CAG00037 |
| CAG00317 | CAG00063 | CAG00357 |

According to a preferred embodiment of the above method, at least one equation used corresponds to a model obtained with a set of bacteria which are all present in the individual's sample. However, the models can be run even if a few bacteria are missing. In such a case, it is preferable to use at least two and preferably at least 3 equations in step (ii).
According to a particular embodiment of the above method based on Models 1.2, 2.2 and/or 3.2, illustrated in the experimental part below, the equations for calculating the probability that said individual responds to the treatment ($P_R$) are as follows:

$$[B] \ P_R = 1 / [1 + \exp{^\wedge}{-}(\sum_{j=1}^{8} \beta_j X_j)]$$

wherein $X_j$ ($j$ = 1 to 8) are the relative abundances of the bacterial species measured in the individual's sample and $\beta_j$ ($j$ = 1 to 8) are the following regression coefficients:

**Table 4:** Regression coefficients for Models 1.2, 2.2 and 3.2.

| Model 1.2 | | Model 2.2 | | Model 3.2 | |
|---|---|---|---|---|---|
| bacterial species | R coefficients | bacterial species | R coefficients | bacterial species | R coefficients |
| CAG00008 | -1.0323 | CAG00727 | -1.0791 | CAG00243 | -1.1735 |
| CAG01039 | -1.084 | CAG01141 | -1.1164 | CAG00473 | -1.027 |
| CAG00473 | -0.9723 | CAG00650 | -1.2839 | CAG00327 | -0.9968 |
| CAG00140 | -0.9907 | CAG00928 | -1.1156 | CAG01039 | -1.0043 |
| CAG00610 | -0.6234 | CAG00473 | -1.014 | CAG00140 | -0.7456 |
| CAG01141 | -0.8468 | CAG00122 | -0.9394 | CAG01263 | -0.8736 |
| CAG00413 | 0.8994 | CAG01144 | -0.8085 | CAG00037 | 0.7032 |
| CAG00317 | 0.6041 | CAG00063 | -0.7679 | CAG00357 | -0.6073 |

Of course, these coefficients can be refined by the skilled in the art by performing the same strategies as those described in the present application starting from other clinical data (*i.e.,* clinical data from another cohort in addition to or in replacement of the data used by the inventors), or even merely because the 10000 iterations employed in the logistic regression (see methods) ensure each time a weak 'floating randomness' of each coefficient, not surpassing the 0.2 units. The coefficients can also differ if a different technique is used for measuring the relative abundances of the bacterial species (e.g., using qPCR instead of MGS analysis). Importantly, even using different techniques, regression coefficients will retain their positive or negative sign, meaning the positive or negative contribution of a definite CAG species to the overall model.

In case the patient did not take any antibiotic during the last two months, the method according to the invention is preferably performed using, in step (i), a set of bacterial species that comprises at least 8 bacterial species selected from the group consisting of:

**Table 5:** Bacterial species preferably used to assess the R or NR status (best outcome criterion) of a patient who did not take antibiotics during the last two months.

| CAG number | Bacterial species |
|---|---|
| CAG00008 | Clostridium_bolteae_ATCC_BAA_613 |
| CAG00048_1 | Clostridium_sp__CAG_226 |
| CAG00140 | Subdoligranulum_sp__4_3_54A2FAA |
| CAG00243 | Lachnospiraceae_bacterium_1_1_57FAA |
| CAG00300 | Prevotella_sp__CAG_891 |
| CAG00327 | Faecalibacterium_sp__CAG_74 |
| CAG00413 | Bacteroides_sp__CAG_144 |
| CAG00473 | Prevotella_sp__CAG_617 |
| CAG00487 | Prevotella_sp__CAG_279 |
| CAG00650 | Dorea_formicigenerans_ATCC_27755 |

(continued)

| CAG number | Bacterial species |
|---|---|
| CAG00698 | Ruminococcus_sp__CAG_177 |
| CAG00720 | Anaerotruncus_colihominis_DSM_17241 |
| CAG00727 | Eggerthella_lenta_DSM_2243 |
| CAG00766 | Firmicutes_bacterium_CAG_176 |
| CAG00897 | Firmicutes_bacterium_CAG_83 |
| CAG00963 | Clostridium_sp__CAG_524 |
| CAG01039 | Faecalibacterium_cf__prausnitzii_KLE1255 |
| CAG01141 | Holdemanella_biformis_DSM_3989 |
| CAG01208 | Coprococcus_catus_GD_7 |

According to a preferred embodiment of the method for assessing the R or NR status (best outcome criterion) of a patient who did not take antibiotics during the last two months, one, two or three equations are used in step (ii), each of which corresponding to a model obtained for the following sets of bacterial species, identified by their CAG numbers:

**Table 6:** Sets of bacteria used in Models 4.2, 5.2 and 6.2.

| set for model 4.2 | set for model 5.2 | set for model 6.2 |
|---|---|---|
| CAG00008 | CAG00048_1 | CAG00243 |
| CAG01039 | CAG00300 | CAG00008 |
| CAG00473 | CAG00473 | CAG00698 |
| CAG00487 | CAG00650 | CAG00473 |
| CAG00140 | CAG00720 | CAG00327 |
| CAG01141 | CAG00727 | CAG01039 |
| CAG01208 | CAG00963 | CAG00897 |
| CAG00413 | CAG01141 | CAG00766 |

According to a preferred embodiment of the above method, at least one equation used corresponds to a model obtained with a set of bacteria which are all present in the individual's sample. However, the models can be run even if a few bacteria are missing. In such a case, it is preferable to use at least two and preferably at least 3 equations in step (ii).
According to a particular embodiment of the above method based on Models 4.2, 5.2 and/or 6.2, illustrated in the experimental part below, the equations for calculating the probability that said individual resists ($P_{NR}$) or responds ($P_R$) to the treatment are as follows:

$$[A] \quad P_{NR} = 1 / [1 + exp^{\wedge}\text{-}(\sum_{j=1}^{8} \beta_j X_j)] \text{ for models 4.2 and 5.2}$$

$$[B] \quad P_R = 1 / [1 + exp^{\wedge}\text{-}(\sum_{j=1}^{8} \beta_j X_j)] \text{ for model 6.2}$$

wherein $X_j$ ($j$ = 1 to 8) are the relative abundances of the bacterial species measured in the individual's sample and $\beta_j$ ($j$ = 1 to 8) are the following regression coefficients:

Table 7: Regression coefficients for Models 4.2, 5.2 and 6.2.

| Model 4.2 | | Model 5.2 | | Model 6.2 | |
|---|---|---|---|---|---|
| bacterial species | NR coefficients | bacterial species | NR coefficients | bacterial species | R coefficients |
| CAG00008 | 0.955 | CAG00048_1 | 0.7112 | CAG00243 | -1.2907 |
| CAG01039 | 0.8761 | CAG00300 | 0.8931 | CAG00008 | -0.8848 |
| CAG00473 | 0.9951 | CAG00473 | 1.0042 | CAG00698 | -0.9776 |
| CAG00487 | 0.7609 | CAG00650 | 1.1282 | CAG00473 | -0.9997 |
| CAG00140 | 1.1107 | CAG00720 | 1.2569 | CAG00327 | -1.0697 |
| CAG01141 | 0.9402 | CAG00727 | 1.2397 | CAG01039 | -0.7806 |
| CAG01208 | 0.7187 | CAG00963 | 0.7595 | CAG00897 | -0.8376 |
| CAG00413 | -0.5812 | CAG01141 | 1.0235 | CAG00766 | -0.7459 |

As mentioned above, these coefficients can be refined for several reasons or even changed if a different technique is used for measuring the relative abundances of the bacterial species (e.g., using qPCR instead of MGS analysis).

The models 1.2, 2.2, 3.2, 4.2, 5.2 and 6.2 above have been obtained using a criterion of best outcome. Patients who first responded to the treatment but relapsed a few months later were thus considered as responders. In order to assess if an individual is likely to have a long-term benefit from the treatment, defined as a time to progression (TTP) of at least 6 months, the inventors obtained additional models by considering only the stable diseases of more than 6 months (SD >6 months) among responders (OUTCOME_2 in the experimental part). These models are based on partly different subsets of the microbiota composition.

For assessing whether an individual whose antibiotic regimen exposure during the last two months is unknown is likely to have a long-term benefit from a treatment with an anti-PD1/PD-L1/PD-L2 Ab-based therapy, the method according to the invention is preferably performed using, in step (i), a set of bacterial species that comprises at least 8 bacterial species selected from the group consisting of:

Table 8: Bacterial species preferably used to assess the R or NR status (TTP>6 months criterion) of a patient whose antibiotic regimen exposure during the last two months is unknown.

| CAG number | Bacterial species |
|---|---|
| CAG00013 | Clostridiales_bacterium_1_7_47FAA |
| CAG00211 | Firmicutes_bacterium_CAG_227 |
| CAG00474 | Sutterella_wadsworthensis_2_1_59BFAA |
| CAG00557 | Ruminococcus_callidus_ATCC_27760 |
| CAG00601 | uncultured_Faecalibacterium_sp_ |
| CAG00607 | Eubacterium_sp__CAG_251 |
| CAG00624 | Firmicutes_bacterium_CAG_552 |
| CAG00650 | Dorea_formicigenerans_ATCC_27755 |
| CAG00668 | Azospirillum_sp__CAG_239 |
| CAG00669 | Firmicutes_bacterium_CAG_103 |
| CAG00676 | Firmicutes_bacterium_CAG_176 |
| CAG00771 | Clostridium_sp__CAG_413 |
| CAG00782 | Eubacterium_rectale_CAG_36 |
| CAG00861 | Oscillibacter_sp__CAG_241 |
| CAG00873 | Butyricimonas_virosa_DSM_23226 |

(continued)

| CAG number | Bacterial species |
|---|---|
| CAG00880 | Subdoligranulum_sp__CAG_314 |
| CAG00886 | Sutterella_sp__CAG_351 |
| CAG00889 | Megasphaera_elsdenii_14_14 |
| CAG00937 | Clostridium_sp__CAG_7 |
| CAG01141 | Holdemanella_biformis_DSM_3989 |
| CAG01197 | Dialister_succinatiphilus_YIT_11850 |
| CAG01321 | Faecalibacterium_prausnitzii_SL3_3 |

According to a preferred embodiment of the method for assessing the R or NR status (TTP> 6months criterion) of a patient whose antibiotic regimen exposure during the last two months is unknown, one, two or three equations are used in step (ii), which correspond to models obtained for the following sets of bacterial species, identified by their CAG numbers:

**Table 9:** Sets of bacteria used in Models 7.2, 8.2 and 9.2.

| set for model 7.2 | set for model 8.2 | set for model 9.2 |
|---|---|---|
| CAG00782 | CAG00211 | CAG00557 |
| CAG00013 | CAG00474 | CAG00601 |
| CAG00873 | CAG00624 | CAG00607 |
| CAG01141 | CAG00650 | CAG00669 |
| CAG00668 | CAG00676 | CAG00861 |
| CAG00669 | CAG00771 | CAG00880 |
| CAG00886 | CAG01197 | CAG00937 |
| CAG00889 | CAG01321 | CAG01321 |

According to a preferred embodiment of the above method, at least one equation used in step (ii) corresponds to a model obtained with a set of bacteria which are all present in the individual's sample. However, the models can be run even if a few bacteria are missing. In such a case, it is preferable to use at least two and preferably at least 3 equations in step (ii).

According to a particular embodiment of the above method based on Models 7.2, 8.2 and/or 9.2, illustrated in the experimental part below, the equations for calculating the probability that said individual resists ($P_{NR}$) to the treatment are as follows:

$$[A] \quad P_{NR} = 1 / [1 + exp\text{^}-(\sum_{j=1}^{8} \beta_j X_j)]$$

wherein $X_j$ ($j$ = 1 to 8) are the relative abundances of the bacterial species measured in the individual's sample and $\beta_j$ ($j$ = 1 to 8) are the following regression coefficients:

**Table 10:** Regression coefficients for Models 7.2, 8.2 and 9.2.

| Model 7.2 | | Model 8.2 | | Model 9.2 | |
|---|---|---|---|---|---|
| bacterial species | NR coefficients | bacterial species | NR coefficients | bacterial species | NR coefficients |
| CAG00782 | -0.2034 | CAG00211 | 0.6422 | CAG00557 | 0.9341 |
| CAG00013 | 0.4092 | CAG00474 | -1.0581 | CAG00601 | 0.8927 |

(continued)

| Model 7.2 | | Model 8.2 | | Model 9.2 | |
|---|---|---|---|---|---|
| bacterial species | NR coefficients | bacterial species | NR coefficients | bacterial species | NR coefficients |
| CAG00873 | 0.2708 | CAG00624 | -0.8984 | CAG00607 | 0.7108 |
| CAG01141 | 0.7665 | CAG00650 | 1.3189 | CAG00669 | -1.2341 |
| CAG00668 | -0.5899 | CAG00676 | -0.7324 | CAG00861 | 1.0379 |
| CAG00669 | -0.5181 | CAG00771 | -0.6623 | CAG00880 | -1.0016 |
| CAG00886 | -0.8415 | CAG01197 | -1.1372 | CAG00937 | -0.872 |
| CAG00889 | -0.7339 | CAG01321 | -0.8525 | CAG01321 | -0.8362 |

As already mentioned, these coefficients can be refined for several reasons or even changed if a different technique is used for measuring the relative abundances of the bacterial species.

In case the patient did not take any antibiotic during the last two months, the method according to the invention for assessing his/her probability of having a long-term benefit (TTP>6 months) from a treatment with an anti-PD1/PD-L1/PD-L2 Ab-based therapy is preferably performed using, in step (i), a set of bacterial species that comprises at least 8 bacterial species selected from the group consisting of:

Table 11: Bacterial species preferably used to assess the R or NR status (best outcome criterion) of a patient who did not take antibiotics during the last two months.

| CAG number | Bacterial species |
|---|---|
| CAG00013 | Clostridiales_bacterium_1_7_47FAA |
| CAG00142 | Bacteroides_stercoris_ATCC_43183 |
| CAG00218 | Bacteroides_sp__CAG_20 |
| CAG00341 | Eubacterium_sp__CAG_115 |
| CAG00346 | Eubacterium_rectale_M104_1 |
| CAG00474 | Sutterella_wadsworthensis_2_1_59BFAA |
| CAG00508 | Alistipes_obesi |
| CAG00530 | Prevotella_sp__CAG_617 |
| CAG00580 | Clostridium_sp__CAG_62 |
| CAG00601 | uncultured_Faecalibacterium_sp_ |
| CAG00607 | Eubacterium_sp__CAG_251 |
| CAG00646 | Alistipes_sp__CAG_268 |
| CAG00668 | Azospirillum_sp__CAG_239 |
| CAG00669 | Firmicutes_bacterium_CAG_103 |
| CAG00713 | Firmicutes_bacterium_CAG_270 |
| CAG00873 | Butyricimonas_virosa_DSM_23226 |
| CAG00880 | Subdoligranulum_sp__CAG_314 |
| CAG00886 | Sutterella_sp__CAG_351 |
| CAG00889 | Megasphaera_elsdenii_14_14 |
| CAG00919 | Clostridium_methylpentosum_DSM_5476 |
| CAG01141 | Holdemanella_biformis_DSM_3989 |
| CAG01158 | Pseudoflavonifractor_capillosus_ATCC_29799 |
| CAG01263 | Clostridium_clostridioforme_2_1_49FAA |

(continued)

| CAG number | Bacterial species |
|---|---|
| CAG01321 | Faecalibacterium_prausnitzii_SL3_3 |

According to a preferred embodiment of the method for assessing the R or NR status (TTP> 6 months criterion) of a patient who did not take antibiotics during the last two months, one, two or three equations are used in step (ii), each of which corresponding to a model obtained for the following sets of bacterial species, identified by their CAG numbers:

Table 12: Sets of bacterial species for Models 10.2, 11.2 and 12.2.

| set for model 10.2 | set for model 11.2 | set for model 12.2 |
|---|---|---|
| CAG00580 | CAG00346 | CAG00142 |
| CAG00013 | CAG00530 | CAG00218 |
| CAG00873 | CAG00601 | CAG00341 |
| CAG01141 | CAG00607 | CAG00474 |
| CAG00668 | CAG00646 | CAG00508 |
| CAG00669 | CAG00713 | CAG00880 |
| CAG00886 | CAG00919 | CAG01263 |
| CAG00889 | CAG01158 | CAG01321 |

According to a preferred embodiment of the above method, at least one equation used in step (ii) corresponds to a model obtained with a set of bacteria which are all present in the individual's sample. However, the models can be run even if a few bacteria are missing. In such a case, it is preferable to use at least two and preferably at least 3 equations in step (ii).

According to a particular embodiment of the above method based on Models 10.2, 11.2 and/or 12.2, illustrated in the experimental part below, the equations for calculating the probability that said individual resists to the treatment ($P_{NR}$) or responds ($P_R$) are as follows:

$$[A] \quad P_{NR} = 1 / [1 + \exp{\char`\^}-(\sum_{j=1}^{8} \beta_j X_j)] \text{ for model 12.2}$$

$$[B] \quad P_R = 1 / [1 + \exp{\char`\^}-(\sum_{j=1}^{8} \beta_j X_j)] \text{ for models 10.2 and 11.2}$$

wherein $X_j$ ($j$ = 1 to 8) are the relative abundances of the bacterial species measured in the individual's sample and $\beta_j$ ($j$ = 1 to 8) are the following regression coefficients:

Table 13: Regressions coefficients for Models 10.2, 11.2 and 12.2.

| Model 10.2 | | Model 11.2 | | Model 12.2 | |
|---|---|---|---|---|---|
| bacterial species | R coefficients | bacterial species | R coefficients | bacterial species | NR coefficients |
| CAG00580 | -0.1456 | CAG00346 | -0.8463 | CAG00142 | 1.138 |
| CAG00013 | -0.5496 | CAG00530 | 1.13 | CAG00218 | 1.1396 |
| CAG00873 | -0.4646 | CAG00601 | -1.183 | CAG00341 | -1.0166 |
| CAG01141 | -0.5496 | CAG00607 | -0.9544 | CAG00474 | -0.8403 |

(continued)

| Model 10.2 | | Model 11.2 | | Model 12.2 | |
|---|---|---|---|---|---|
| bacterial species | R coefficients | bacterial species | R coefficients | bacterial species | NR coefficients |
| CAG00668 | 0.5684 | CAG00646 | 0.8929 | CAG00508 | -0.7067 |
| CAG00669 | 0.5129 | CAG00713 | 1.0919 | CAG00880 | -1.185 |
| CAG00886 | 0.6623 | CAG00919 | 0.4881 | CAG01263 | 0.8466 |
| CAG00889 | 0.7102 | CAG01158 | 1.1606 | CAG01321 | -0.8895 |

As for the other models, these coefficients can be refined for several reasons or even changed if a different technique is used for measuring the relative abundances of the bacterial species.

According to a particular embodiment of the method of the invention, the fecal sample is obtained before the first administration of any ICI, such as an anti-PD1/PD-L1/PD-L2 antibody.

According to another particular embodiment, the individual already received a first-line therapy different from PD1/PD-L1/PD-L2 Ab-based therapies for treating his/her RCC. The method of the invention is however not limited to patients receiving a PD1/PD-L1/PD-L2 Ab-based therapy as a second line therapy, and the presently disclosed methods can also be used to assess the responder or non-responder status of RCC patients receiving a first-line PD1/PD-L1/PD-L2 Ab-based therapy either alone or in combination with other antineoplastic drugs (chemotherapy or another ICI such as anti-CTLA-4, as specified above).

According to another particular embodiment, the anti-PD1/PD-L1/PD-L2 Ab-based therapy administered to the patient is a treatment with an anti-PD1 antibody such as nivolumab or pembrolizumab or an anti-PD-L1 antibody such as atezolizumab or durvalumab, for example.

According to another of its aspects, the present invention pertains to a theranostic method for determining if a cancer patient needs a bacterial compensation before administration of an anti-PD1/PD-L1/PD-L2 Ab-based therapy and/or during this therapy, comprising assessing, by a method as above-disclosed, whether the patient is likely to be a good responder to such a therapy, wherein if the patient is not identified as likely to be a good responder, the patient needs a bacterial compensation.

According to this aspect of the invention, the bacterial compensation can be done either by fecal microbiota transplant (FMT), using microbiota from one or several donors (for example, from responders to the treatment), possibly enriched with bacterial strains known to be beneficial in this situation, or by administration of a bacterial composition. The inventors already described bacterial compositions that can be used for such a compensation and restore the ability, for the patient, to respond to the treatment (e.g., in WO 2016/063263 and in WO 2018/115519). Non-limitative examples of bacterial strains which can be beneficial to patients with an initial NR status are: *Enterococcus hirae, Akkermansia muciniphila, Blautia* strains, *Coprococcus comes* strains, *Alistipes shahii,* other *Alistipes* species (e.g. *Alistipes indistinctus* and/or *onderdonkii* and/or *finegoldii*), *Ruminococcacae, Clostridiales* species, *Bacteroidales* species, *Actinobacteria, Coriobacteriales* species, *Methanobrevibacter smithii, Burkholderia cepacia, Bacteroides fragilis, Actinotignum schaalii,* as well as the following additional bacteria:

• *Clostridiales* bacteria of the species *Christensenella minuta;*
• *Erysipelotrichia* of the species *Dielma fastidiosa or Erysipelatoclostridium ramosum*;
• *Eubacterium limosum*;
• *Bacteroidales* bacteria of the species *Bacteroides salyersiae* and/or *Barnesiella intestinihominis;*
• *Coriobacteriales bacteria* of the species *Collinsella intestinalis* and/or *Collinsella tanakaei*;
• *Firmicutes bacteria* of the species *Flavonifractor plautii.*

Examples of compositions which can be beneficial to a patient with an initial NR status assessed by the method according to the invention are:

• a composition comprising:

(i) *Enterococcus hirae* selected from the group consisting of *Enterococcus hirae* strain 13144 deposited on November 7, 2013 at the *Collection Nationale de Cultures de Microorganismes* (CNCM) under the number I-4815, *Enterococcus hirae* strain IGR7 deposited on August 31, 2017 at the CNCM under the number I-5224, *Enterococcus hirae* strain IGR4 deposited on November 27, 2017 at the CNCM under the number CNCM I-5260, *Enterococcus hirae* strain IGR11 deposited on November 27, 2017 at the CNCM under the number CNCM I-5261 and mixtures thereof; and

(ii) *Akkermansia muciniphila* selected from the group consisting of *Akkermansia muciniphila* strains p2261 and p3415, both deposited at the *Collection de souches de l'Unité des Rickettsies* (CSUR) and mixtures thereof; and

(iii) *Eubacterium limosum.*

- a composition comprising:

  (i) *Enterococcus hirae* selected from the group consisting of *Enterococcus hirae* strain 13144 deposited on November 7, 2013 at the *Collection Nationale de Cultures de Microorganismes* (CNCM) under the number I-4815, *Enterococcus hirae* strain IGR7 deposited on August 31, 2017 at the CNCM under the number I-5224, *Enterococcus hirae* strain IGR4 deposited on November 27, 2017 at the CNCM under the number CNCM I-5260, *Enterococcus hirae* strain IGR11 deposited on November 27, 2017 at the CNCM under the number CNCM I-5261 and mixtures thereof; and

  (ii) *Barnesiella intestinihominis.*

- a composition comprising:

  (i) *Enterococcus hirae* selected from the group consisting of *Enterococcus hirae* strain 13144 deposited on November 7, 2013 at the *Collection Nationale de Cultures de Microorganismes* (CNCM) under the number I-4815, *Enterococcus hirae* strain IGR7 deposited on August 31, 2017 at the CNCM under the number I-5224, *Enterococcus hirae* strain IGR4 deposited on November 27, 2017 at the CNCM under the number CNCM I-5260, *Enterococcus hirae* strain IGR11 deposited on November 27, 2017 at the CNCM under the number CNCM I-5261 and mixtures thereof; and

  (ii) *Christensenella minuta.*

- a composition comprising:

  (i) *Enterococcus hirae* selected from the group consisting of *Enterococcus hirae* strain 13144 deposited on November 7, 2013 at the *Collection Nationale de Cultures de Microorganismes* (CNCM) under the number I-4815, *Enterococcus hirae* strain IGR7 deposited on August 31, 2017 at the CNCM under the number I-5224, *Enterococcus hirae* strain IGR4 deposited on November 27, 2017 at the CNCM under the number CNCM I-5260, *Enterococcus hirae* strain IGR11 deposited on November 27, 2017 at the CNCM under the number CNCM I-5261 and mixtures thereof; and

  (ii) *Actinotignum schaalii.*

A nucleic acid microarray designed to perform the method according to the invention is also part of the present invention. Such a nucleic acid microarray comprises nucleic acid probes specific for each of the microorganism species to be detected in step (i) of said method (*i.e.,* at least 8 species selected amongst those recited in Table 1). In a specific embodiment, the nucleic acid microarray is an oligonucleotide microarray comprising at least one oligonucleotide specific for at least one sequence selected from SEQ ID NOs: 1-2950. For example, the said microarray comprises at least 8 oligonucleotides, each oligonucleotide being specific for one sequence of a distinct species recited in table 1. The microarray of the invention can of course comprise more oligonucleotides specific for sequences of SEQ ID NOs: 1-2950, for example at least nx96 oligonucleotides, divided into 12 sets of nx8 oligonucleotides corresponding to the 12 models described herein, with n being an integer comprised between 1 and 25 which corresponds to the number of oligonucleotides used to specifically assess the presence of one specific bacterial species. The microarray according to the invention may further comprise at least one oligonucleotide for detecting at least one gene of at least one control bacterial species. A convenient bacterial species may be e.g. a bacterial species the abundance of which does not vary between individuals having a R or a NR status. Preferably, the oligonucleotides are about 50 bases in length. Suitable microarray oligonucleotides specific for any gene of SEQ ID NOs: 1-2950 may be designed, based on the genomic sequence of each gene, using any method of microarray oligonucleotide design known in the art. In particular, any available software developed for the design of microarray oligonucleotides may be used, such as, for instance, the OligoArray software, the GoArrays software, the Array Designer software, the Primer3 software, or the Promide software, all known by the skilled in the art.

As mentioned above, the relative abundance of the recited bacterial species can be measured by techniques different from the metagenomics analysis used herein, especially when the skilled in the art knows which bacterial species are to be measured. A particular technique which can be used to this purpose is qPCR (quantitative PCR). The PCR-based techniques are performed with amplification primers designed to be specific for the sequences which are measured. The present invention hence also pertains to a set of primers suitable for performing the above method, *i.e.,* a set of primers comprising primer pairs for amplifying sequences specific for each of the microorganism species to be detected in step (i) of said method (*i.e.,* at least 8 species selected amongst those recited in Table 1). Such a set of primers comprises a minimum of 16 primers, but it can comprise more primers, for example 30, 40, 50, 60, 70, 80, 100, 200, 300, 500, 1000, 2000 or more. According to a particular embodiment, the set of primers comprises at least one primer pair specifically amplifying part of a sequence selected amongst SEQ ID Nos: 1-2950. Of course, primer sets according to the invention can advantageously comprise 2, 3, 4, 5, 10, 20, 30, 40, 50, 60, 70, 80, 100, 200, 300, 500, 1000 or more pairs of primers each specifically amplifying part of a sequence selected amongst SEQ ID Nos: 1-2950.

Other characteristics of the invention will also become apparent in the course of the description which follows of the biological assays which have been performed in the framework of the invention and which provide it with the required experimental support, without limiting its scope.

## EXAMPLES

### Materials and Methods

### *Metagenomic analysis of stool samples*

Quantitative metagenomics pipeline developed at MetaGenoPolis (Jouy-en-Josas, France) was employed to reach the species-level description of RCC gut microbiota. DNA was extracted from 69 stool samples through an automated platform (SAMBO, MetaGenoPolis) and subjected to shotgun sequencing using Ion Proton sequencer to reach > 20 million short DNA sequence reads (MetaQuant platform, MetaGenoPolis). Reads were filtered (Q>25) and cleaned to eliminate possible contaminants as human reads. The high-quality (HQ) reads were then mapped against the MetaHIT hs_9.9M genes catalogue and counted through a two-step procedure: 1) using uniquely mapped reads; 2) attributing shared reads according to their mapping ratio based on unique reads. Identity threshold for mapping was set at >95%, in order to overcome gene allelic variants and the non-redundant nature of the catalogue itself. HQ reads were downsized at 13 millions for each sample in order to correct for sequencing depth, then normalized through RPKM (reads per kilo base per million mapped reads) method. A profile matrix of gene frequencies was thus obtained, and used as an input file for MetaOMineR, a suite of R packages developed at MetaGenoPolis.

In order to achieve the species-level description of microbes in RCC stool samples, the hs_9.9M catalogue was clustered into 1436 MetaGenomic Species (MGS), roughly >500 genes that covary in abundance among hundreds of samples, ultimately belonging to the same microbial species. The taxonomical annotation of MGS was performed using gene homology with previously sequenced organisms using BLASTn against nt and wgs NCBI databases. After taxonomical assignment of each MGS, a matrix of frequency profiles of 50 co-abundant genes (CAG) was built using normalized MGS mean signals, thus the sum of each MGS within a sample resulted equal to 1. With this approach each species was assigned to a definite CAG number.

### *Statistical analysis*

Multivariate statistics were employed on the matrix of frequency profiles in order to describe the gut microbial composition and diversity. Python 2.7 and related statistical/graphical libraries (Matplotlib, Scikit learn, Pandas, Numpy) were used to compare all MGS through pairwise analysis, linear discriminant analysis effect size (LEfSe) and volcano plots. A P value less than or equal to 0.05 was considered statistically significant. Volcano plots were generated computing for each bacterial species: i) the log2 of fold ratio (FR) among the mean relative abundances of R versus NR (x axis) and ii) the co-log10 of P values deriving from Mann-Whitney U test calculated on relative abundances in absolute values (y axis). In order to ease the volcano graphical representation, at TTP6 the chosen P value was less than or equal to 0.7. LEfSe plots were generated with Python 2.7 on output files derived from LEfSe pipeline, and all species with LDA score $\geq$ 2 were considered for subsequent analysis.

### *Building the predictive models*

A double strategy was implemented to select the bacterial species used to build the models.

The first one ("human-oriented") relies on: i) performing Receiver Operating Characteristic (ROC) curves implementing Support Vector Machine (SVM) classifier on all possible combinations of bacterial species, looking for consortia giving the best Area Under Curve (AUC) values; ii) building a logistic regression classifier and a RFECV (recursive feature elimination with cross-validation) feature selection with selected bacterial species; iii) performing metrics as ROC curves and confusion matrices using the train/test subsets deriving from the original dataset.

The second strategy ("machine-learning") relies on : i) building a logistic regression classifier and a RFECV feature selection on all bacterial species (without permutation), selecting the ones with higher ranking (equal to 1); ii) performing a new feature selection by means of RFE (recursive feature elimination), selecting the 8 species with highest ranking; iii) re-building a new logistic regression classifier plus RFECV feature selection on the 8 selected bacterial species; iv) performing metrics as ROC curves and confusion matrices using the train/test subsets deriving from the original dataset.

The main difference among the first and the second strategies is that the first one allows combinations of all the bacterial species present in the original dataset selecting sub-groups of them (called 'consortia') based on the highest AUC, while the second one would be able to select single species due to the natural convergence of the logistic regression model to select all the variables in order to reach the maximum predictability.

The implementations for building the logistic regression classifier and the feature selection models were made in Python (version 2.7) using the methods 'LogisticRegression', 'RFE' and 'RFECV' from the Scikit Learn module (version 0.19.1). The implementation for building the SVM classifier (C-Support Vector Classification) was made in Python (version 2.7) using the method SVC from the Scikit Learn module (version 0.19.1). The implementation of combinations for ROC curves in the first strategy was made in Python (version 2.7) using the method 'combinations' from the 'itertools' module present as a built-in in Python 2.7.

Logistic regression coefficients $\beta$ were calculated by logistic regression classifier with the following requirements: a constant (also known as intercept) not added to the decision function; a max iteration equal to 10000; a liblinear solver (with L2 penalization). RFECV feature selection model (step=1, cross-validation equal to 3, scoring accuracy) was implemented in Python with logistic regression estimator (no intercept, 10000 max iteration, liblinear solver), providing rankings within the interval $[1 : (n_{species}-1)]$, and it was used to make a recursive feature selection on all bacterial species, selecting only those species having a rank equal to 1. RFE feature selection model (step=1, feature to select = 8) was similarly implemented in Python with the same estimator (logistic regression, no intercept, 10000 max iteration, liblinear solver) of RFECV.

Receiver Operating Characteristic (ROC) curves were built with Python 2.7 using the SVM classifier (linear kernel) and employed to assess the actual predictive meaning of selected bacterial species or consortia. Two different approaches were used to achieve such a selection: 1) species retrieved from LEfSe analysis (with LDA score $\geq$ 2) and combined within all possible combinations (2x2, 3x3, 4x4, 5x5, etc...) and 2) all species were considered and the first 5 having the best AUC were combined into a consortium. Area Under Curve (AUC), specificity and sensitivity were computed for each best combination, and reported as ROC curve and tabular data. A 5-fold cross-validation was used, with no added noise. Mean relative abundances ($\pm$ Standard Error of the Mean), Fold Ratio (FR), Log10 of FR and P value of cohort comparison (R vs NR, non-parametric Mann-Whitney U test) were also reported.

According to the aforementioned methods, bacterial species were chosen in the following manner: i) for the first strategy, taking into account all the combinations for all available species, and especially those derived from LEfSe, we selected the 'consortium' for each OUTCOME_1, OUTCOME_2, TTP3 and TTP6 timepoints having the highest AUC value, both for NR and R status; ii) for the second strategy, we selected the bacterial species having a RFECV and then RFE rank equal to 1, regardless negative and positive logistic regression coefficients ($\beta$), for OUTCOME_1 and OUTCOME_2.

Taking into account the two aforementioned strategies, a logistic regression classifier was implemented, for both of them, on standardized raw data (removing the mean and scaling to unit variance) in order to calculate regression coefficients ($\beta$) to be used in equation [2]. Thus, the relative contribution of each selected bacterial species to each model was weighted by the $\beta$ coefficient and put into the equation [2] in order to predict through equation [1] the estimation (in probability percentage) of the outcome for a definite new subject.

$$P = 1 / (1 + \exp^{-z}) \qquad [1]$$

and

$$z = \beta 1 * x 1 + \ldots + \beta n * x n \qquad [2]$$

For both strategies, two feature selection models (RFECV and then RFE) model were implemented to refine the number of selected species at 8, a number useful to be implemented in future and forthcoming diagnostic kits for clinical practical use. Evaluation of the final Models (built on three different sets of 8 bacterial species) was performed through metrics as ROC curves and confusion matrices using t train/test subsets of the original dataset (implemented in Python 2.7 and Scikit learn 0.19, through the train_test_split method).

For OUTCOME_1, taking into account all the aforementioned embodiments, methods and strategies, and taking into account the dichotomous/complementary nature of logistic regression classifier, in the present invention we propose four equations ([3], [4], [6], [7]) to predict the NR or R status of a definite new subject JD. Two of them ([3], [4]) take into account the eventuality of an unknown antibiotic regimen/treatment status, while the other two ([6], [7]) consider that the individual did not take antibiotics during the past two months.

In order to circumvent the caveats deriving from the natural sources of variability within the subjects' cohort (feces sampling, feces consistencies, sample storage, sequencing biases, etc...) that could affect relative abundances of under-represented bacterial species, the inventors built two further equations ([5], [8]) based solely on the species having an average relative abundance greater than or equal to 0.001 if expressed in the interval [0 : 1] (or 0.1% if expressed in the interval [0 : 100]) for subjects with unknown antibiotic treatment ([5]) and for subjects who did not take antibiotics during the last two months prior sampling ([8]) These pattern of species underwent the second strategy of modeling as described above.

For OUTCOME_2, taking into account all the aforementioned embodiments, methods and strategies, and taking into account the dichotomous/complementary nature of logistic regression classifier, in the present invention we propose four equations ([9], [10], [12], [13]) to predict the NR or R status of a definite new subject JD. Two of them ([9], [10]) take into account the eventuality of an unknown antibiotic regimen/treatment status, while the other two ([12], [13]) consider that the individual did not take antibiotics during the past two months.

In order to circumvent the caveats deriving from the natural sources of variability within the subjects' cohort (feces sampling, feces consistencies, sample storage, sequencing biases, etc...) that could affect relative abundances of under-represented bacterial species, the inventors built two further equations ([11], [14]) based solely on the species having an average relative abundance greater than or equal to 0.001 if expressed in the interval [0 : 1] (or 0.1% if expressed in the interval [0 : 100]) for subjects with unknown antibiotic treatment ([11]) and for subjects who did not take antibiotics during the last two months prior sampling ([14]) These pattern of species underwent the second strategy of modeling as described above.

**Example 1: Metagenomics-based GOMS associated with clinical outcome according to RECIST 1.1 criteria (Figure 1).**

We explored the composition of the gut microbiota using quantitative metagenomics by shotgun sequencing, reaching >20 million short DNA sequence reads per sample followed by analysis of the results in a 9.9 million-gene reference catalogue (J. Li, Nat. Biotechnol. 32, 834-841 (2014). Total DNA was extracted from 69 patients diagnosed with RCC prior to starting therapy (Table 1). Among the 69 RCC patients, 58 did not take ATB, while 11 had been prescribed ATB before the MG analyses. As reported in Materials and Methods, each metagenomic species (MGS) was assigned to a definite co-abundant gene profile (CAG), and the subsequent taxonomical annotation was performed based on gene homology to previously sequenced organisms (using blastN against the nt and WGS databanks). R (responders) were defined as those patients exhibiting a complete or partial or stable disease >3 months, while NR (non responders) were defined as harbouring a progressive disease.

| | | Total (n=69) absolute numbers (%) |
|---|---|---|
| Age-yr | Median | 62 |
| | Range | 30-82 |
| Age-yr-no.(%) | <65 | 40 (58) |
| | ≥65 <75 | 22 (32) |
| | ≥75 | 7 (10) |
| Gender-no.(%) | Male | 48 (69) |
| | Female | 21 (31) |

| Histology-no.(%) | Clear cell | 67 (97) |
|---|---|---|
| | Non-clear cell | 2 (3) |
| Nephrectomyy-no.(%) | Yes | 63 (91) |
| | No | 6 (9) |
| IMDC risk group-no.(%) | Good | 14 (20) |
| | Intermediate | 39 (57) |
| | Poor | 14 (20) |
| | Unknown | 2 (3) |
| Number of prior treatments-no.(%) | <2 | 55 (80) |
| | ≥3 | 14 (20) |
| ATB-no.(%) | Yes | 11 (16) |
| | No | 58 (84) |

IMDC, International Metastatic Renal Cell Carcinoma Database Consortium (includes: Karnofsky performance status, time from diagnosis to treatment, hemoglobin, serum calcium concentration, neutrophil and platelet counts); ATB, Antibiotics

**Table 14. Patients' characteristics.** The clinical parameters relevant for the 69 RCC included in this study are described in this table (gender, age, antibiotics, disease status at diagnosis, responses, follow up).

When segregating responders (R) from non-responders (NR) in all 69 patients (according to the best clinical response as assessed by RECIST1.1), we failed to observe significant differences with a FDR of 10 %.

*GOMS associated with worse clinical outcome*

However, the best predictive model allowing to identify the "resistant" patients with AUC value of 0.882, a specificity of 0.778 (true positive cases) and a sensitivity (false positive cases) of 0.860 was obtained when considering LEfSe combination species, and the commensals retained in the model, that were increased in NR ("bad bacteria") were:

- *Clostridium boltae ATCC BAA 613* (fold increase 13)
- *Faecalibacterium prausnitzii KLE1255-3* (fold increase 4.4)
- *Prevotella sp CAG617* (detectable only in NR)
- *Prevotella copri CAG164* (detectable only in NR)
- *Subdoligranulum sp 4_3_54A2FAA* (fold increase 2.6)

while those that were decreased in NR ("good bacteria") were:

- *Bacteroides sp CAG 144* (decreased by 62 fold)
- *Acinetobacter CAG196* (decreased by 3.4 fold)
- *Eubacterium sp CAG38* (decreased by 2.3 fold)

The cut-off values correspond to the means±SEM featuring in Table 2. We kept only those species having a p value <0.05 for the final model, and claim the equilibrium between good and bad bacteria for the best outcome in this patent.

When segregating responders (R) from non-responders (NR) in RCC who did not take ATB (according to the best clinical response as assessed by RECIST1.1), we failed to observe significant differences with a FDR p value <0.05.

However, the best predictive model allowing to identify the "resistant" patients with AUC value of 0.879, a specificity of 0.707 (true positive cases) and a sensitivity (false positive cases) of 0.950 was obtained when considering LEfSe combination species, and the commensals retained in the model that were increased in NR ("bad bacteria") were:

- *Clostridium boltae ATCC BAA 613* (fold increase 15.7)
- *Prevotella sp CAG617* (detectable only in NR)

while those that were decreased in NR ("good bacteria") were:

- *Bacteroides sp CAG 144* (decreased by 50 fold)
- *Eubacterium sp CAG115* (decreased by 47 fold)
- *Eubacterium siraeum sp CAG80* (decreased by 14 fold)
- *Firmicutes bacterium sp. CAG65_1* (decreased by 1.9 fold)

The cut-off values correspond to the means±SEM featuring in Table 15. We kept only those species having a p value <0.05 for the final model. According to the present invention, the equilibrium between good and bad bacteria is critical for the best outcome.

## GOMS associated with best clinical outcome

The best predictive model allowing to identify the "responding" patients (R) among all patients with AUC value of 0.863, a specificity of 0.798 (true positive cases) and a sensitivity (false positive cases) of 0.842 was obtained when considering LEfSe combo species: the decrease of the following detrimental commensals retained in the model were:

- *Clostridium boltae ATCC BAA 613* (fold decrease 13)
- *Faecalibacterium prausnitzii KLE1255-3* (fold decrease 15)
- *Prevotella sp CAG617* (infini, detectable only in NR)
- *Subdoligranulum sp 4_3_54A2 FAA* (fold decrease 2.6)

while those that were increased in R ("good bacteria") were:

- *Bacteroides sp CAG 144* (increased by 62 fold)
- *Eubacterium sp CAG38* (increased by 2.3 fold)
- *Azospirillum sp CAG 239* (increased by 3.8 fold)

The cut-off values correspond to the means±SEM featuring in Table 15. We kept only these species (that had a p value <0.05) for the final model and claim the equilibrium between good and bad bacteria for the best outcome in this patent.

The best predictive model allowing to identify the "responding" patients (R) among those who did not take ATB with AUC value of 0.845, Spec=0.990; Sens=0.614 was obtained when considering LEfSe combo species, and the commensals retained in the model were:

Are decreased in R the following ones:

- *Prevotella_sp_GAG_617* (infini, absent in R)
- *Clostridium_clostridioforme_2_1_49FAA_1* (decreased by 6.3 fold in R)

Are increased in R the following ones:

- *Bacteroides plebeius CAG 211* (increased by *327* fold in R)
- *Bacteroides coprophilus DSM 18228 JCM 13818* (present only in R)
- *Eubacterium_siraeum_CAG_80* (increased by 14 fold in R)
- *Eubacterium_sp_CAG_202* (increased by 5.4 fold in R)
- *Eubacterium_sp_CAG_115* (increased by 47 fold in R)

The cut-off values correspond to the means±SEM featuring in Table 15. We kept only these species (that had a p value <0.05) for the final model. According to the present invention, the equilibrium between good and bad bacteria is critical for the best outcome.

**Table 15. Commensal bacteria associated with best (or worse) clinical outcome).** Part A includes all RCC patients while part B considered only patients who did not take antibiotics. The mean relative abundance with SEM (standard error mean) of each commensal is indicated, as well as the ratio of this mean comparing responders (R) versus non responders (NR) as the fold change and its log2 value with the p value (not considered significantly relevant when <0.05).

| Part A | Commensal bacteria associated with best (or worse) clinical outcome). | | | | | | | |
|---|---|---|---|---|---|---|---|---|
| NR --- KIDNEY / OUTCOME_1 / AUC=0,882; Spec=0,778; Sens=0,860 | | | | | | | | |
| Species | CAG | NR Mean rel, abund, | NR Sem | R Mean rel, abund, | R Sem | Fold Ratio (FR) R / NR | log2FR R / NR | P value NR vs R |
| Clostridium _bolteae_ ATCC_ BAA_ 613 | CAG00008 | 0,0069 | 0,00429 | 0,000531 | 0,000133 | 0,0769 | -3,7 | 0,00999 |
| Bacteroides_ sp__CAG_144 | CAG00413 | 2,90E-05 | 2,06E-05 | 0,00181 | 0,000585 | 62,55 | 5,96 | 0,0287 |
| Faecalibacterium_ cf__ prausnitzii_ KLE1255_ 3 | CAG01039 | 0,00301 | 0,00208 | 0,000687 | 0,000199 | 0,227 | -2,133 | 0,0351 |
| Eubacterium_sp__CAG_38 | CAG00178 | 0,0012 | 0,000685 | 0,0028 | 0,00072 | 2,336 | 1,224 | 0,0181 |
| Prevotella_ sp__CAG_617 | CAG00473 | 0,00326 | 0,00192 | 0 | 0 | 0 | -inf | 0,0296 |
| Subdoligranulum_sp__4_3_54A2FAA | CAG00140 | 0,00338 | 0,00087 | 0,00128 | 0,000195 | 0,379 | -1,398 | 0,0204 |
| Prevotella _copri_CAG_164 | CAG01004 | 0,000576 | 0,000558 | 0 | 0 | 0 | -inf | 0,0296 |
| Acinetobacter_sp__CAG_196 | CAG00761 | 0,000557 | 0,000417 | 0,00191 | 0,000695 | 3,44 | 1,782 | 0,0186 |
| Azospirillum_sp__CAG_239 | CAG00302 | 0,000452 | 0,000331 | 0,00172 | 0,00083 | 3,8 | 1,926 | 0,0501 |
| R --- KIDNEY / OUTCOME_1 / AUC=0,863; Spec=0,798; Sens=0,842 | | | | | | | | |
| Species | CAG | NR Mean rel, abund, | NR Sem | R Mean rel, abund, | R Sem | Fold Ratio (FR) R / NR | log2FR R / NR | P value NR vs R |
| Clostridium_ bolteae_ ATCC_ BAA_ 613 | CAG00008 | 0,0069 | 0,00429 | 0,000531 | 0,000133 | 0,0769 | -3,7 | 0,00999 |
| Bacteroides_ sp__CAG_144 | CAG00413 | 2,90E-05 | 2,06E-05 | 0,00181 | 0,000585 | 62,55 | 5,96 | 0,0287 |
| Faecalibacterium_ cf_ _prausnitzii_ KLE1255_ 3 | CAG01039 | 0,00301 | 0,00208 | 0,000687 | 0,000199 | 0,227 | -2,133 | 0,0351 |
| Eubacterium_ sp__CAG_38 | CAG00178 | 0,0012 | 0,000685 | 0,0028 | 0,00072 | 2,336 | 1,224 | 0,0181 |
| Prevotella _sp__CAG_617 | CAG00473 | 0,00326 | 0,00192 | 0 | 0 | 0 | -inf | 0,0296 |
| Subdoligranulum_sp__4_3_54A2FAA | CAG00140 | 0,00338 | 0,00087 | 0,00128 | 0,000195 | 0,379 | -1,398 | 0,0204 |
| Azospirillum_sp__CAG 239 | CAG00302 | 0,000452 | 0,000331 | 0,00172 | 0,00083 | 3,8 | 1,926 | 0,0501 |

(continued)

| R --- KIDNEY / OUTCOME_1 / AUC=0,863; Spec=0,798; Sens=0,842 | | | | | | | | |
|---|---|---|---|---|---|---|---|---|
| Species | CAG | NR Mean rel, abund, | NR Sem | R Mean rel, abund, | R Sem | Fold Ratio (FR) R / NR | log2FR R / NR | P value NR vs R |
| | | | | | | | | |
| Part B | Commensal bacteria associated with best (or worse) clinical outcome. | | | | | | | |
| NR --- KIDNEY_noatb / OUTCOME_1 / AUC=0,879; Spec=0,707; Sens=0,950 | | | | | | | | |
| Species | CAG | NR Mean rel, abund, | NR Sem | R Mean rel, abund, | R Sem | Fold Ratio (FR) R / NR | log2FR R / NR | P value NR vs R |
| Clostridium_ bolteae_ ATCC_ BAA_ 613 | CAG00008 | 0,00806 | 0,00605 | 0,000513 | 0,000142 | 0,0636 | -3,972 | 0,00974 |
| Bacteroides_ _sp__CAG 144 | CAG00413 | 3,88E-05 | 2,91 E-05 | 0,00195 | 0,000625 | 50,203 | 5,649 | 0,0368 |
| Firmicutes_bacterium_ CAG_65_1 | CAG00792 | 0,00413 | 0,00234 | 0,00798 | 0,00259 | 1,931 | 0,949 | 0,0395 |
| Eubacterium_ _sp__CAG 115 | CAG00341 | 5,33E-05 | 3,66E-05 | 0,00252 | 0,000927 | 47,381 | 5,566 | 0,0148 |
| Prevotella_ sp__CAG_617 | CAG00473 | 0,00378 | 0,00261 | 0 | 0 | 0 | -inf | 0,0436 |
| Bacteroides_plebeius_ CAG_211 | CAG00711 | 1,66E-05 | 1,66E-05 | 0,00544 | 0,00195 | 327,612 | 8,355 | 0,0557 |
| Eubacterium_ siraeum_CAG_80 | CAG00653 | 0,000425 | 0,000185 | 0,00608 | 0,00215 | 14,277 | 3,835 | 0,0232 |
| Bacteroides_ coprophilus_DSM_ 18228_ JCM_ 13818 | CAG00132 | 0 | 0 | 0,00234 | 0,00121 | 0 | -inf | 0,0533 |
| R --- KIDNEY_noatb / OUTCOME_1 / AUC=0,845; Spec=0,990; Sens=0,614 | | | | | | | | |
| Species | CAG | NR Mean rel, abund, | NR Sem | R Mean rel, abund, | R Sem | Fold Ratio (FR) R / NR | log2FR R / NR | P value NR vs R |
| Eubacterium_ _sp__CAG_115 | CAG00341 | 5,33E-05 | 3,66E-05 | 0,00252 | 0,000927 | 47,381 | 5,566 | 0,0148 |
| Prevotella_ sp__CAG_617 | CAG00473 | 0,00378 | 0,00261 | 0 | 0 | 0 | -inf | 0,0436 |
| Clostridium_clostridioforme_ 2_1_49FAA_1 | CAG01263 | 0,0053 | 0,00356 | 0,000839 | 0,000471 | 0,158 | -2,659 | 0,0415 |
| Bacteroides_plebeius_ CAG_211 | CAG00711 | 1,66E-05 | 1,66E-05 | 0,00544 | 0,00195 | 327,612 | 8,355 | 0,0557 |
| Eubacterium_ siraeum_CAG_80 | CAG00653 | 0,000425 | 0,000185 | 0,00608 | 0,00215 | 14,277 | 3,835 | 0,0232 |
| Eubacterium_sp__CAG_202 | CAG00796 | 0,000954 | 0,00095 | 0,00514 | 0,00162 | 5,39 | 2,43 | 0,0402 |

(continued)

| | | R --- KIDNEY_noatb / OUTCOME_1 / AUC=0,845; Spec=0,990; Sens=0,614 | | | | | | |
|---|---|---|---|---|---|---|---|---|
| Species | CAG | NR Mean rel, abund, | NR Sem | R Mean rel, abund, | R Sem | Fold Ratio (FR) R / NR | log2FR R / NR | P value NR vs R |
| Bacteroides_coprophilus_DSM_18228_JCM_13818 | CAG00132 | 0 | 0 | 0,00234 | 0,00121 | 0 | -inf | 0,0533 |

**Example 2: Metagenomics-based GOMS associated with TTP>3 months according to RECIST 1.1 criteria (Figure 2).**

Metagenomics-based GOMS associated with TTP>3 months according to RECIST 1.1 criteria (Figure 2).

When segregating responders (R, TTP>3 months) from non-responders (NR, TTP<3 months) in all 69 patients (according to the best clinical response as assessed by RECIST1.1), we failed to observe significant differences with a FDR p value <0.05. When segregating responders (R, TTP>3 months) from non-responders (NR, TTP<3 months) in RCC who did not take ATB (according to the best clinical response as assessed by RECIST1.1), we failed to observe significant differences with a FDR p value <0.05.

However, the multivariate model allowed to find some GOMS with clinical relevance.

*GOMS associated with TTP<3 months*

Resistance at 3 months was the best clinical readout of our analysis in RCC patients.

The best predictive model allowing to identify the "resistant" patients (NR) among all patients with AUC value of 0.913, a specificity of 0.798 (true positive cases) and a sensitivity (false positive cases) of 0.910 was obtained when considering LEfSe combination, and the commensals retained in the model as "bad bacteria" increased in NR were the following, as shown in Table 16:

- *Glostridium_sp_GAG_7_1* (increased by 2 fold in NR)
- *Hungatella_hathewayi_12489931_1* (increased by 47 fold in NR)
- *Prevotella_sp_GAG_617* (infinite, detectable only in NR) and
- *Holdemanella_biformis_DSM_3989_1* (increased by 48 fold in NR),

whereas in *Azospirillum_sp_GAG_239* was decrease by 11 fold in NR (good bacterium)

However, in patients who did not took ATB, the list of bacteria retained in this model which gave an AUC of 0.884, specificity 0.667, sensitivity 1.000 were:

For the bad bacteria, increase of:

- *Clostridium_bolteae_ATCC_BAA_613* (increased by 15 fold in NR)
- *Prevotella_sp_GAG_617* (present only in NR)
- *Holdemanella_biformis_DSM_3989_1* (increased by 32 fold in NR) and
- Faecalibacterium_cf_prausnitzii_KLE1255 (increased by 8.8 fold in NR)

For the good bacteria, decrease of:

- *Eubacterium_sp_CAG_38* (decreased by 2 fold in NR)
- *Eubacterium_sp_GAG_115* (decreased by 37 fold in NR)

We did retain the ratio between good and bad bacteria for TTP3 NR in our final model (see Table 16, part B).

*GOMS associated with TTP>3 months*

The best predictive model allowing to identify the "responders" patients (R) among all patients, with AUC=0.876; Spec=0.990; Sens=0.727, was obtained when considering LEfSe combination, and the commensals retained in the model as "bad bacteria" decreased in R were the following, as shown in Table 16:

- *Clostridium_bolteae_ATCC_BAA_613* (decreased by 92% in R)
- *Glostridium_sp_GAG_7_1* (decreased by 46%)
- *Faecalibacterium_cf_prausnitzii_KLE1255_3* (decreased by 83%)
- *Prevotella_sp_GAG_617* (present only in NR)
- *Holdemanella_biformis_DSM_3989_1* (decreased by 79%)
- *Peptoniphilus_sp_oral_taxon_836_str_F0141* (present only in NR)

whereas *Azospirillum_sp_GAG_239* was increased in R by 11 fold (good bacterium).

However, in patients who did not took ATB, the list of bacteria retained in this model which gave AUC=0.899; Spec=0.990; Sens=0.836 were:

For the bad bacteria, a decrease in:

- *Clostridium_bolteae_ATCC_BAA_613* (decreased by 93.2 % in R)
- *Glostridium_sp_GAG_7_1* (decreased by 44.5%)
- *Prevotella_sp_GAG_617* (present only in NR)
- *Prevotella_copri_CAG_164* (present only in NR)
- *Holdemanella_biformis_DSM_3989_1* (decreased by 96.9%)
- *Prevotella_sp_GAG_279_1* (present only in NR)
- *Faecalibacterium_cf_prausnitzii_KLE1255* (decreased by 88.6%)

For the good bacteria, an increase in:

- *Akkermansia_muciniphila_GAG_154* (2.5 fold increase in R)
- *Eubacterium_sp_GAG_115* (37 fold increase in R)

We did retain the ratio between good and bad bacteria for TTP3 R in our final model (see Table 16, part B).

**Table 16. Commensal bacteria associated with TTP> or <3 months.** Part A includes all RCC patients while part B considered only patients who did not take antibiotics. The mean relative abundance with SEM (standard error mean) of each commensal is indicated, as well as the ratio of this mean comparing responders (R) versus non responders (NR) as the fold change and its log2 value with the p value (not considered significantly relevant when <0.05).

| Part A | Commensal bacteria associated with TTP> or <3 months. | | | | | | | |
|---|---|---|---|---|---|---|---|---|
| **NR --- KIDNEY / TTP3 / AUC=0,913; Spec=0,798; Sens=0,910** | | | | | | | | |
| Species | CAG | NR Mean rel, abund, | NR Sem | R Mean rel, abund, | R Sem | Fold Ratio (FR) R / NR | log2FR R / NR | P value NR vs R |
| Clostridium_sp__CAG_7_1 | CAG00047 | 0,0031 | 0,000679 | 0,00166 | 0,000576 | 0,537 | -0,895 | 0,00404 |
| Hungatella_hathewayi_ 12489931_1 | CAG00610 | 0,000963 | 0,000659 | 2,15E-05 | 9,31E-06 | 0,0223 | -5,483 | 0,0118 |
| Prevotella_ sp__CAG_617 | CAG00473 | 0,004 | 0,00233 | 0 | 0 | 0 | -inf | 0,0107 |
| Holdemanella_biformis DSM_ 3989_1 | CAG01141 | 0,00249 | 0,0013 | 5,19E-05 | 5,19E-05 | 0,0208 | -5,586 | 0,0154 |
| Azospirillum_sp__CAG_239 | CAG00302 | 0,000157 | 0,00011 | 0,00172 | 0,00076 | 10,914 | 3,448 | 0,0454 |
| **R --- KIDNEY / TTP3 / AUC=0,876; Spec=0,990; Sens=0,727** | | | | | | | | |
| Species | CAG | NR Mean rel, abund, | NR Sem | R Mean rel, abund, | R Sem | Fold Ratio (FR) R / NR | log2FR R / NR | P value NR vs R |
| Clostridium_bolteae_ATCC_BAA_ 613 | CAG00008 | 0,00813 | 0,00525 | 0,000635 | 0,000163 | 0,0781 | -3,678 | 0,0387 |
| Clostridium_sp__CAG_7_1 | CAG00047 | 0,0031 | 0,000679 | 0,00166 | 0,000576 | 0,537 | -0,895 | 0,00404 |
| Faecalibacterium_ cf_ _prausnitzii_ KLE1255 3 | CAG01039 | 0,00363 | 0,00254 | 0,000645 | 0,00018 | 0,177 | -2,494 | 0,0242 |
| Prevotella_ sp__CAG_617 | CAG00473 | 0,004 | 0,00233 | 0 | 0 | 0 | -inf | 0,0107 |
| Holdemanella_biformis_ DSM_ 3989_1 | CAG01141 | 0,00249 | 0,0013 | 5,19E-05 | 5,19E-05 | 0,0208 | -5,586 | 0,0154 |
| Azospirillum_sp_CAG_239 | CAG00302 | 0,000157 | 0,00011 | 0,00172 | 0,00076 | 10,914 | 3,448 | 0,0454 |
| Peptoniphilus_ sp__oral_taxon_ 836_str__ F0141 | CAG01339 | 6,19E-06 | 5.00E-06 | 0 | 0 | 0 | -inf | 0,0393 |

(continued)

**Part B** — Commensal bacteria associated with TTP> or <3 months.

### NR --- KIDNEY_noatb / TTP3 / AUC=0,884; Spec=0,667; Sens=1,000

| Species | CAG | NR Mean rel, abund, | NR Sem | R Mean rel, abund, | R Sem | Fold Ratio (FR) R / NR | log2FR R / NR | P value NR vs R |
|---|---|---|---|---|---|---|---|---|
| Clostridium_bolteae_ATCC_BAA_613 | CAG00008 | 0,00919 | 0,00718 | 0,000619 | 0,000181 | 0,0673 | -3,892 | 0,0242 |
| Eubacterium_sp_CAG_38 | CAG00178 | 0,00141 | 0,00112 | 0,00281 | 0,000721 | 1,998 | 0,998 | 0,011 |
| Eubacterium_sp__CAG_115 | CAG00341 | 6,32E-05 | 4,32E-05 | 0,00234 | 0,000866 | 37,05 | 5,211 | 0,0472 |
| Prevotella_sp_CAG_617 | CAG00473 | 0,00449 | 0,00308 | 0 | 0 | 0 | -inf | 0,0223 |
| Holdemanella_biformis_DSM_3989_1 | CAG01141 | 0,00187 | 0,00102 | 5,81 E-05 | 5,81 E-05 | 0,031 | -5,009 | 0,0265 |
| Faecalibacterium_cf_prausnitzii_KLE1255 | CAG00495 | 0,00342 | 0,00215 | 0,00039 | 0,000277 | 0,114 | -3,132 | 0,0397 |

### R --- KIDNEY_noatb / TTP3 / AUC=0,899; Spec=0,990; Sens=0,836

| Species | CAG | NR Mean rel, abund, | NR Sem | R Mean rel, abund, | R Sem | Fold Ratio (FR) R / NR | log2FR R / NR | P value NR vs R |
|---|---|---|---|---|---|---|---|---|
| Clostridium_bolteae_ATCC_BAA_613 | CAG00008 | 0,00919 | 0,00718 | 0,000619 | 0,000181 | 0,0673 | -3,892 | 0,0242 |
| Clostridium_sp__CAG_7_1 | CAG00079 | 0,00319 | 0,000675 | 0,00177 | 0,000642 | 0,557 | -0,843 | 0,00285 |
| Akkermansia_muciniphila_CAG_154 | CAG00301 | 0,00228 | 0,00209 | 0,00571 | 0,00159 | 2,495 | 1,319 | 0,016 |
| Eubacterium_sp__CAG_115 | CAG00341 | 6,32E-05 | 4,32E-05 | 0,00234 | 0,000866 | 37,05 | 5,211 | 0,0472 |
| Prevotella sp_CAG_617 | CAG00473 | 0,00449 | 0,00308 | 0 | 0 | 0 | -inf | 0,0223 |
| Prevotella copri_CAG_164 | CAG01004 | 0,000972 | 0,000941 | 0 | 0 | 0 | -inf | 0,00461 |
| Holdemanella_biformis DSM_3989_1 | CAG01141 | 0,00187 | 0,00102 | 5,81 E-05 | 5,81 E-05 | 0,031 | -5,009 | 0,0265 |
| Prevotella sp_CAG_279_1 | CAG00487 | 0,000701 | 0,000568 | 0 | 0 | 0 | -inf | 0,0223 |
| Faecalibacterium_cf_prausnitzii_KLE1255 | CAG00495 | 0,00342 | 0,00215 | 0,00039 | 0,000277 | 0,114 | -3,132 | 0,0397 |

**Example 3: Metagenomics-based GOMS associated with TTP>6 months according to RECIST 1.1 criteria (Figure 3).**

When segregating responders (R, TTP>6 months) from non-responders (NR, TTP<6 months) in all RCC patients (according to the TTP6 as assessed by RECIST1.1), we did not observe significant differences with a FDR p value <0.05 (not shown). When segregating responders (R, TTP>6 months) from non-responders (NR, TTP<6 months) in RCC who did not take ATB (according to the TTP6 as assessed by RECIST1.1), we observed significant differences with a FDR p value <0.05 (Figure 4). Of note, *Subdoligranulum_sp_CAG_314_1* and *Eubacterium_sp_CAG_115* were associated with TTP > 6 months in RCC patients who took no antibiotics.

We conducted the multivariate model allowing to find very robust GOMS with clinical relevance.

*GOMS associated with TTP>6 months*

The best predictive model allowing to identify the "responding" patients (R) among all patients with AUC value of 0.845, a specificity of 0.889 (true positive cases) and a sensitivity (false positive cases) of 0.800 was obtained when considering LEfSe combination, and the good commensals retained in the model were the following, as shown in Table 17 (part A):

- *Clostridium_sp_CAG_230* (increased by 6.7 fold in R)
- *Clostridium_sp_CAG_167* (increased by 2.8 fold in R)
- *Azospirillum_sp_CAG_239_3* (detectable only in R)
- *Firmicutes_bacterium_CAG_176_12* (increased by 6.9 fold in R)

The bad commensals that should be decreased are:

- *Coprococcus_catus_GD_7_6* (decreased by 65.6%)
- *Oscillibacter_sp_KLE_1745_6* (decreased by 56.6%)

The cut-off values correspond to the means±SEM featuring in Table 17.

However, in patients who did not took ATB, the list of favorable bacteria retained in this model was different and included the following ones, reaching AUC value of 0.804, a specificity of 0.848 (true positive cases) and a sensitivity (false positive cases) of 0.770 was obtained when considering LEfSe combination (Table 17 part B):

- *Eubacterium_sp_CAG_180_1* (increased by 3 fold in R)
- *Eubacterium_sp_CAG_115* (increased by 13 fold in R)
- *Eubacterium_siraeum_CAG_80* (increased by 5.4 fold in R)
- *Butyrivibrio_crossotus_DSM_2876* (detectable only in R)

The cut-off values correspond to the means±SEM featuring in Table 17. These favorable and unfavorable commensals are major spp influencing favorable responses to PD1 blockade.

*GOMS associated with TTP<6 months*

As for the bacteria associated with short TTP<6 months in all patients, the list of significant bacteria was very restrained even more without ATB. Very few can be found significantly associated with resistance:

- *Clostridiales_bacterium_VE202_14_1* (undetectable in NR)
- *Clostridium_sp_CAG_230* (6.7 fold increase in R)
- *Azospirillum_sp_CAG 239_1* (undetectable in NR).

**Table 17. Commensal bacteria associated with TTP> or <6 months.** Part A includes all RCC patients while part B considered only patients who did not take antibiotics. The mean relative abundance with SEM (standard error mean) of each commensal is indicated, as well as the ratio of this mean comparing responders (R) versus non responders (NR) as the fold change and its log2 value with the p value (not considered significantly relevant when <0.05).

| Part A | Commensal bacteria associated with TTP> or <6 months. | | | | | | | |
|---|---|---|---|---|---|---|---|---|
| **NR --- KIDNEY /TTP6 / AUC=0,796; Spec=0,828; Sens=0,688** | | | | | | | | |
| Species | CAG | NR Mean rel, abund, | NR Sem | R Mean rel, abund, | R Sem | Fold Ratio (FR) R / NR | log2FR R/NR | P value NR vs R |
| Clostridium_bolteae_ ATCC_BAA_613 | CAG00008 | 0,00477 | 0,00285 | 0,000508 | 0,000154 | 0,106 | -3,229 | 0,0623 |
| Alistipes_sp_CAG_ 514_1 | CAG00827 | 0 | 0 | 0,00618 | 0,00562 | 0 | -inf | 0,0714 |
| Clostridiales_bacterium_ VE202_14_1 | CAG00821 | 0 | 0 | 1,88E-05 | 1,13E-05 | 0 | -inf | 0,0254 |
| Clostridium_sp_CAG_ 230 | CAG00317 | 9,10E-05 | 6,01 E-05 | 0,000612 | 0,000309 | 6,733 | 2,751 | 0,00989 |
| Azospirillum_sp_CAG_ 239_1 | CAG00472 | 0 | 0 | 0,000412 | 0,000328 | 0 | -inf | 0,0714 |
| Azospirillum_sp_CAG_ 239_3 | CAG00668 | 0 | 0 | 0,000816 | 0,000574 | 0 | -inf | 0,0254 |
| Alistipes_sp_CAG_268 | CAG00421 | 0 | 0 | 0,002 | 0,00193 | 0 | -inf | 0,0714 |
| **R --- KIDNEY / TTP6/AUC=0,845; Spec=0,889; Sens=0,800** | | | | | | | | |
| Species | CAG | NR Mean rel, abund, | NR Sem | R Mean rel, abund, | R Sem | Fold Ratio (FR) R/NR | log2FR R/NR | P value NR vs R |
| Coprococcus_catus_ GD_7_6 | CAG01208 | 0,000926 | 0,000278 | 0,000318 | 9,32E-05 | 0,343 | -1,541 | 0,103 |
| Clostridium_sp_CAG_ 230 | CAG00317 | 9,10E-05 | 6,01 E-05 | 0,000612 | 0,000309 | 6,733 | 2,751 | 0,00989 |
| Clostridium_sp_CAG_ 167 | CAG00452 | 0,000271 | 0,000164 | 0,000781 | 0,00029 | 2,878 | 1,525 | 0,0277 |
| Oscillibacter_sp_KLE_ 1745_6 | CAG00931 | 0,000795 | 0,000203 | 0,000345 | 7,49E-05 | 0,433 | -1,204 | 0,0367 |

| R --- KIDNEY / TTP6/AUC=0,845; Spec=0,889; Sens=0,800 | | | | | | | | |
|---|---|---|---|---|---|---|---|---|
| Species | CAG | NR Mean rel, abund, | NR Sem | R Mean rel, abund, | R Sem | Fold Ratio (FR) R/NR | log2FR R/NR | P value NR vs R |
| Azospirillum_sp_CAG_239_3 | CAG00668 | 0 | 0 | 0,000816 | 0,000574 | 0 | -inf | 0,0254 |
| Firmicutes_bacterium_CAG_176_12 | CAG01043 | 5,85E-05 | 5,85E-05 | 0,000404 | 0,00025 | 6,912 | 2,789 | 0,0501 |
| | | | | | | | | |
| Part B | Commensal bacteria associated with TTP> or <6 months. | | | | | | | |
| NR --- KIDNEY_noatb/TTP61 AUC=0,721; Spec=0,798; Sens=0,606 | | | | | | | | |
| Species | CAG | NR Mean rel, abund, | NR Sem | R Mean rel, abund, | R Sem | Fold Ratio (FR) R / NR | log2FRR/NR | P value NR vs R |
| Firmicutes_bacterium_CAG_110_1 | CAG00604 | 0,00166 | 0,000682 | 0,00201 | 0,000735 | 1,212 | 0,277 | 0,207 |
| R --- KIDNEY_noatb / TTP6 / AUC=0,804; Spec=0,848; Sens=0,770 | | | | | | | | |
| Species | CAG | NR Mean rel, abund, | NR Sem | R Mean rel, abund, | R Sem | Fold Ratio (FR) R / NR | log2FR R/NR | P value NR vs R |
| Eubacterium_sp_CAG_180_1 | CAG00523 | 0,00221 | 0,00124 | 0,00672 | 0,00195 | 3,039 | 1,603 | 0,0272 |
| Eubacterium_sp_CAG_115 | CAG00341 | 0,000274 | 0,000241 | 0,00355 | 0,0015 | 12,961 | 3,696 | 0,000501 |
| Eubacterium_siraeum_CAG_80 | CAG00653 | 0,00161 | 0,00095 | 0,00872 | 0,00348 | 5,396 | 2,431 | 0,0252 |
| Butyrivibrio_crossotus_DSM_2876 | CAG00542 | 0 | 0 | 0,0136 | 0,00801 | 0 | -inf | 0,0324 |

**Example 4: Results for RCC patients regardless antibiotic usage, OUTCOME 1 criterion**

In this example, three different models were built based on a bundle of 69 "reference patterns", which are the standardized abundances profiles (zero mean and unit variance) obtained from the 69 samples used in training the logistic regression models. Standardization is necessary to homogenize the dynamic range of bacterial species abundances.

Based on the first strategy described in the Materials and Methods, applied to 69 RCC patients (NR = 27; R = 42) with unknown antibiotic usage history and OUTCOME_1 criterion (best outcome), we selected 25 bacterial species that gave a logistic regression model overall predictability equal to 89.86% (model 1.1). In order to measure the goodness of the model with 25 species, after a 5-fold cross-validation performed on a subset of the original dataset, the model itself was able to predict 60% of NR and 69% of R subjects, with a CV_AUC = 0.917 and an AUC = 0.785. Upon RFECV and RFE feature selection, 8 species were selected from the aforementioned 25: the model built on these 8 species gave an overall predictability of 79.71% (model 1.2, Figure 5), and the summary statistics for these species are reported in Table 18 below. In order to measure the goodness of the model, after a 5-fold cross-validation performed on a subset of the original dataset, the model itself was able to predict 40% of NR and 77% of R subjects, with a CV_AUC = 0.635 and an AUC = 0.815.

Based on the second strategy (machine-learning) applied to 69 RCC patients (NR = 27; R = 42) with unknown antibiotic usage history and OUTCOME_1 criterion, we took into account all the bacterial species of the dataset (n = 1112). After a first round of feature selection, RFECV gave a rank = 1 to 31 species which collectively, after a 5-fold cross-validation, were able to predict 100% of NR and 100% of R subjects, with a CV_AUC = 1 and an AUC = 1 (model 2.1). Upon a second round of feature selection, RFE gave a rank = 1 to 8 species, which were selected for the subsequent logistic regression classifier. The model built on these 8 species gave an overall predictability of 89.86% (model 2.2, Figure 5), and the summary statistics for these species are reported in Table 19 below. In order to measure the goodness of the model, after a 5-fold cross-validation performed on a subset of the original dataset, the model itself was able to predict 80% of NR and 92% of R subjects, with a CV_AUC = 1 and an AUC = 0.923.

**Table 18**: Statistics for the species selected in Model 1.2.

| Group | op. | Thr | NR (n=27) (%) | R (n=42) (%) | Pval_Fisher | NR_coeff | NR_Odds Ratio | R_coeff | R_Odds Ratio | rank_R FECV | rank_RFE | NR Mean relabund | NR Sem | R Mean relabund | R Sem | Fold Ratio (FR) R/NR | log2FR R / NR | P value NR vs R |
|---|---|---|---|---|---|---|---|---|---|---|---|---|---|---|---|---|---|---|
| CAG00008_Clostridium_bolteae_ATCC_BAA_613 | > | 0 | 92.6 | 95.2 | 0.6415 | 1.0323 | 2.0453 | **-1.0323** | 0.4889 | 1 | 1 | 0.0069045 | 0.00429957 | 0.00053101 | 0.00013359 | 0.08 | -3.7 | 0.00999 |
| CAG01039_Faecalibacterium_cf_prausnitzii_KLE1255 | > | 0 | 77.8 | 47.6 | 0.0229 | 1.084 | 2.1199 | **-1.084** | 0.4717 | 1 | 1 | 0.00301567 | 0.0020844 | 0.00068723 | 0.00019997 | 0.23 | -2.13 | 0.03517 |
| CAG00473_Prevotella_sp_CAG_617 | > | 0 | 11.1 | 0.0 | 0.0558 | 0.9723 | 1.9619 | **-0.9723** | 0.5097 | 1 | 1 | 0.00326379 | 0.00192 | 0.0 | 0.0 | 0.0 | -inf | 0.02965 |
| CAG00140_Subdoligranulum_sp_4_3_54A2_FAA | > | 0 | 92.6 | 100.0 | 0.1496 | 0.9907 | 1.9872 | **-0.9907** | 0.5032 | 1 | 1 | 0.00338708 | 0.00087016 | 0.0012847 | 0.00019541 | 0.38 | -1.4 | 0.02047 |
| CAG00610_Hungatella_hathewayi_12489931 | > | 0 | 51.9 | 21.4 | 0.0174 | 0.6234 | 1.5406 | **-0.6234** | 0.6491 | 1 | 1 | 0.00079124 | 0.00053962 | 2.023e-05 | 1.029e-05 | 0.03 | -5.29 | 0.00354 |

(continued)

| Group | op. | Thr | NR (n=27) (%) | R (n=42) (%) | Pval_Fisher | NR_coeff | NR_Odds Ratio | **R_coeff** | R_Odds Ratio | rank_R FECV | rank_RFE | NR Mean relabund | NR Sem | R Mean relabund | R Sem | Fold Ratio (FR) R/NR | log2FR R / NR | P value NR vs R |
|---|---|---|---|---|---|---|---|---|---|---|---|---|---|---|---|---|---|---|
| CAG01141_Holdemanella_biformis_DSM_3989 | > | 0 | 14.8 | 2.4 | 0.0728 | 0.8468 | 1.7986 | **-0.8468** | 0.556 | 1 | 1 | 0.00203209 | 0.00107945 | 5.809e-05 | 5.809e-05 | 0.03 | -5.13 | 0.0489 |
| CAG00413_Bacteroides_sp_CAG_144 | > | 0 | 11.1 | 31.0 | 0.0803 | -0.8994 | 0.5361 | **0.8994** | 1.8653 | 1 | 1 | 2.895e-05 | 2.056e-05 | 0.0018108 | 0.00058512 | 62.56 | 5.97 | 0.02879 |
| CAG00317_Clostridium_sp_CAG_230 | > | 0 | 11.1 | 35.7 | 0.027 | -0.6041 | 0.6579 | **0.6041** | 1.52 | 1 | 1 | 2.568e-05 | 1.459e-05 | 0.00047284 | 0.00019556 | 18.41 | 4.2 | 0.0214 |

**Table 19:** Statistics for the species selected in Model 2.2.

| Group | op. | Thr | (n=27) (%) | R (n=42) (%) | Pval_ Fisher | NR_coeff | NR_ Odds Ratio | R_coeff | R_ Odds Ratio | rank_R FECV | rank_ R FE | NR Mean relabund | NR Sem | R Mean re-labund | R Sem | FRR/ NR | log2FR R / NR | P value NR vs R |
|---|---|---|---|---|---|---|---|---|---|---|---|---|---|---|---|---|---|---|
| CAG00727_Eggerthella_lenta_DSM_2243 | > | 0 | 77.8 | 50.0 | 0.0251 | 1.0791 | 2.1127 | **-1.0791** | 0.4733 | 1 | 1 | 0.00062355 | 0.00025745 | 9.683e-05 | 2.923e-05 | 0.16 | -2.69 | 0.00559 |
| CAG01141_Holdemanella_biformis_DSM_3989 | > | 0 | 14.8 | 2.4 | 0.0728 | 1.1164 | 2.1681 | **-1.1164** | 0.4612 | 1 | 1 | 0.00203209 | 0.00107945 | 5.809e-05 | 5.809e-05 | 0.03 | -5.13 | 0.0489 |
| CAG00650_Dorea_formicigenerans_ATCC_27755 | > | 0 | 81.5 | 88.1 | 0.4972 | 1.2839 | 2.4349 | **-1.2839** | 0.4107 | 1 | 1 | 0.00113438 | 0.0003259 | 0.00041466 | 6.136e-05 | 0.37 | -1.45 | 0.18765 |
| CAG00928_Acidiphilium_sp_CAG_727 | > | 0 | 14.8 | 4.8 | 0.2006 | 1.1156 | 2.1668 | **-1.1156** | 0.4615 | 1 | 1 | 0.00021637 | 0.00013333 | 5.87e-06 | 4.1e-06 | 0.03 | -5.2 | 0.14111 |
| CAG00473_Prevotella_sp_CAG_617 | > | 0 | 11.1 | 0.0 | 0.0558 | 1.014 | 2.0194 | **-1.014** | 0.4952 | 1 | 1 | 0.00326379 | 0.00192 | 0.0 | 0.0 | 0.0 | -inf | 0.02965 |
| CAG00122_Coprococcus_catus_GD_7 | > | 0 | 11.1 | 0.0 | 0.0558 | 0.9394 | 1.9177 | **-0.9394** | 0.5214 | 1 | 1 | 0.00015959 | 0.00010288 | 0.0 | 0.0 | 0.0 | -inf | 0.02965 |

(continued)

| Group | op. | Thr | (n=27) (%) | R (n=42) (%) | Pval_ Fisher | NR_coeff | NR_ Odds Ratio | R_coeff | R_ Odds Ratio | rank_R FECV | rank_ R FE | NR Mean relabund | NR Sem | R Mean re-labund | R Sem | FRR/ NR | log2FR R / NR | P value NR vs R |
|---|---|---|---|---|---|---|---|---|---|---|---|---|---|---|---|---|---|---|
| CAG01144_Lactobacillus_vaginalis_ DSM_ 5837_ATCC _49540 | > | 0 | 7.4 | 0.0 | 0.1496 | 0.8085 | 1.7514 | **-0.8085** | 0.571 | 1 | 1 | 4.76e-06 | 3.39e-06 | 0.0 | 0.0 | 0.0 | -inf | 0.07916 |
| CAG00063__Barnesiella_ viscericola_ DSM_ 18177 | > | 0 | 7.4 | 0.0 | 0.1496 | 0.7679 | 1.7028 | **-0.7679** | 0.5873 | 1 | 1 | 0.00049676 | 0.00035498 | 0.0 | 0.0 | 0.0 | -inf | 0.07916 |

Taking into account the bacterial species (n = 181, above 1112 total species, 16.3% of the total microbial composition) having an average relative abundance greater than or equal to 0.001 if expressed in the interval [0 : 1] (thus, the 0.1%) within the overall RCC cohort, we applied the second strategy (machine-learning) for 69 RCC patients (NR = 27; R = 42) with unknown antibiotic usage history and OUTCOME_1 criterion. After a first round of feature selection, RFECV gave a rank = 1 to 21 species which collectively, after a 5-fold cross-validation, were able to predict 100% of NR and 92% of R subjects, with a CV_AUC = 0.573 and an AUC = 1 (model 3.1). After a second round of feature selection, RFE gave a rank = 1 to 8 species which were selected for the subsequent logistic regression classifier. The model built on these 8 species gave an overall predictability of 85.51% (model 3.2, Figure 5), and the summary statistics for these species are reported in Table 20 below. In order to measure the goodness of the model, after a 5-fold cross-validation performed on a subset of the original dataset, the model itself was able to predict 80% of NR and 85% of R subjects, with a CV_AUC = 0.635 and an AUC = 0.831. This last model was designed to be more affordable in case of problems in fecal samples storage, sequencing, stiffness, etcetera.

According to the Models 1.2, 2.2 and 3.2, which were modeled on unknown antibiotic usage history and OUTCOME_1 criterion, and are all based on 8 bacterial species so they can be properly compared, it is shown that the Model 2.2 has the highest predictability (89.86%) with the highest CV_AUC and AUC.

**Table 20:** Statistics for the species selected in Model 3.2.

| Group | op. | Thr | NR (n=27) (%) | R (n=42) (%) | Pval_Fisher | NR_co-eff | NR_OddsRatio | R_co-eff | R_Odds Ratio | rank_RFE CV | rank_RFE | NR Mean relabund | NR Sem | R Mean relabund | R Sem | FR R/NR | log2FR R / NR | P value NR vs R |
|---|---|---|---|---|---|---|---|---|---|---|---|---|---|---|---|---|---|---|
| CAG00243_Lachnospiraceae_bacterium_1_1_57FAA | > | 0 | 85.2 | 90.5 | 0.7024 | 1.1735 | 2.2555 | **-1.1735** | 0.4434 | 1 | 1 | 0.00861298 | 0.00346076 | 0.00153833 | 0.00031904 | 0.18 | -2.49 | 0.26546 |
| CAG00473_Prevotella_sp_CAG_617 | > | 0 | 11.1 | 0.0 | 0.0558 | 1.027 | 2.0378 | **-1.027** | 0.4907 | 1 | 1 | 0.00326379 | 0.00192 | 0.0 | 0.0 | 0.0 | -inf | 0.02965 |
| CAG00327_Faecalibacterium_sp_CAG_74 | > | 0 | 44.4 | 59.5 | 0.3226 | 0.9968 | 1.9956 | **-0.9968** | 0.5011 | 1 | 1 | 0.00212113 | 0.00139362 | 0.00043694 | 0.00012343 | 0.21 | -2.28 | 0.92773 |
| CAG01039_Faecalibacterium_cf_prausnitzii_KLE1255 | > | 0 | 77.8 | 47.6 | 0.0229 | 1.0043 | 2.006 | **-1.0043** | 0.4985 | 1 | 1 | 0.00301567 | 0.0020844 | 0.00068723 | 0.00019997 | 0.23 | -2.13 | 0.03517 |
| CAG00140_Subdoligranulum_sp_4_3_54A2FAA | > | 0 | 92.6 | 100.0 | 0.1496 | 0.7456 | 1.6767 | **-0.7456** | 0.5964 | 1 | 1 | 0.00338708 | 0.00087016 | 0.0012847 | 0.00019541 | 0.38 | -1.4 | 0.02047 |

(continued)

| Group | op. | Thr | NR (n=27) (%) | R (n=42) (%) | Pval_Fisher | NR_co-eff | NR_OddsRatio | R_co-eff | R_OddsRatio | rank_RFE CV | rank_RFE | NR Mean relabund | NR Sem | R Mean relabund | R Sem | FR R/NR | log2FR R / NR | P value NR vs R |
|---|---|---|---|---|---|---|---|---|---|---|---|---|---|---|---|---|---|---|
| CAG01263_Clostridium_clostridioforme_2_1_49FAA | > | 0 | 81.5 | 78.6 | 1.0 | 0.8736 | 1.8322 | **-0.8736** | 0.5458 | 1 | 1 | 0.00452728 | 0.00253406 | 0.0009867 | 0.00045528 | 0.22 | -2.2 | 0.05257 |
| CAG00037_Bacteroides_faecis_MAJ27 | > | 0 | 14.8 | 28.6 | 0.2478 | -0.7032 | 0.6142 | **0.7032** | 1.6282 | 1 | 1 | 0.00031624 | 0.00019911 | 0.00320497 | 0.00119881 | 10.13 | 3.34 | 0.12006 |
| CAG00357_Bacteroides_ovatus_V975 | > | 0 | 92.6 | 92.9 | 1.0 | 0.6073 | 1.5234 | **-0.6073** | 0.6564 | 1 | 1 | 0.01574035 | 0.00485731 | 0.00872078 | 0.00225535 | 0.55 | -0.85 | 0.27645 |

**Example 5: Predictive models for RCC patients regardless antibiotic usage, OUTCOME 1 criterion**

According to the results obtained in Example 4, and due to the fact that responders (R) are predicted better than non-responders (NR) in all the three models proposed with 8 species (Figure 5), we took into consideration the logistic regression $\beta$ coefficients relative to R.

For the Model 1.2, according to the $\beta$ coefficients of R (R_coeff), the probability estimate (expressed as a percentage) of belonging to R cohort for a tested subject JD is calculated as follows:

$$P_{R\,(JD)} = \ 1 \,/\, [1 + \exp^{\wedge}{-}(-1.0323 * CAG00008 +$$
$$-1.084 * CAG01039 +$$
$$-0.9723 * CAG00473 +$$
$$-0.9907 * CAG00140 +$$
$$-0.6234 * CAG00610 +$$
$$-0.8468 * CAG01141 +$$
$$0.8994 * CAG00413 +$$
$$0.6041 * CAG00317)] \qquad [3]$$

In equation [3], "CAGXXXXX" represents the relative abundance of the bacterial species defined by the recited CAG, in a stool sample from the tested subject JD. These relative abundances need to be expressed as standardized values (zero mean and unit variance). The best option is to standardize the "abundances pattern" of the tested subject with a bundle of known "reference patterns", *i.e.,* all the relative abundances of each bacterial species for each fecal sample used in training the logistic regression model and the present invention refers a claim of a Python script used to ease the computation of the $P_R$ (JD). From the standardized abundance pattern of a tested subject, the equation [3] can be applied to predict his/her belonging to the NR cohort with a model predictability of 79.71% and a success rate of 77%.

For the Model 2.2, according to the $\beta$ coefficients of R (R_coeff), the probability estimate (expressed as a percentage) of belonging to R cohort for a tested subject JD is calculated as follows:

$$P_{R\,(JD)} = \ 1 \,/\, [1 + \exp^{\wedge}{-}(-1.0791 * CAG00727 +$$
$$-1.1164 * CAG01141 +$$
$$-1.2839 * CAG00650 +$$
$$-1.1156 * CAG00928 +$$
$$-1.014 * CAG00473 +$$
$$-0.9394 * CAG00122 +$$
$$-0.8085 * CAG01144 +$$
$$-0.7679 * CAG00063)] \qquad [4]$$

In equation [4], "CAGXXXXX" represents the relative abundance of the bacterial species defined by the recited CAG, in a stool sample from the tested subject JD. These relative abundances need to be expressed as standardized values (zero mean and unit variance). The best option is to standardize the "abundances pattern" of the tested subject with a bundle of known "reference patterns", and the present invention refers a claim of a Python script used to ease the computation of the $P_{R(JD)}$. From the standardized abundance pattern of a tested subject, the equation [4] can be applied to predict his/her belonging to the R cohort with a model predictability of 89.86% and a success rate of 92%.

For the Model 3.2, according to the $\beta$ coefficients of R (R_coeff), the probability estimate (expressed as a percentage) of belonging to R cohort for a tested subject JD is calculated as follows:

$$P_{R\,(JD)} = 1 / [1 + \exp\hat{}-(-1.1735 * CAG00243 +$$
$$-1.027 * CAG00473 +$$
$$-0.9968 * CAG00327 +$$
$$-1.0043 * CAG01039 +$$
$$-0.7456 * CAG00140 +$$
$$-0.8736 * CAG01263 +$$
$$0.7032 * CAG00037 +$$
$$-0.6073 * CAG00357)] \qquad [5]$$

In equation [5], "CAGXXXXX" represents the relative abundance of the bacterial species defined by the recited CAG, in a stool sample from the tested subject JD. These relative abundances need to be expressed as standardized values (zero mean and unit variance). The best option is to standardize the "abundances pattern" of the tested subject with a bundle of known "reference patterns", and the present invention refers a claim of a Python script used to ease the computation of the $P_{R(JD)}$. From the standardized abundance pattern of a tested subject, the equation [5] can be applied to predict his/her belonging to the R cohort with a model predictability of 85.51% and a success rate of 85%.

**Example 6: Results for RCC patients with no antibiotic usage, OUTCOME 1 criterion**

In this example, three different models were built based on a bundle of 58 "reference patterns", which are the standardized abundances profiles (zero mean and unit variance) obtained from the 58 samples used in training the logistic regression models. Standardization is necessary to homogenize the dynamic range of bacterial species abundances.

Based on the first strategy applied to 58 RCC patients (NR = 19; R = 39) with no antibiotic usage history and OUTCOME_1 criterion (best outcome), we selected 25 bacterial species that gave a logistic regression model overall predictability equal to 89.66% (model 4.1). In order to measure the goodness of the model with 25 species, after a 5-fold cross-validation performed on a subset of the original dataset, the model itself was able to predict 67% of NR and 100% of R subjects, with a CV_AUC = 1 and an AUC = 0.778. Upon RFECV and RFE feature selection, 8 species were selected from the aforementioned 25: the model built on these 8 species gave an overall predictability of 87.93% (model 4.2, Figure 6), and the summary statistics for these species are reported in Table 21 below. In order to measure the goodness of the model, after a 5-fold cross-validation performed on a subset of the original dataset, the model itself was able to predict 100% of NR and 92% of R subjects, with a CV_AUC = 1 and an AUC = 1.

Based on the second strategy (machine-learning) applied to 58 RCC patients (NR = 19; R = 39) with no antibiotic usage history and OUTCOME_1 criterion, we took into account all the bacterial species of the dataset (n = 1083). After a first round of feature selection, RFECV gave a rank = 1 to 9 species which collectively, after a 5-fold cross-validation, were able to predict 100% of NR and 92% of R subjects, with a CV_AUC = 1 and an AUC = 1 (model 5.1). Upon a second round of feature selection, RFE gave a rank = 1 to 8 species, which were selected for the subsequent logistic regression classifier. The model built on these 8 species gave an overall predictability of 93.1% (model 5.2, Figure 6), and the summary statistics for these species are reported in Table 22 below. In order to measure the goodness of the model, after a 5-fold cross-validation performed on a subset of the original dataset, the model itself was able to predict 100% of NR and 92% of R subjects, with a CV_AUC = 1 and an AUC = 1.

**Table 21:** Statistics for the species selected in Model 4.2.

| Group | op. | Thr | NR (n=19) (%) | R (n=39) (%) | Pval_Fisher | NR_coeff | NR_Odds Ratio | R_coeff | R_Odds Ratio | rank_RF ECV | rank_RF E | NR Mean relabund | NR Sem | R Mean relabund | R Sem | FR R / NR | log2FR R / NR | P value NR vs R |
|---|---|---|---|---|---|---|---|---|---|---|---|---|---|---|---|---|---|---|
| CAG00008_Clostridium_bolteae_ATCC_BAA_613 | > | 0 | 94.7 | 94.9 | 1.0 | **0.955** | 1.9386 | -0.955 | 0.5158 | 1 | 1 | 0.00806075 | 0.00605804 | 0.00051335 | 0.00014219 | 0.06 | -3.97 | 0.00975 |
| CAG01039_Faecalibacterium_cf_prausnitzii_KLE1255 | > | 0 | 73.7 | 51.3 | 0.1561 | **0.8761** | 1.8354 | -0.8761 | 0.5449 | 1 | 1 | 0.00364533 | 0.00295908 | 0.0007401 | 0.00021316 | 0.2 | -2.3 | 0.21913 |
| CAG00473_Prevotella_sp_CAG_617 | > | 0 | 10.5 | 0.0 | 0.1034 | **0.9951** | 1.9932 | -0.9951 | 0.5017 | 1 | 1 | 0.00378162 | 0.00261518 | 0.0 | 0.0 | 0.0 | -inf | 0.04363 |
| CAG00487_Prevotella_sp_CAG_279 | > | 0 | 10.5 | 0.0 | 0.1034 | **0.7609** | 1.6945 | -0.7609 | 0.5901 | 1 | 1 | 0.00059085 | 0.00047993 | 0.0 | 0.0 | 0.0 | -inf | 0.04363 |
| CAG00140_Subdoligranulum_sp_4_3_54A2FAA | > | 0 | 89.5 | 100.0 | 0.1034 | **1.1107** | 2.1595 | -1.1107 | 0.4631 | 1 | 1 | 0.00380225 | 0.00120314 | 0.00128653 | 0.00020791 | 0.34 | -1.56 | 0.08193 |
| CAG01141_Holdemanella_biformis_DSM_3989 | > | 0 | 15.8 | 2.6 | 0.0982 | **0.9402** | 1.9188 | -0.9402 | 0.5212 | 1 | 1 | 0.0015759 | 0.00087372 | 6.256e-05 | 6.256e-05 | 0.04 | -4.65 | 0.05935 |

(continued)

| Group | op. | Thr | NR (n=19) (%) | R (n=39) (%) | Pval_Fisher | NR_coeff | NR_Odds Ratio | R_coeff | R_OddsRatio | rank_RF ECV | rank_RF E | NR Mean relabund | NR Sem | R Mean relabund | R Sem | FR R /NR | log2FR R/NR | P value NR vs R |
|---|---|---|---|---|---|---|---|---|---|---|---|---|---|---|---|---|---|---|
| CAG01208_Coprococcus_catus_GD_7 | > | 0 | 84.2 | 69.2 | 0.3399 | **0.7187** | 1.6456 | -0.7187 | 0.6077 | 1 | 1 | 0.00134292 | 0.00056097 | 0.00040444 | 7.399e-05 | 0.3 | -1.73 | 0.36682 |
| CAG00413_Bacteroides_sp_CAG_144 | > | 0 | 10.5 | 33.3 | 0.1084 | **0.5812** | 0.6684 | 0.5812 | 1.4961 | 1 | 1 | 3.884e-05 | 2.907e-05 | 0.00195009 | 0.00062503 | 50.2 | 5.65 | 0.03684 |

**Table 22:** Statistics for the species selected in Model 5.2.

| Group | op. | Thr | NR n=19 (%) | R n=39 (%) | Pval_ Fisher | NR_coeff | NR_ Odds Ratio | R_coeff | R_ Odds Ratio | rank RFE CV | rank RFE | NR Mean rebund | NR Sem | R Mean relabund | R Sem | FR R/ NR | log2FR R / NR | P value NR vs R |
|---|---|---|---|---|---|---|---|---|---|---|---|---|---|---|---|---|---|---|
| CAG00048 _1_Clostrid ium_sp _CAG_226 | > | 0 | 21.1 | 5.1 | 0.0828 | **0.7112** | 1.6372 | -0.7112 | 0.6108 | 1 | 1 | 6.032e-05 | 3.159e-05 | 3.2e-06 | 2.68e-06 | 0.05 | -4.24 | 0.05012 |
| CAG00300 _Prevotella _sp_CAG_ 891 | > | 0 | 15.8 | 0.0 | 0.0314 | **0.8931** | 1.8571 | -0.8931 | 0.5385 | 1 | 1 | 7.957e-05 | 5.076e-05 | 0.0 | 0.0 | 0.0 | -inf | 0.01229 |
| CAG00473 _Prevotella _sp_CAG_ 617 | > | 0 | 10.5 | 0.0 | 0.1034 | **1.0042** | 2.0058 | -1.0042 | 0.4986 | 1 | 1 | 0.00378162 | 0.00261518 | 0.0 | 0.0 | 0.0 | -inf | 0.04363 |
| CAG00650 _Dorea_ formicigen erans_ ATCC_ 27755 | > | 0 | 84.2 | 92.3 | 0.3822 | **1.1282** | 2.1858 | -1.1282 | 0.4575 | 1 | 1 | 0.00129344 | 0.00043426 | 0.00044294 | 6.381e-05 | 0.34 | -1.55 | 0.1252 |
| CAG00720 _Anaerotru ncus_ colihominis _DSM_ 17241 | > | 0 | 84.2 | 69.2 | 0.3399 | **1.2569** | 2.3898 | -1.2569 | 0.4184 | 1 | 1 | 0.00086591 | 0.00038685 | 0.00010478 | 2.71e-05 | 0.12 | -3.05 | 0.01338 |
| CAG00727 _Eggerthell a_ lenta_ DSM_2243 | > | 0 | 78.9 | 48.7 | 0.0458 | **1.2397** | 2.3615 | -1.2397 | 0.4235 | 1 | 1 | 0.00063799 | 0.00031339 | 9.214e-05 | 2.989e-05 | 0.14 | -2.79 | 0.00697 |
| CAG00963 _Clostridiu m_sp_ CAG_524 | > | 0 | 10.5 | 0.0 | 0.1034 | **0.7595** | 1.6929 | -0.7595 | 0.5907 | 1 | 1 | 5.774e-05 | 4.984e-05 | 0.0 | 0.0 | 0.0 | -inf | 0.04363 |

| Group | op. | Thr | NR n=19 (%) | R n=39 (%) | Pval_ Fisher | NR_coeff | NR_ Odds Ratio | R_coeff | R_ Odds Ratio | rank RFE CV | rank RFE | NR Mean rebund | NR Sem | R Mean relabund | R Sem | FR R/ NR | log2FR R / NR | P value NR vs R |
|---|---|---|---|---|---|---|---|---|---|---|---|---|---|---|---|---|---|---|
| CAG01141 _Holdema nella_ biformis_ DSM_3989 | > | 0 | 15.8 | 2.6 | 0.0982 | **1.0235** | 2.0328 | -1.0235 | 0.4919 | 1 | 1 | 0.0015759 | 0.00087372 | 6.256e-05 | 6.256e-05 | 0.04 | -4.65 | 0.05935 |

Taking into account the bacterial species (n = 183, above 1083 total species, 16.9% of the total microbial composition) having an average relative abundance greater than or equal to 0.001 if expressed in the interval [0 : 1] (thus, the 0.1%) within the overall RCC cohort, we applied the second strategy (machine-learning) for 58 RCC patients (NR = 19; R = 39) with no antibiotic usage history and OUTCOME_1 criterion. After a first round of feature selection, RFECV gave a rank = 1 to 20 species which collectively, after a 5-fold cross-validation, were able to predict 100% of NR and 92% of R subjects, with a CV_AUC = 0.917 and an AUC = 1 (model 6.1). After a second round of feature selection, RFE gave a rank = 1 to 8 species which were selected for the subsequent logistic regression classifier. The model built on these 8 species gave an overall predictability of 84.48% (model 6.2, Figure 6), and the summary statistics for these species are reported in Table 23 below. In order to measure the goodness of the model, after a 5-fold cross-validation performed on a subset of the original dataset, the model itself was able to predict 67% of NR and 100% of R subjects, with a CV_AUC = 0.917 and an AUC = 0.889. This last model was designed to be more affordable in case of problems in fecal samples storage, sequencing, stiffness, *etc.*

According to the Models 4.2, 5.2 and 6.2, which were modeled on no antibiotic usage history and OUTCOME_1 criterion and were all based on 8 bacterial species in order to be properly compared, it is shown that the Model 5.2 has the highest predictability (93.1 %) with optimal CV_AUC and AUC.

**Table 23:** Statistics for the species selected in Model 6.2.

| Group | op. | Thr | NR (n=19) (%) | R (n=39) (%) | Pval_ Fisher | NR_coeff | NR_ Odds Ratio | **R_coeff** | R_ Odds Ratio | rank_ RFECV | rank_ RFE | NR Mean relabund | NR Sem | R Mean relabund | R Sem | Fold Ratio (FR)R / NR | log2FR R / NR | P value NR vs R |
|---|---|---|---|---|---|---|---|---|---|---|---|---|---|---|---|---|---|---|
| CAG0024 3_Lachno spiraceae _ bacterium _1_1_57FA A | > | 0 | 84.2 | 89.7 | 0.6726 | 1.2907 | 2.4465 | **-1.2907** | 0.4087 | 1 | 1 | 0.0104986 | 0.00482111 | 0.00156745 | 0.00034161 | 0.15 | -2.74 | 0.42608 |
| CAG0000 8_Clostridi um_ bolteae_ ATCC_ BAA_613 | > | 0 | 94.7 | 94.9 | 1.0 | 0.8848 | 1.8465 | **-0.8848** | 0.5416 | 1 | 1 | 0.00806075 | 0.00605804 | 0.00051335 | 0.00014219 | 0.06 | -3.97 | 0.00975 |
| CAG0069 8_Rumino coccus_ sp_ CAG_ 177 | > | 0 | 15.8 | 10.3 | 0.6726 | 0.9776 | 1.9691 | **-0.9776** | 0.5078 | 1 | 1 | 0.00683429 | 0.00584606 | 0.00013458 | 8.414e-05 | 0.02 | -5.67 | 0.51949 |
| CAG0047 3_Prevote lla_sp_ CAG_617 | > | 0 | 10.5 | 0.0 | 0.1034 | 0.9997 | 1.9996 | **-0.9997** | 0.5001 | 1 | 1 | 0.00378162 | 0.00261518 | 0.0 | 0.0 | 0.0 | -inf | 0.04363 |
| CAG0032 7 _Faecalib acterium_ sp _CAG_ 74 | > | 0 | 36.8 | 61.5 | 0.0973 | 1.0697 | 2.099 | **-1.0697** | 0.4764 | 1 | 1 | 0.00262438 | 0.0019694 | 0.00046816 | 0.00013167 | 0.18 | -2.49 | 0.518 |

(continued)

| Group | op. | Thr | NR (n=19) (%) | R (n=39) (%) | Pval_ Fisher | NR_coeff | NR_ Odds Ratio | R_coeff | R_ Odds Ratio | rank_ RFECV | rank_ RFE | NR Mean relabund | NR Sem | R Mean relabund | R Sem | Fold Ratio (FR)R / NR | log2FR R / NR | P value NR vs R |
|---|---|---|---|---|---|---|---|---|---|---|---|---|---|---|---|---|---|---|
| CAG01 039_Faec alibacteri- um _cf_ prausnitzii _KLE1255 | > | 0 | 73.7 | 51.3 | 0.1561 | 0.7806 | 1.7178 | **-0.7806** | 0.5821 | 1 | 1 | 0.00364533 | 0.00295908 | 0.0007401 | 0.00021316 | 0.2 | -2.3 | 0.21913 |
| CAG0089 7_Firmicut es _bacteriu m_CAG_ 83 | > | 0 | 89.5 | 89.7 | 1.0 | 0.8376 | 1.7871 | **-0.8376** | 0.5596 | 1 | 1 | 0.00380745 | 0.00159185 | 0.00166388 | 0.00044938 | 0.44 | -1.19 | 0.35326 |
| CAG0076 6_Firmicut es _bacteriu m_CAG_ 176 | > | 0 | 36.8 | 48.7 | 0.4172 | 0.7459 | 1.6771 | **-0.7459** | 0.5963 | 1 | 1 | 0.01478181 | 0.00761963 | 0.00510704 | 0.00148355 | 0.35 | -1.53 | 0.74374 |

**Example 7: Predictive models for RCC patients with no antibiotic usage, OUTCOME 1 criterion**

According to the results obtained in Example 6, and due to the fact that non-responders (NR) are predicted better than responders (R) in the first two models proposed with 8 species (Figure 7), while is the opposite for the last model, we took into consideration the logistic regression $\beta$ coefficients relative to NR for Models 4.2 and 5.2, and the logistic regression $\beta$ coefficients relative to R for the Model 6.2.

Regarding the Model 4.2, and according to the $\beta$ coefficients of NR (NR_coeff), the probability estimate (expressed as a percentage) of belonging to NR cohort for a tested subject JD is calculated as follows:

$$P_{\text{NR (JD)}} = 1 \,/\, [1 + \exp\char`\^{-}(0.955 * \text{CAG00008} +$$
$$0.8761 * \text{CAG01039} +$$
$$0.9951 * \text{CAG00473} +$$
$$0.7609 * \text{CAG00487} +$$
$$1.1107 * \text{CAG00140} +$$
$$0.9402 * \text{CAG01141} +$$
$$0.7187 * \text{CAG01208} +$$
$$-0.5812 * \text{CAG00413})] \qquad [6]$$

In equation [6], "CAGXXXXX" represents the relative abundance of the bacterial species defined by the recited CAG, in a stool sample from the tested subject JD. These relative abundances need to be expressed as standardized values (zero mean and unit variance). The best option is to standardize the "abundances pattern" of the tested subject with a bundle of known "reference patterns", and the present invention refers a claim of a Python script used to ease the computation of the $P_{\text{NR}}$ (JD). From the standardized abundance pattern of a tested subject, the equation [6] can be applied to predict his/her belonging to the NR cohort with a model predictability of 87.93% and a success rate of 100%.

Regarding the Model 5.2, and according to the $\beta$ coefficients of NR (NR_coeff), the probability estimate (expressed as a percentage) of belonging to NR cohort for a tested subject JD is calculated as follows:

$$P_{\text{NR (JD)}} = 1 \,/\, [1 + \exp\char`\^{-}(0.7112 * \text{CAG00048\_1} +$$
$$0.8931 * \text{CAG00300} +$$
$$1.0042 * \text{CAG00473} +$$
$$1.1282 * \text{CAG00650} +$$
$$1.2569 * \text{CAG00720} +$$
$$1.2397 * \text{CAG00727} +$$
$$0.7595 * \text{CAG00963} +$$
$$1.0235 * \text{CAG01141})] \qquad [7]$$

In equation [7], "CAGXXXXX" represents the relative abundance of the bacterial species defined by the recited CAG, in a stool sample from the tested subject JD. These relative abundances need to be expressed as standardized values (zero mean and unit variance). The best option is to standardize the "abundances pattern" of the tested subject with a bundle of known "reference patterns", and the present invention refers a claim of a Python script used to ease the computation of the $P_{\text{NR}}$ (JD). From the standardized abundance pattern of a tested subject, the equation [7] can be applied to predict his/her belonging to the NR cohort with a model predictability of 93.1% and a success rate of 100%.

Regarding the Model 6.2, and according to the $\beta$ coefficients of R (R_coeff), the probability estimate (expressed as a percentage) of belonging to R cohort for a tested subject JD is calculated as follows:

$$P_{R\,(JD)} = 1 / [1 + exp^\wedge{-}(-1.2907 * CAG00243 +$$
$$-0.8848 * CAG00008 +$$
$$-0.9776 * CAG00698 +$$
$$-0.9997 * CAG00473 +$$
$$-1.0697 * CAG00327 +$$
$$-0.7806 * CAG01039 +$$
$$-0.8376 * CAG00897 +$$
$$-0.7459 * CAG00766)] \qquad [8]$$

In equation [8], "CAGXXXXX" represents the relative abundance of the bacterial species defined by the recited CAG, in a stool sample from the tested subject JD. These relative abundances need to be expressed as standardized values (zero mean and unit variance). The best option is to standardize the "abundances pattern" of the tested subject with a bundle of known "reference patterns", and the present invention refers a claim of a Python script used to ease the computation of the $P_R$ (JD). From the standardized abundance pattern of a tested subject, the equation [8] could be applied to predict his/her belonging to the R cohort with a model predictability of 84.48% and a success rate of 100%.

**Example 8: Results for RCC patients regardless antibiotic usage, OUTCOME 2 criterion**

In this example, three different models were built based on a bundle of 67 "reference patterns", which are the standardized abundances profiles (zero mean and unit variance) obtained from the 67 samples used in training the logistic regression models. Standardization is necessary to homogenize the dynamic range of bacterial species abundances.

Based on the first strategy applied to 67 RCC patients (NR = 37; R = 30) with unknown antibiotic usage history and OUTCOME_2 criterion (TPP > 6 months), we selected 10 bacterial species that gave a logistic regression model overall predictability equal to 65.22% (model 7.1). In order to measure the goodness of the model with 10 species, after a 5-fold cross-validation performed on a subset of the original dataset, the model itself was able to predict 78% of NR and 44% of R subjects, with a CV_AUC = 0.370 and an AUC = 0.716. Upon RFECV and RFE feature selection, 8 species were selected from the aforementioned 10: the model built on these 8 species gave an overall predictability of 66.67% (model 7.2, Figure 7), and the summary statistics for these species are reported in Table 24. In order to measure the goodness of the model, after a 5-fold cross-validation performed on a subset of the original dataset, the model itself was able to predict 78% of NR and 44% of R subjects, with a CV_AUC = 0.481 and an AUC = 0.704.

Based on the second strategy (machine-learning) applied to 67 RCC patients (NR = 37; R = 30) with unknown antibiotic usage history and OUTCOME_2 criterion, we took into account all the bacterial species of the dataset (n = 1112). After a first round of feature selection, RFECV gave a rank = 1 to 1070 species which collectively, after a 5-fold cross-validation, were able to predict 11% of NR and 100% of R subjects, with a CV_AUC = 0.667 and an AUC = 0.667 (model 8.1). Upon a second round of feature selection, RFE gave a rank = 1 to 8 species, which were selected for the subsequent logistic regression classifier. The model built on these 8 species gave an overall predictability of 88.41% (model 8.2, Figure 7), and the summary statistics for these species are reported in Table 25. In order to measure the goodness of the model, after a 5-fold cross-validation performed on a subset of the original dataset, the model itself was able to predict 100% of NR and 67% of R subjects, with a CV_AUC = 0.667 and an AUC = 0.926.

Taking into account the bacterial species (n = 181, above 1112 total species, 16.3% of the total microbial composition) having an average relative abundance greater than or equal to 0.001 if expressed in the interval [0 : 1] (thus, the 0.1%) within the overall RCC cohort, we applied the second strategy (machine-learning) for 67 RCC patients (NR = 37; R = 30) with unknown antibiotic usage history and OUTCOME_2 criterion. After a first round of feature selection, RFECV gave a rank = 1 to 175 species which collectively, after a 5-fold cross-validation, were able to predict 33% of NR and 78% of R subjects, with a CV_AUC = 0.222 and an AUC = 0.605 (model 9.1). After a second round of feature selection, RFE gave a rank = 1 to 8 species which were selected for the subsequent logistic regression classifier. The model built on these 8 species gave an overall predictability of 88.41% (model 9.2, Figure 7), and the summary statistics for these species are reported in Table 26. In order to measure the goodness of the model, after a 5-fold cross-validation performed on a subset of the original dataset, the model itself was able to predict 89% of NR and 78% of R subjects, with a CV_AUC = 0.481 and an AUC = 0.864. This last model was designed to be more affordable in case of problems in fecal samples storage, sequencing, stiffness, etcetera.

According to all Models, which were modeled on unknown antibiotic usage history and OUTCOME_2 criterion, it is shown that the Model 8.2 has a good predictability (84.41%) with good CV_AUC and AUC, and can be chosen as the most discriminant among NR and R patients.

**Table 24:** Statistics for the species selected in Model 7.2.

| Group | op. | Thr | NR (n=37) (%) | R (n=30) (%) | Pval_ Fisher | NR_ coeff | NR_ Odds Ratio | R_coeff | R_ Odds Ratio | rank_ RFECV | rank_ RFE | NR Mean relabund | NR Sem | R Mean relabund | R Sem | Fold Ratio (FR) R/NR | log2FR R/NR | P value NR vs R |
|---|---|---|---|---|---|---|---|---|---|---|---|---|---|---|---|---|---|---|
| CAG00782_Eubacterium_rectale_CAG_36 | > | 0 | 86.5 | 83.3 | 0.7431 | **-0.2034** | 0.8685 | 0.2009 | 1.1494 | 3 | 1 | 0.01855862 | 0.00499803 | 0.0158587 | 0.00446139 | 0.85 | -0.23 | 0.84974 |
| CAG00013_Clostridiales_bacterium_1_7_47FAA | > | 0 | 78.4 | 76.7 | 1.0 | **0.4092** | 1.328 | -0.348 | 0.7857 | 1 | 1 | 0.00056515 | 0.00023638 | 0.00030914 | 0.00011594 | 0.55 | -0.87 | 0.57258 |
| CAG00873_Butyricimonas_virosa_DSM_23226 | > | 0 | 70.3 | 73.3 | 1.0 | **0.2708** | 1.2065 | -0.2435 | 0.8447 | 2 | 1 | 0.00109418 | 0.00020006 | 0.00063137 | 0.00012234 | 0.58 | -0.79 | 0.29268 |
| CAG01141_Holdemanella_biformis_DSM_3989 | > | 0 | 13.5 | 0.0 | 0.0599 | **0.7665** | 1.7011 | -0.6748 | 0.6264 | 1 | 1 | 0.00154882 | 0.00079727 | 0.0 | 0.0 | 0.0 | -inf | 0.03925 |
| CAG00668_Azospirillum_sp_CAG_239 | > | 0 | 0.0 | 10.0 | 0.0848 | **-0.5899** | 0.6644 | 0.6848 | 1.6075 | 1 | 1 | 0.0 | 0.0 | 0.00068027 | 0.00048003 | 0.0 | -inf | 0.05297 |

| Group | op. | Thr | NR (n=37) (%) | R (n=30) (%) | Pval_ Fisher | NR_ coeff | NR_ Odds Ratio | R_coeff | R_ Odds Ratio | rank_ RFECV | rank_ RFE | NR Mean relabund | NR Sem | R Mean relabund | R Sem | Fold Ratio (FR) R/NR | log2FR R/NR | P value NR vs R |
|---|---|---|---|---|---|---|---|---|---|---|---|---|---|---|---|---|---|---|
| CAG00669_Firmicutes_ bacterium _CAG_ 103 | > | 0 | 32.4 | 53.3 | 0.1345 | **-0.5181** | 0.6983 | 0.4599 | 1.3754 | 1 | 1 | 0.00062774 | 0.00020461 | 0.00180915 | 0.00055553 | 2.88 | 1.53 | 0.0513 |
| CAG00886_Sutterella_sp_ CAG_351 | > | 0 | 0.0 | 10.0 | 0.0848 | **-0.8415** | 0.5581 | 0.9265 | 1.9007 | 1 | 1 | 0.0 | 0.0 | 0.0007941 | 0.00060463 | 0.0 | -inf | 0.05297 |
| CAG00889_Megasphaera_ elsdenii_ 14_14 | > | 0 | 0.0 | 13.3 | 0.0358 | **-0.7339** | 0.6013 | 0.8563 | 1.8104 | 1 | 1 | 0.0 | 0.0 | 0.00133655 | 0.00079124 | 0.0 | -inf | 0.02403 |

Table 25: Statistics for the species selected in Model 8.2.

| Group | op. | Thr | NR (n=37) (%) | R (n=30) (%) | Pval_Fisher | NR_coeff | NR_Odds Ratio | R_coeff | R_Odds Ratio | rank_RFECV | rank_RFE | NR Mean relabund | NR Sem | R Mean relabund | R Sem | Fold Ratio (FR) R/NR | log2FR R/NR | P value NR vs R |
|---|---|---|---|---|---|---|---|---|---|---|---|---|---|---|---|---|---|---|
| CAG00211_Firmicutes_bacterium_CAG_227 | > | 0 | 16.2 | 10.0 | 0.7206 | **0.6422** | 1.5607 | -0.5746 | 0.6715 | 1 | 1 | 5.982e-05 | 2.678e-05 | 1.092e-05 | 7.11e-06 | 0.18 | -2.45 | 0.39482 |
| CAG00474_Sutterella_wadsworthensis_2_1_59BFAA | > | 0 | 8.1 | 23.3 | 0.0982 | **-1.0581** | 0.4803 | 1.1768 | 2.2608 | 1 | 1 | 0.00023198 | 0.00014013 | 0.0019427 | 0.00086912 | 8.37 | 3.07 | 0.07186 |
| CAG00624_Firmicutes_bacterium_CAG_552 | > | 0 | 0.0 | 16.7 | 0.0148 | **-0.8984** | 0.5365 | 1.0055 | 2.0076 | 1 | 1 | 0.0 | 0.0 | 7.419e-05 | 3.936e-05 | 0.0 | -inf | 0.0109 |
| CAG00650_Dorea_formicigenerans_ATCC_27755 | > | 0 | 81.1 | 90.0 | 0.4926 | **1.3189** | 2.4947 | -1.271 | 0.4144 | 1 | 1 | 0.00092982 | 0.0002447 | 0.00039615 | 7.209e-05 | 0.43 | -1.23 | 0.30037 |
| CAG00676_Firmicutes_bacterium_CAG_176 | > | 0 | 16.2 | 20.0 | 0.7553 | **-0.7324** | 0.6019 | 0.0679 | 1.0482 | 1 | 1 | 0.0001876 | 0.00010047 | 0.00087187 | 0.00055567 | 4.65 | 2.22 | 0.67128 |

EP 3 629 023 A1

(continued)

| Group | op. | Thr | NR (n=37) (%) | R (n=30) (%) | Pval_ Fisher | NR_ coeff | NR_ Odds Ratio | R_coeff | R_ Odds Ratio | rank_ RFECV | rank_ RFE | NR Mean relabund | NR Sem | R Mean relabund | R Sem | Fold Ratio (FR) R/NR | log2FR R/NR | P value NR vs R |
|---|---|---|---|---|---|---|---|---|---|---|---|---|---|---|---|---|---|---|
| CAG00771_Clostridium_sp_CAG_413 | > | 0 | 0.0 | 10.0 | 0.0848 | -0.6623 | 0.6319 | 0.4204 | 1.3383 | 1 | 1 | 0.0 | 0.0 | 5.164e-05 | 3.902e-05 | 0.0 | -inf | 0.05297 |
| CAG01197_Dialister_succinatiphilus_YIT_11850 | > | 0 | 8.1 | 30.0 | 0.0268 | -1.1372 | 0.4546 | 0.9425 | 1.9219 | 1 | 1 | 3.257e-05 | 2.681e-05 | 0.00014594 | 5.12e-05 | 4.48 | 2.16 | 0.01886 |
| CAG01321_Faecalibacterium_prausnitzii_SL3_3 | > | 0 | 83.8 | 90.0 | 0.7206 | -0.8525 | 0.5538 | 0.8843 | 1.8458 | 1 | 1 | 0.00480104 | 0.00091279 | 0.01054466 | 0.00251204 | 2.2 | 1.14 | 0.13467 |

56

**Table 26:** Statistics for the species selected in Model 9.2.

| Group | op. | Thr | NR (n=37) (%) | R (n=30) (%) | Pval_ Fisher | **NR_ coeff** | NR_ Odds Ratio | R_coeff | R-Odds Ratio | rank_ RFECV | rank_ RFE | NR Mean relabund | NR Sem | R Mean relabund | R Sem | Fold Ratio (FR) R/NR | log2FR R/NR | P value NR vs R |
|---|---|---|---|---|---|---|---|---|---|---|---|---|---|---|---|---|---|---|
| CAG00557_Rumino coccus_callidus_ ATCC_ 27760 | > | 0 | 24.3 | 33.3 | 0.4303 | **0.9341** | 1.9107 | -0.8033 | 0.573 | 1 | 1 | 0.00160652 | 0.00069973 | 0.0004874 | 0.00020579 | 0.3 | -1.72 | 0.69767 |
| CAG00601_uncultur ed _Faecalib acterium_ sp_ | > | 0 | 70.3 | 70.0 | 1.0 | **0.8927** | 1.8567 | -1.0252 | 0.4913 | 1 | 1 | 0.00483175 | 0.0009123 | 0.00223149 | 0.00050757 | 0.46 | -1.11 | 0.12832 |
| CAG00607 _Eubacter ium_sp _CAG_ 251 | > | 0 | 37.8 | 30.0 | 0.6076 | **0.7108** | 1.6367 | -0.5952 | 0.662 | 1 | 1 | 0.00225675 | 0.00078393 | 0.00059667 | 0.00025431 | 0.26 | -1.92 | 0.2972 |
| CAG00669_Firmicut es_ bacterium _CAG_ 103 | > | 0 | 32.4 | 53.3 | 0.1345 | **-1.2341** | 0.4251 | 1.1675 | 2.2462 | 1 | 1 | 0.00062774 | 0.00020461 | 0.00180915 | 0.00055553 | 2.88 | 1.53 | 0.0513 |
| CAG00861_Oscillib acter_sp _CAG_ 241 | > | 0 | 37.8 | 26.7 | 0.4347 | **1.0379** | 2.0532 | -0.9407 | 0.521 | 1 | 1 | 0.00475511 | 0.00195861 | 0.000911 | 0.00043555 | 0.19 | -2.38 | 0.26701 |

| Group | op. | Thr | NR (n=37) (%) | R (n=30) (%) | Pval_ Fisher | NR_ coeff | NR_ Odds Ratio | R_coeff | R-Odds Ratio | rank_ RFECV | rank_ RFE | NR Mean relabund | NR Sem | R Mean relabund | R Sem | Fold Ratio (FR) R/NR | log2FR R/NR | P value NR vs R |
|---|---|---|---|---|---|---|---|---|---|---|---|---|---|---|---|---|---|---|
| CAG00880_Subdoli granulum _sp_ CAG_314 | > | 0 | 21.6 | 36.7 | 0.188 | **-1.0016** | 0.4995 | 0.8717 | 1.8298 | 1 | 1 | 0.00084578 | 0.00045115 | 0.00390209 | 0.00155646 | 4.61 | 2.21 | 0.10582 |
| CAG00937_Clostridi um_sp _CAG_7 | > | 0 | 86.5 | 90.0 | 0.7225 | **-0.872** | 0.5464 | 0.9665 | 1.9541 | 1 | 1 | 0.00087447 | 0.00015342 | 0.00125129 | 0.00028594 | 1.43 | 0.52 | 0.48764 |
| CAG01321_Faecali bacterium _prausnitz ii_SL3_3 | > | 0 | 83.8 | 90.0 | 0.7206 | **-0.8362** | 0.5601 | 0.8646 | 1.8208 | 1 | 1 | 0.00480104 | 0.00091279 | 0.01054466 | 0.00251204 | 2.2 | 1.14 | 0.13467 |

**Example 9: Predictive models for RCC patients regardless antibiotic usage, OUTCOME 2 criterion**

According to the results obtained in Example 8, we took into consideration the logistic regression $\beta$ coefficients relative to NR for Models 7.2, 8.2 and 9.2.

For the Model 7.2, according to the $\beta$ coefficients of non-responders (NR_coeff), the probability estimate (expressed as a percentage) of belonging to NR cohort for a tested subject JD is calculated as follows:

$$P_{NR\,(JD)} = 1 / [1 + \exp\hat{}-(-0.2034 * CAG00782 +$$
$$0.4092 * CAG00013 +$$
$$0.2708 * CAG00873 +$$
$$0.7665 * CAG01141 +$$
$$-0.5899 * CAG00668 +$$
$$-0.5181 * CAG00669 +$$
$$-0.8415 * CAG00886 +$$
$$-0.7339 * CAG00889)] \qquad [9]$$

In equation [9], "CAGXXXXX" represents the relative abundance of the bacterial species defined by the recited CAG, in a stool sample from the tested subject JD. These relative abundances need to be expressed as standardized values (zero mean and unit variance). The best option is to standardize the "abundances pattern" of the tested subject with a bundle of known "reference patterns", and the present invention refers a claim of a Python script used to ease the computation of the $P_{NR}$ (JD). From the standardized abundance pattern of a tested subject, the equation [9] can be applied to predict his/her belonging to the NR cohort with a model predictability of 66.67% and a success rate of 78%.

For the Model 8.2, according to the $\beta$ coefficients of NR (NR_coeff), the probability estimate (expressed as a percentage) of belonging to NR cohort for a tested subject JD is calculated as follows:

$$P_{NR\,(JD)} = 1 / [1 + \exp\hat{}-(0.6422 * CAG00211 +$$
$$-1.0581 * CAG00474 +$$
$$-0.8984 * CAG00624 +$$
$$1.3189 * CAG00650 +$$
$$-0.7324 * CAG00676 +$$
$$-0.6623 * CAG00771 +$$
$$-1.1372 * CAG01197 +$$
$$-0.8525 * CAG01321)] \qquad [10]$$

In equation [10], "CAGXXXXX" represents the relative abundance of the bacterial species defined by the recited CAG, in a stool sample from the tested subject JD. These relative abundances need to be expressed as standardized values (zero mean and unit variance). The best option is to standardize the "abundances pattern" of the tested subject with a bundle of known "reference patterns", and the present invention refers a claim of a Python script used to ease the computation of the $P_{NR}$ (JD). From the standardized abundance pattern of a tested subject, the equation [10] can be applied to predict his/her belonging to the NR cohort with a model predictability of 84.41% and a success rate of 100%.

For the Model 9.2, according to the $\beta$ coefficients of NR (NR_coeff), the probability estimate (expressed as a percentage) of belonging to NR cohort for a tested subject JD is calculated as follows:

$$P_{NR\,(JD)} = 1 / [1 + \exp^{\wedge}-(0.9341 * CAG00557 +$$
$$0.8927 * CAG00601 +$$
$$0.7108 * CAG00607 +$$
$$-1.2341 * CAG00669 +$$
$$1.0379 * CAG00861 +$$
$$-1.0016 * CAG00880 +$$
$$-0.872 * CAG00937 +$$
$$-0.8362 * CAG01321)] \qquad [11]$$

In equation [11], "CAGXXXXX" represents the relative abundance of the bacterial species defined by the recited CAG, in a stool sample from the tested subject JD. These relative abundances need to be expressed as standardized values (zero mean and unit variance). The best option is to standardize the "abundances pattern" of the tested subject with a bundle of known "reference patterns", and the present invention refers a claim of a Python script used to ease the computation of the $P_{NR}$ (JD). From the standardized abundance pattern of a tested subject, the equation [11] can be applied to predict his/her belonging to the NR cohort with a model predictability of 88.41% and a success rate of 89%.

**Example 10: Results for RCC patients with no antibiotic usage, OUTCOME 2 criterion**

In this example, three different models were built based on a bundle of 56 "reference patterns", which are the standardized abundances profiles (zero mean and unit variance) obtained from the 56 samples used in training the logistic regression models. Standardization is necessary to homogenize the dynamic range of bacterial species abundances.

Based on the first strategy applied to 56 RCC patients (NR = 29; R = 27) with no antibiotic usage history and OUTCOME_2 criterion, we selected 10 bacterial species that gave a logistic regression model overall predictability equal to 72.41% (model 10.1). In order to measure the goodness of the model with 10 species, after a 5-fold cross-validation performed on a subset of the original dataset, the model itself was able to predict 78% of NR and 67% of R subjects, with a CV_AUC = 0.667 and an AUC = 0.796. Upon RFECV and RFE feature selection, 8 species were selected from the aforementioned 10: the model built on these 8 species gave an overall predictability of 68.97% (model 10.2, Figure 8), and the summary statistics for these species are reported in Table 27. In order to measure the goodness of the model, after a 5-fold cross-validation performed on a subset of the original dataset, the model itself was able to predict 89% of NR and 100% of R subjects, with a CV_AUC = 0.556 and an AUC = 0.981.

Based on the second strategy (machine-learning) applied to 56 RCC patients (NR = 29; R = 27) with no antibiotic usage history and OUTCOME_2 criterion, we took into account all the bacterial species of the dataset (n = 1083). After a first round of feature selection, RFECV gave a rank = 1 to 51 species which collectively, after a 5-fold cross-validation, were able to predict 89% of NR and 83% of R subjects, with a CV_AUC = 0.944 and an AUC = 0.944 (model 11.1). Upon a second round of feature selection, RFE gave a rank = 1 to 8 species, which were selected for the subsequent logistic regression classifier. The model built on these 8 species gave an overall predictability of 91.38% (model 11.2, Figure 8), and the summary statistics for these species are reported in Table 28. In order to measure the goodness of the model, after a 5-fold cross-validation performed on a subset of the original dataset, the model itself was able to predict 78% of NR and 100% of R subjects, with a CV_AUC = 1 and an AUC = 0.981.

**Table 27:** Statistics for the species selected in Model 10.2.

| Group | op. | Thr | NR (n=29) (%) | R (n=27) (%) | Pval_ Fisher | NR_ coeff | NR_ Odds Ratio | R_coeff | R_ Odds Ratio | rank_ RFECV | rank_ RFE | NR Mean relabund | NR Sem | R Mean relabund | R Sem | Fold Ratio (FR) R/NR | log2FR R/NR | P value NR vs R |
|---|---|---|---|---|---|---|---|---|---|---|---|---|---|---|---|---|---|---|
| CAG00580_Clostridium_sp_CAG_62 | > | 0 | 75.9 | 77.8 | 1.0 | 0.2713 | 1.2069 | **-0.1456** | 0.904 | 2 | 1 | 0.00355474 | 0.00090909 | 0.00216117 | 0.00057471 | 0.61 | -0.72 | 0.43804 |
| CAG00013_Clostridiales_bacterium_1_7_47FAA | > | 0 | 79.3 | 74.1 | 0.7555 | 0.662 | 1.5823 | **-0.5496** | 0.6832 | 1 | 1 | 0.00068832 | 0.00029843 | 0.00017732 | 6.043e-05 | 0.26 | -1.96 | 0.27615 |
| CAG00873_Butyricimonas_virosa_DSM_23226 | > | 0 | 72.4 | 74.1 | 1.0 | 0.4991 | 1.4133 | **-0.4646** | 0.7247 | 1 | 1 | 0.00123868 | 0.000242 | 0.00057747 | 0.00012007 | 0.47 | -1.1 | 0.14061 |
| CAG01141_Holdemanella_biformis_DSM_3989 | > | 0 | 13.8 | 0.0 | 0.1124 | 0.6615 | 1.5817 | **-0.5496** | 0.6832 | 1 | 1 | 0.00111662 | 0.00058518 | 0.0 | 0.0 | 0.0 | -inf | 0.04946 |
| CAG00668_Azospirillum_sp_CAG_239 | > | 0 | 0.0 | 11.1 | 0.1055 | -0.4506 | 0.7318 | **0.5684** | 1.4829 | 1 | 1 | 0.0 | 0.0 | 0.00075585 | 0.00053233 | 0.0 | -inf | 0.0708 |

| Group | op. | Thr | NR (n=29) (%) | R (n=27) (%) | Pval_ Fisher | NR_ coeff | NR_ Odds Ratio | R_coeff | R_ Odds Ratio | rank_ RFECV | rank_ RFE | NR Mean relabund | NR Sem | R Mean relabund | R Sem | Fold Ratio (FR) R/NR | log2FR R/NR | P value NR vs R |
|---|---|---|---|---|---|---|---|---|---|---|---|---|---|---|---|---|---|---|
| CAG00669_Firmicutes_ bacterium _CAG_ 103 | > | 0 | 27.6 | 55.6 | 0.0563 | -0.6129 | 0.6539 | **0.5129** | 1.4269 | 1 | 1 | 0.00047936 | 0.00020785 | 0.00194815 | 0.00061023 | 4.06 | 2.02 | 0.01602 |
| CAG00886_Sutterel la_sp _CAG_ 351 | > | 0 | 0.0 | 7.4 | 0.2279 | -0.5618 | 0.6775 | **0.6623** | 1.5826 | 1 | 1 | 0.0 | 0.0 | 0.00023671 | 0.00021212 | 0.0 | -inf | 0.14611 |
| CAG00889_Megasp haera -elsdenii_ 14_14 | > | 0 | 0.0 | 11.1 | 0.1055 | -0.5819 | 0.6681 | **0.7102** | 1.6361 | 1 | 1 | 0.0 | 0.0 | 0.00147009 | 0.00087685 | 0.0 | -inf | 0.0708 |

EP 3 629 023 A1

**Table 28:** Statistics for the species selected in Model 11.2.

| Group | op. | Thr | NR (n=29) (%) | R (n=27) (%) | Pval_Fisher | NR_coeff | NR_Odds Ratio | R_coeff | R_Odds Ratio | rank_RFECV | rank_RFE | NR Mean relabund | NR Sem | R Mean relabund | R Sem | Fold Ratio (FR) R/NR | log2FR R/NR | P value NR vs R |
|---|---|---|---|---|---|---|---|---|---|---|---|---|---|---|---|---|---|---|
| CAG00346_Eubacterium_rectale_M104_1 | > | 0 | 10.3 | 0.0 | 0.2373 | 0.9631 | 1.9495 | **-0.8463** | 0.5562 | 1 | 1 | 2.084e-05 | 1.172e-05 | 0.0 | 0.0 | 0.0 | -inf | 0.09282 |
| CAG00530_Prevotella_sp__CAG_617 | > | 0 | 10.3 | 29.6 | 0.0965 | -1.2447 | 0.422 | **1.13** | 2.1886 | 1 | 1 | 0.00032714 | 0.0002189 | 0.00163373 | 0.00059881 | 4.99 | 2.32 | 0.06874 |
| CAG00601_uncultured_Faecalibacterium_sp_ | > | 0 | 79.3 | 74.1 | 0.7555 | 1.0027 | 2.0038 | **-1.183** | 0.4404 | 1 | 1 | 0.00595075 | 0.00106984 | 0.00227143 | 0.00053733 | 0.38 | -1.39 | 0.01999 |
| CAG00607_Eubacterium_sp__CAG_251 | > | 0 | 37.9 | 33.3 | 0.7849 | 1.181 | 2.2673 | **-0.9544** | 0.5161 | 1 | 1 | 0.00265039 | 0.00097373 | 0.00066297 | 0.00028011 | 0.25 | -2.0 | 0.37879 |
| CAG00646_Alistipes_sp__CAG_268 | > | 0 | 13.8 | 25.9 | 0.3224 | -0.7539 | 0.593 | **0.8929** | 1.8569 | 1 | 1 | 0.00020628 | 0.00010205 | 0.00156952 | 0.00064613 | 7.61 | 2.93 | 0.17786 |
| CAG00713_Firmicutes_bacterium_CAG_270 | > | 0 | 6.9 | 33.3 | 0.0184 | -0.9526 | 0.5167 | **1.0919** | 2.1316 | 1 | 1 | 2.89e-06 | 2.09e-06 | 0.00021793 | 8.762e-05 | 75.46 | 6.24 | 0.0087 |

EP 3 629 023 A1

| Group | op. | Thr | NR (n=29) (%) | R (n=27) (%) | Pval_ Fisher | NR_coeff | NR_ Odds Ratio | R_coeff | R_ Odds Ratio | rank_ RFECV | rank_ RFE | NR Mean relabund | NR Sem | R Mean relabund | R Sem | Fold Ratio (FR) R/NR | log2FR R/NR | P value NR vs R |
|---|---|---|---|---|---|---|---|---|---|---|---|---|---|---|---|---|---|---|
| CAG00919_Clostridium_ methylpentosum_ DSM-5476_ | > | 0 | 17.2 | 33.3 | 0.2214 | -0.7227 | 0.606 | **0.4881** | 1.4026 | 1 | 1 | 1.004e-05 | 4.17e-06 | 7.683e-05 | 3.389e-05 | 7.65 | 2.94 | 0.16102 |
| CAG01158_Pseudoflavonifractor _capillosus_ATCC_ 29799 | > | 0 | 3.4 | 29.6 | 0.0104 | -0.9687 | 0.511 | **1.1606** | 2.2355 | 1 | 1 | 6.3e-06 | 6.3e-06 | 0.00024491 | 9.486e-05 | 38.88 | 5.28 | 0.00656 |

**Table 29:** Statistics for the species selected in Model 12.2.

| Group | op. | Thr | NR (n=29) (%) | R (n=27) (%) | Pval_ Fisher | NR_ coeff | NR Odds Ratio | R_coeff | R_ Odds Ratio | rank_ RFECV | rank_ RFE | NR Mean relabund | NR Sem | R Mean relabund | R Sem | Fold Ratio (FR) R/NR | log2FR R/NR | P value NR vs R |
|---|---|---|---|---|---|---|---|---|---|---|---|---|---|---|---|---|---|---|
| CAG00142_Bacteroides_ stercoris_ ATCC_ 43183 | > | 0 | 48.3 | 37.0 | 0.4303 | **1.138** | 2.2007 | -0.8803 | 0.5433 | 1 | 1 | 0.01293929 | 0.00368976 | 0.003047 | 0.00114758 | 0.24 | -2.09 | 0.14334 |
| CAG00218_Bacteroides_sp__ CAG_20 | > | 0 | 89.7 | 81.5 | 0.462 | **1.1396** | 2.2031 | -0.8724 | 0.5462 | 1 | 1 | 0.01321164 | 0.00169549 | 0.00941315 | 0.00154558 | 0.71 | -0.49 | 0.07752 |
| CAG00341_Eubacterium_sp __CAG_ 115 | > | 0 | 13.8 | 44.4 | 0.0174 | **-1.0166** | 0.4943 | 0.6772 | 1.599 | 1 | 1 | 0.00031187 | 0.00027514 | 0.00289445 | 0.00124963 | 9.28 | 3.21 | 0.00822 |
| CAG00474_Sutterella_ wadsworthensis_ 2_1_59BFAA | > | 0 | 6.9 | 25.9 | 0.0733 | **-0.8403** | 0.5585 | 0.8566 | 1.8108 | 1 | 1 | 0.00020181 | 0.00015501 | 0.00215856 | 0.00095824 | 10.7 | 3.42 | 0.04832 |
| CAG00508_A!istipes_ obesi | > | 0 | 72.4 | 92.6 | 0.0798 | **-0.7067** | 0.6127 | 0.6586 | 1.5785 | 1 | 1 | 0.00262606 | 0.00069393 | 0.00471117 | 0.0009454 | 1.79 | 0.84 | 0.08724 |
| CAG00880_Subdoligranulum _sp_ CAG_314 | > | 0 | 13.8 | 33.3 | 0.1165 | **-1.185** | 0.4398 | 1.0027 | 2.0038 | 1 | 1 | 0.00036879 | 0.00020934 | 0.00400279 | 0.00171271 | 10.85 | 3.44 | 0.05113 |

EP 3 629 023 A1

| Group | op. | Thr | NR (n=29) (%) | R (n=27) (%) | Pval_ Fisher | **NR_ coeff** | NR Odds Ratio | R_coeff | R_ Odds Ratio | rank_ RFECV | rank_ RFE | NR Mean relabund | NR Sem | R Mean relabund | R Sem | Fold Ratio (FR) R/NR | log2FR R/NR | P value NR vs R |
|---|---|---|---|---|---|---|---|---|---|---|---|---|---|---|---|---|---|---|
| CAG01263_Clostridium_ clostridioforme_ 2_1_49FAA | > | 0 | 86.2 | 74.1 | 0.3224 | **0.8466** | 1.7982 | -0.5783 | 0.6698 | 1 | 1 | 0.00425323 | 0.00240791 | 0.0003713 | 0.00011117 | 0.09 | -3.52 | 0.05414 |
| CAG01321_Faecali bacterium _prausnitz ii_SL3_3 | > | 0 | 79.3 | 92.6 | 0.2544 | **-0.8895** | 0.5398 | 0.9053 | 1.8729 | 1 | 1 | 0.00465506 | 0.00103762 | 0.01086215 | 0.00270038 | 2.33 | 1.22 | 0.07615 |

EP 3 629 023 A1

Taking into account the bacterial species (n = 183, above 1083 total species, 16.9% of the total microbial composition) having an average relative abundance greater than or equal to 0.001 if expressed in the interval [0 : 1] (thus, the 0.1%) within the overall RCC cohort, we applied the second strategy (machine-learning) for 56 RCC patients (NR = 29; R = 27) with no antibiotic usage history and OUTCOME_2 criterion. After a first round of feature selection, RFECV gave a rank = 1 to 20 species which collectively, after a 5-fold cross-validation, were able to predict 78% of NR and 67% of R subjects, with a CV_AUC = 0.833 and an AUC = 0.852 (model 12.1). After a second round of feature selection, RFE gave a rank = 1 to 8 species which were selected for the subsequent logistic regression classifier. The model built on these 8 species gave an overall predictability of 89.66% (model 12.2, Figure 8), and the summary statistics for these species are reported in Table 29. In order to measure the goodness of the model, after a 5-fold cross-validation performed on a subset of the original dataset, the model itself was able to predict 78% of NR and 50% of R subjects, with a CV_AUC = 0.889 and an AUC = 0.611. This last model was designed to be more affordable in case of problems in fecal samples storage, sequencing, stiffness, etc.

According to the Models 10.2, 11.2 and 12.2, which were modeled on no antibiotic usage history and OUTCOME_2 criterion, and were all based on 8 bacterial species in order to be properly compared, it is shown that the Model 11.2 has the highest discriminatory power among NR and R, with good predictability (91.38%), and excellent CV_AUC and AUC.

**Example 11: Predictive models for RCC patients with no antibiotic usage, OUTCOME 2 criterion**

According to the results obtained in Example 10, and due to the fact that responders (R) are predicted better than non-responders (NR) in models 10.2 and 11.2, while is the opposite for model 12.2, and that all models were proposed with 8 species (Figure 7) to make them comparable, we took into consideration the logistic regression $\beta$ coefficients relative to R for models 10.2 and 11.2, while the logistic regression $\beta$ coefficients relative to NR for model 12.2.

Regarding the Model 10.2, and according to the $\beta$ coefficients of R (R_coeff), the probability estimate (expressed as a percentage) of belonging to R cohort for a tested subject JD is calculated as follows:

$$P_{R\,(JD)} = 1 / [1 + \exp\textasciicircum{-}(-0.1456 * CAG00580 +$$
$$-0.5496 * CAG00013 +$$
$$-0.4646 * CAG00873 +$$
$$-0.5496 * CAG01141 +$$
$$0.5684 * CAG00668 +$$
$$0.5129 * CAG00669 +$$
$$0.6623 * CAG00886 +$$
$$0.7102 * CAG00889)] \qquad [12]$$

In equation [12], "CAGXXXXX" represents the relative abundance of the bacterial species defined by the recited CAG, in a stool sample from the tested subject JD. These relative abundances need to be expressed as standardized values (zero mean and unit variance). The best option is to standardize the "abundances pattern" of the tested subject with a bundle of known "reference patterns", and the present invention refers a claim of a Python script used to ease the computation of the $P_R$ (JD). From the standardized abundance pattern of a tested subject, the equation [12] can be applied to predict his/her belonging to the R cohort with a model predictability of 68.97% and a success rate of 100%.

Regarding the Model 11.2, and according to the $\beta$ coefficients of R (R_coeff), the probability estimate (expressed as a percentage) of belonging to R cohort for a tested subject JD is calculated as follows:

$$P_{R\,(JD)} = 1 / [1 + \exp^{\wedge}-(-0.8463 * CAG00346 +$$
$$1.13 * CAG00530 +$$
$$-1.183 * CAG00601 +$$
$$-0.9544 * CAG00607 +$$
$$0.8929 * CAG00646 +$$
$$1.0919 * CAG00713 +$$
$$0.4881 * CAG00919 +$$
$$1.1606 * CAG01158)] \qquad [13]$$

In equation [13], "CAGXXXXX" represents the relative abundance of the bacterial species defined by the recited CAG, in a stool sample from the tested subject JD. These relative abundances need to be expressed as standardized values (zero mean and unit variance). The best option is to standardize the "abundances pattern" of the tested subject with a bundle of known "reference patterns", and the present invention refers a claim of a Python script used to ease the computation of the $P_R$ (JD). From the standardized abundance pattern of a tested subject, the equation [13] can be applied to predict his/her belonging to the R cohort with a model predictability of 91.38% and a success rate of 100%.

Regarding the Model 12.2, and according to the $\beta$ coefficients of NR (NR_coeff), the probability estimate (expressed as a percentage) of belonging to NR cohort for a tested subject JD is calculated as follows:

$$P_{NR\,(JD)} = 1 / [1 + \exp^{\wedge}-(1.138 * CAG00142 +$$
$$1.1396 * CAG00218 +$$
$$-1.0166 * CAG00341 +$$
$$-0.8403 * CAG00474 +$$
$$-0.7067 * CAG00508 +$$
$$-1.185 * CAG00880 +$$
$$0.8466 * CAG01263 +$$
$$-0.8895 * CAG01321)] \qquad [14]$$

In equation [14], "CAGXXXXX" represents the relative abundance of the bacterial species defined by the recited CAG, in a stool sample from the tested subject JD. These relative abundances need to be expressed as standardized values (zero mean and unit variance). The best option is to standardize the "abundances pattern" of the tested subject with a bundle of known "reference patterns", and the present invention refers a claim of a Python script used to ease the computation of the $P_{NR}$ (JD). From the standardized abundance pattern of a tested subject, the equation [14] can be applied to predict his/her belonging to the NR cohort with a model predictability of 89.66% and a success rate of 78%.

**Discussion**

The method described above relies on the following positive features:

(i) it is possible to use different NGS sequencing platforms (454, Illumina, IonTorrent, Nanopore, PacBio) and molecular biology techniques (qPCR, microarray, ...)
(ii) the expression of the relative abundances of model-selected bacterial species within the closed interval [0 : 1].
(iii) if the tested subject does not have one or more selected species belonging to the chosen model, this model retains a high predictability due to its additive nature and the proposed double strategy.
(iv) each one of both strategies is split into two different models, one which is limited to individuals who did not take antibiotics during the past two months, while the other one considers the eventuality of an unknown antibiotic regimen/treatment status.

According to the statistical method used to properly fit the "abundances pattern" of a definite subject into one of the proposed models, as described in Materials and Methods, we propose the following steps:

(i) a fecal sample taken from the subject undergoes DNA extraction and shotgun sequencing with the preferred NGS platform (as described above), and relative abundances within the closed interval [0 : 1] are measured through the bioinformatic pipeline described in Materials and Methods.
(ii) the predictability score (P) for the subject JD, which represents the probability estimate (expressed as a percentage) of belonging to a definite cohort (NR or R) is defined as follows:

$$P = 1 / (1 + \exp^{\wedge}{-z}) \qquad [1]$$

and

$$z = \beta 1 * x1 + \ldots + \beta n * xn \qquad [2]$$

where z is the log-odds ratio (expressing the natural logarithm of the ratio between the probability that an event will occur to the probability that it will not occur), *exp* is the exponent in natural base, each $\beta$ is the regression coefficient pre-computed according to the invention, and each *x* is the relative abundance of selected bacterial species expressed within the closed interval [0 : 1], obligatory expressed as standardized data (mean removed and unit variance scaled);
(iii) the *P* score obtained in step (ii) is interpreted as clinically meaningful if it is higher than 75% (fixed threshold) and/or it is concordant among the different Models used, always taking into account the antibiotic usage (known or unknown).

In the above method, the regression coefficients $\beta$ have to be calculated by logistic regression classifier with the following requirements: a constant (also known as intercept) not added to the decision function; a max iteration equal to 10000; a liblinear solver (with L2 penalization). All these parameters for logistic regression are explained and described thoroughly in section 1.1.11. of Sci-Kit Learn library guide.

OUTCOME_2 means one considers only SD>6 months among responders.

Based on the results disclosed herein, we conclude the following notions:

1/ GOMS ("*gut oncomicrobiome signatures*) can definitely predict good clinical outcome during PD-1 blockade with AUC>0.9, and decent specificity and sensitivity in RCC.
2/ Some clinical criteria contrasting R versus NR can be impacted by the composition of the gut oncomicrobiome.
3/ The GOMS of R and NR are by and large the same ones when taking into consideration the 'human-driven' strategy, and they can predict differently the NR or R status based on logistic regression coefficients.
4/ Clinical significance relies on an equilibrium between "favorable" and "unfavorable" commensals, detectable in higher abundance in R feces or NR feces, respectively
5/ Some species withstand ATB, mainly members of Firmicutes phylum.
6/ Our best predicting minimal ecosystem (called "GOMS") on the whole population of RCC 2L is based on few MGS (unfavorable *Prevotella spp., Faecalibcaterium spp., Coprococcus catus, Eggerthella lenta, Clostridium bolteae* versus favorable *Sutterella spp., Akkermansia, Alistipes spp.*), as evidenced by pairwise analysis and logistic regression models.
7/ Of note, some favorable commensals are part of the differences observed between healthy versus cancer individuals (overexpressed bacteria in HV (vs cancer pts) are also associated with R phenotype in anti-PD1 treated patients)

In the present text, we proposed different GOMS (*gut oncomicrobiome signatures*) to predict the NR or R status of a definite subject suffering from RCC depending, before an ICI treatment, on two main criteria: i) the presence of an antibiotic treatment/usage within the last two months; ii) the RECIST definition of outcome (OUTCOME_1 or OUTCOME_2). As a first step, we evidenced how GOMS are able to differentiate among NR and R at OUTCOME_1, TTP3 and TTP6, with this latter showing significant alfa- and beta-diversity in the first cohort reported in Routy et al Science 2018. Mean AUC values (among NR and R) for the best descriptors (single bacterial species or consortia) based on ROC curves showed the higher prediction value at TTP3: regardless antibiotic usage we have Mean $\text{AUC}_{\text{OUTCOME1}}$ = 0.872, Mean $\text{AUC}_{\text{TTP3}}$ = 0.894, Mean $\text{AUC}_{\text{TTP6}}$ = 0.840, while with no antibiotic treatment we have Mean $\text{AUC}_{\text{OUTCOME1}}$ = 0.862, Mean $\text{AUC}_{\text{TTP3}}$ = 0.891, Mean $\text{AUC}_{\text{TTP6}}$ = 0.762. From these observations, it seems that in RCC the antibiotic pressure (at least within the two months before ICI treatment) seems to enhance predictability of the GOMS signature especially at TTP6 (+10%), while leaving unaltered the one at OUTCOME1 and TTP3.

In the above study, we deeply analyzed by means of Volcano, LEfSe and pairwise analysis the bacterial signature of NR and R in patients regardless antibiotic usage and of those who did not take antibiotics during the two months preceeding ICI treatment. We found that from the original signature at TTP6 no bacterial species resisted ATB among NR, while 18% resisted ATB among responders (Figure 3). All these species could be good predictors for a RCC patient with unknown antibiotic regimen. Different logistic regression models are proposed herein, based on the aforementioned GOMS, antibiotic usage, and OUTCOME status following RECIST criteria. Upon a double strategy (as described in Materials and Methods) and after an internal feature selection and 5-fold cross-validation we made six models (Model 1.2, Model 2.2, Model 3.2, Model 7.2, Model 8.2, Model 9.2) for RCC patients regardless ATB, while we made other six models (Model 4.2, Model 5.2, Model 6.2, Model 10.2, Model 11.2, Model 12.2) for RCC patients without ATB treatment (see Table 30). From a first survey, after logistic regression models, it seems that ATB worsens by around 4% the overall model predictability for both OUTCOMEs: in fact, we have Mean Pred.$_{OUTCOME1\_regardlessATB}$ = 85.02%, Mean Pred.$_{OUTCOME1\_noATB}$ = 88.50%, Mean Pred.$_{OUTCOME2\_regardlessATB}$ = 79.83%, and finally Mean Pred.$_{OUTCOME2\_noATB}$ = 83.34%. From these results OUTCOME_1 criterion seems to a best predictor than OUTCOME_2, at least for the overall predictability. Notably, only around 50% of species were in common among models without and regardless antibiotic usage, mainly belonging to Firmicutes phylum (genera *Faecalibacterium, Holdemanella, Subdoligranulum* for OUTCOME_1 and OUTCOME_2, while genus *Eubacterium* only for OUTCOME_2). Members of Proteobacteria phylum (genera *Sutterella* and *Azospirillum*) withstanding antibiotic treatment were found only with models generated with OUTCOME_2.

| Bacterial species | outcome 1 | | | | | | outcome 2 | | | | | |
|---|---|---|---|---|---|---|---|---|---|---|---|---|
| | ± Ab | | | No Ab | | | ± Ab | | | No Ab | | |
| | 1.2 | 2.2 | 3.2 | 4.2 | 5.2 | 6.2 | 7.2 | 8.2 | 9.2 | 10.2 | 11.2 | 12.2 |
| CAG00008_Clostridium_bolteae_ATCC_BAA_613 | X | | | X | | X | | | | | | |
| CAG00013_Clostridiales_bacterium_1_7_47FAA | | | | | | | X | | | X | | |
| CAG00037_Bacteroides_faecis_MAJ27 | | | X | | | | | | | | | |
| CAG00048_1_Clostridium_sp__CAG_226 | | | | | X | | | | | | | |
| CAG00063_Barnesiella_viscericola_DSM_18177 | | X | | | | | | | | | | |
| CAG00122_Coprococcus_catus_GD_7 | | X | | | | | | | | | | |
| CAG00140_Subdoligranulum_sp__4_3_54A2FAA | X | | X | X | | | | | | | | |
| CAG00142_Bacteroides_stercoris_ATCC_43183 | | | | | | | | | | | | X |
| CAG00211_Firmicutes_bacterium_CAG_227 | | | | | | | | X | | | | |
| CAG00218_Bacteroides_sp__CAG_20 | | | | | | | | | | | | X |
| CAG00243_Lachnospiraceae_bacterium_1_1_57FAA | | | X | | | X | | | | | | |
| CAG00300_Prevotella_sp__CAG_891 | | | | | X | | | | | | | |
| CAG00317_Clostridium_sp__CAG_230 | X | | | | | | | | | | | |
| CAG00327_Faecalibacterium_sp__CAG_74 | | | X | | | X | | | | | | |
| CAG00341_Eubacterium_sp__CAG_115 | | | | | | | | | | | | X |
| CAG00346_Eubacterium_rectale_M104_1 | | | | | | | | | | | X | |
| CAG00357_Bacteroides_ovatus_V975 | | | X | | | | | | | | | |
| CAG00413_Bacteroides_sp__CAG_144 | X | | | X | | | | | | | | |
| CAG00473_Prevotella_sp__CAG_617 | X | X | X | X | X | X | | | | | | |
| CAG00474_Sutterella_wadsworthensis_2_1_59BFAA | | | | | | | | X | | | | X |
| CAG00487_Prevotella_sp__CAG_279 | | | | X | | | | | | | | |
| CAG00508_Alistipes_obesi | | | | | | | | | | | | X |
| CAG00530_Prevotella_sp__CAG_617 | | | | | | | | | | | X | |
| CAG00557_Ruminococcus_callidus_ATCC_27760 | | | | | | | | | X | | | |
| CAG00580_Clostridium_sp__CAG_62 | | | | | | | | | | X | | |
| CAG00601_uncultured_Faecalibacterium_sp_ | | | | | | | | | X | | X | |
| CAG00607_Eubacterium_sp__CAG_251 | | | | | | | | | X | | X | |
| CAG00610_Hungatella_hathewayi_12489931 | X | | | | | | | | | | | |
| CAG00624_Firmicutes_bacterium_CAG_552 | | | | | | | | X | | | | |
| CAG00646_Alistipes_sp__CAG_268 | | | | | | | | | | | X | |
| CAG00650_Dorea_formicigenerans_ATCC_27755 | | X | | | X | | | X | | | | |
| CAG00668_Azospirillum_sp__CAG_239 | | | | | | | X | | | X | | |
| CAG00669_Firmicutes_bacterium_CAG_103 | | | | | | | X | | X | X | | |
| CAG00676_Firmicutes_bacterium_CAG_176 | | | | | | | | X | | | | |
| CAG00698_Ruminococcus_sp__CAG_177 | | | | | | X | | | | | | |
| CAG00713_Firmicutes_bacterium_CAG_270 | | | | | | | | | | | X | |
| CAG00720_Anaerotruncus_colihominis_DSM_17241 | | | | | X | | | | | | | |
| CAG00727_Eggerthella_lenta_DSM_2243 | | X | | | X | | | | | | | |
| CAG00766_Firmicutes_bacterium_CAG_176 | | | | | | X | | | | | | |
| CAG00771_Clostridium_sp__CAG_413 | | | | | | | | X | | | | |
| CAG00782_Eubacterium_rectale_CAG_36 | | | | | | | X | | | | | |
| CAG00861_Oscillibacter_sp__CAG_241 | | | | | | | | | X | | | |
| CAG00873_Butyricimonas_virosa_DSM_23226 | | | | | | | X | | | X | | |
| CAG00880_Subdoligranulum_sp__CAG_314 | | | | | | | | | X | | | X |
| CAG00886_Sutterella_sp__CAG_351 | | | | | | | X | | | X | | |
| CAG00889_Megasphaera_elsdenii_14_14 | | | | | | | X | | | X | | |
| CAG00897_Firmicutes_bacterium_CAG_83 | | | | | | X | | | | | | |
| CAG00919_Clostridium_methylpentosum_DSM_5476 | | | | | | | | | | | X | |
| CAG00928_Acidiphilium_sp__CAG_727 | | X | | | | | | | | | | |
| CAG00937_Clostridium_sp__CAG_7 | | | | | | | | | X | | | |
| CAG00963_Clostridium_sp__CAG_524 | | | | | X | | | | | | | |
| CAG01039_Faecalibacterium_cf__prausnitzii_KLE1255 | X | | X | X | | X | | | | | | |
| CAG01141_Holdemanella_biformis_DSM_3989 | X | X | | X | X | | X | | | X | | |
| CAG01144_Lactobacillus_vaginalis_DSM_5837 | | X | | | | | | | | | | |
| CAG01158_Pseudoflavonifractor_capillosus | | | | | | | | | | | X | |
| CAG01197_Dialister_succinatiphilus_YIT_11850 | | | | | | | | X | | | | |
| CAG01208_Coprococcus_catus_GD_7 | | | | X | | | | | | | | |
| CAG01263_Clostridium_clostridioforme_2_1_49FAA | | X | | | | | | | | | | X |
| CAG01321_Faecalibacterium_prausnitzii_SL3_3 | | | | | | | | X | X | | | X |

**Table 30:** Bacterial species in each model. "Good bacteria" are highlighted in grey.

**Table 31:** Specific sequences for each bacterial species.

| Bacterial species | Specific sequences |
|---|---|
| CAG00008_Clostridium bolteae_ ATCC_ BAA_ 613 | SEQ ID NO: 1-50 |
| CAG00013_Clostridiales bacterium_ 1_7_47FAA | SEQ ID NO: 51-100 |
| CAG00037_ Bacteroides_ faecis_ MAJ27 | SEQ ID NO: 101-150 |
| CAG00048_1_Clostridium_sp__CAG_226 | SEQ ID NO: 2001-2050 |
| CAG00063_ Barnesiella_ viscericola_DSM_18177 | SEQ ID NO: 151-200 |
| CAG00122_Coprococcus_catus_GD_7 | SEQ ID NO: 401-450 |
| CAG00140_Subdoligranulum_sp__4_3_54A2FAA | SEQ ID NO: 451-500 |
| CAG00142_ Bacteroides_ stercoris_ATCC_43183 | SEQ ID NO: 501-550 |
| CAG00211_ Firmicutes_bacterium_ CAG_227 | SEQ ID NO: 601-650 |
| CAG00218_ Bacteroides_ sp__CAG_20 | SEQ ID NO: 651-700 |
| CAG00243_ Lachnospiraceae_bacterium_ _1_1_57FAA | SEQ ID NO: 551-600 |
| CAG00300_ Prevotella_ sp__CAG_891 | SEQ ID NO: 701-750 |
| CAG00317_Clostridium_sp__CAG_230 | SEQ ID NO: 751-800 |
| CAG00327_ Faecalibacterium_ sp__CAG_74 | SEQ ID NO: 801-850 |
| CAG00341_ Eubacterium_ sp__CAG_115 | SEQ ID NO: 851-900 |
| CAG00346_ Eubacterium_rectale_M104_ 1 | SEQ ID NO: 901-950 |
| CAG00357_ Bacteroides_ ovatus_ V975 | SEQ ID NO: 951-1000 |
| CAG00413_ Bacteroides_ sp__CAG_144 | SEQ ID NO: 1001-1050 |
| CAG00473_ Prevotella_ sp__CAG_617 | SEQ ID NO: 1051-1100 |
| CAG00474_Sutterella_wadsworthensis_2_1_59BFAA | SEQ ID NO: 1101-1150 |
| CAG00487_ Prevotella_ sp__CAG_279 | SEQ ID NO: 351-400 |
| CAG00508_Alistipes_obesi | SEQ ID NO: 1151-1200 |
| CAG00530_ Prevotella_ sp__CAG_617 | SEQ ID NO: 1201-1250 |
| CAG00557_ Ruminococcus_ callidus_ATCC_27760 | SEQ ID NO: 1251-1300 |
| CAG00580_Clostridium_sp__CAG_62 | SEQ ID NO: 301-350 |
| CAG00601_ uncultured_Faecalibacterium_ sp_ | SEQ ID NO: 201-250 |
| CAG00607_ Eubacterium_ sp__CAG_251 | SEQ ID NO: 1301-1350 |
| CAG00610_ Hungatella_hathewayi_ 12489931 | SEQ ID NO: 1351-1400 |
| CAG00624_ Firmicutes_bacterium_ CAG_552 | SEQ ID NO: 1401-1450 |
| CAG00646_Alistipes_sp__CAG 268 | SEQ ID NO: 1451-1500 |
| CAG00650_Dorea_formicigenerans_ATCC_27755 | SEQ ID NO: 1501-1550 |
| CAG00668_Azospirillum_sp__CAG_239 | SEQ ID NO: 1551-1600 |
| CAG00669_ Firmicutes_bacterium_ CAG_103 | SEQ ID NO: 1601-1650 |
| CAG00676_ Firmicutes_bacterium_ CAG_176 | SEQ ID NO: 1651-1700 |
| CAG00698_ Ruminococcus_ sp__CAG_177 | SEQ ID NO: 1701-1750 |
| CAG00713_ Firmicutes_bacterium_ CAG_270 | SEQ ID NO: 1751-1800 |
| CAG00720_Anaerotruncus_colihominis_DSM_17241 | SEQ ID NO: 1801-1850 |

(continued)

| Bacterial species | Specific sequences |
|---|---|
| CAG00727_ Eggerthella_ lenta_DSM_2243 | SEQ ID NO: 1851-1900 |
| CAG00766_ Firmicutes_bacterium_ CAG_176 | SEQ ID NO: 251-300 |
| CAG00771_Clostridium_sp__CAG 413 | SEQ ID NO: 1901-1950 |
| CAG00782_ Eubacterium_rectale_ CAG_36 | SEQ ID NO: 1951-2000 |
| CAG00861_Oscillibacter_sp__CAG_241 | SEQ ID NO: 2051-2100 |
| CAG00873_ Butyricimonas_virosa_ DSM_23226 | SEQ ID NO: 2101-2150 |
| CAG00880_Subdoligranulum_sp__CAG_314 | SEQ ID NO: 2151-2200 |
| CAG00886_Sutterella_sp__CAG_351 | SEQ ID NO: 2201-2250 |
| CAG00889_ Megasphaera_ elsdenii_14_14 | SEQ ID NO: 2251-2300 |
| CAG00897_ Firmicutes_bacterium_ CAG_83 | SEQ ID NO: 2301-2350 |
| CAG00919_Clostridium_methylpentosum_DSM_5476 | SEQ ID NO: 2351-2400 |
| CAG00928_Acidiphilium_sp__CAG_727 | SEQ ID NO: 2401-2450 |
| CAG00937_Clostridium_sp__CAG_7 | SEQ ID NO: 2451-2500 |
| CAG00963_Clostridium_sp__CAG_524 | SEQ ID NO: 2501-2550 |
| CAG01039_ Faecalibacterium_ cf__ prausnitzii_KLE1255 | SEQ ID NO: 2551-2600 |
| CAG01141_ Holdemanella_biformis_ DSM_3989 | SEQ ID NO: 2601-2650 |
| CAG01144_ Lactobacillus_ vaginalis_DSM_5837 | SEQ ID NO: 2651-2700 |
| CAG01158_ Pseudoflavonifractor_ capillosus | SEQ ID NO: 2701-2750 |
| CAG01197_Dialister_succinatiphilus_YIT_11850 | SEQ ID NO: 2751-2800 |
| CAG01208_Coprococcus_catus_GD_7 | SEQ ID NO: 2801-2850 |
| CAG01263_Clostridium_clostridioforme_2_1_49FAA | SEQ ID NO: 2851-2900 |
| CAG01321_Faecalibacterium_prausnitzii_SL3_3 | SEQ ID NO: 2901-2950 |

## REFERENCES

Borghaei, H., Paz-Ares, L., Horn, L., Spigel, D.R., Steins, M., Ready, N.E., Chow, L.Q., Vokes, E.E., Felip, E., Holgado, E., et al. (2015). Nivolumab versus Docetaxel in Advanced Nonsquamous Non-Small-Cell Lung Cancer. N. Engl. J. Med. 373, 1627-1639.

Gao, J., Shi, L.Z., Zhao, H., Chen, J., Xiong, L., He, Q., Chen, T., Roszik, J., Bernatchez, C., Woodman, S.E., et al. (2016). Loss of IFN-γ Pathway Genes in Tumor Cells as a Mechanism of Resistance to Anti-CTLA-4 Therapy. Cell 167, 397-404.e9.

Koyama, S., Akbay, E.A., Li, Y.Y., Herter-Sprie, G.S., Buczkowski, K.A., Richards, W.G., Gandhi, L., Redig, A.J., Rodig, S.J., Asahina, H., et al. (2016). Adaptive resistance to therapeutic PD-1 blockade is associated with upregulation of alternative immune checkpoints. Nat. Commun. 7, 10501.

Li J.1, Jia H., Cai X., Zhong H., Feng Q., Sunagawa S., Arumugam M., Kultima J.R., Prifti E., Nielsen T. et al. (2014). An integrated catalog of reference genes in the human gut microbiome. Nat Biotechnol. 2014 Aug;32(8):834-41.

Nghiem, P.T., Bhatia, S., Lipson, E.J., Kudchadkar, R.R., Miller, N.J., Annamalai, L., Berry, S., Chartash, E.K., Daud, A., Fling, S.P., et al. (2016). PD-1 Blockade with Pembrolizumab in Advanced Merkel-Cell Carcinoma. N. Engl. J. Med. 374, 2542-2552.

Pardoll, D. (2015). Cancer and the Immune System: Basic Concepts and Targets for Intervention. Semin. Oncol. 42, 523-538.

Pardoll, D.M. (2012). The blockade of immune checkpoints in cancer immunotherapy. Nat. Rev. Cancer 12, 252-264.

Restifo, N.P., Dudley, M.E., and Rosenberg, S.A. (2012). Adoptive immunotherapy for cancer: harnessing the T cell response. Nat. Rev. Immunol. 12, 269-281.

Riaz, N., Havel, J.J., Kendall, S.M., Makarov, V., Walsh, L.A., Desrichard, A., Weinhold, N., and Chan, T.A. (2016). Recurrent SERPINB3 and SERPINB4 mutations in patients who respond to anti-CTLA4 immunotherapy. Nat. Genet. 48, 1327-1329.

Ribas, A., Hamid, O., Daud, A., Hodi, F.S., Wolchok, J.D., Kefford, R., Joshua, A.M., Patnaik, A., Hwu, W.-J., Weber, J.S., et al. (2016). Association of Pembrolizumab With Tumor Response and Survival Among Patients With Advanced Melanoma. JAMA 315, 1600-1609.

Rizvi, N.A., Hellmann, M.D., Snyder, A., Kvistborg, P., Makarov, V., Havel, J.J., Lee, W., Yuan, J., Wong, P., Ho, T.S., et al. (2015). Cancer immunology. Mutational landscape determines sensitivity to PD-1 blockade in non-small cell lung cancer. Science 348,124-128.

Robert, C., Thomas, L., Bondarenko, I., O'Day, S., Weber, J., Garbe, C., Lebbe, C., Baurain, J.-F., Testori, A., Grob, J.-J., et al. (2011). Ipilimumab plus dacarbazine for previously untreated metastatic melanoma. N. Engl. J. Med. 364, 2517-2526.

Rosenberg, J.E., Hoffman-Censits, J., Powles, T., van der Heijden, M.S., Balar, A.V., Necchi, A., Dawson, N., O'Donnell, P.H., Balmanoukian, A., Loriot, Y., et al. (2016). Atezolizumab in patients with locally advanced and metastatic urothelial carcinoma who have progressed following treatment with platinum-based chemotherapy: a single-arm, multicentre, phase 2 trial. Lancet Lond. Engl. 387, 1909-1920.

Sharma, P., and Allison, J.P. (2015a). Immune checkpoint targeting in cancer therapy: toward combination strategies with curative potential. Cell 161, 205-214.

Sharma, P., and Allison, J.P. (2015b). The future of immune checkpoint therapy. Science 348, 56-61.

Smyth, M.J., Ngiow, S.F., Ribas, A., and Teng, M.W.L. (2016). Combination cancer immunotherapies tailored to the tumour microenvironment. Nat. Rev. Clin. Oncol. 13, 143-158.

Spranger, S., Bao, R., and Gajewski, T.F. (2015). Melanoma-intrinsic $\beta$-catenin signalling prevents anti-tumour immunity. Nature 523, 231-235.

Taube, J.M., Anders, R.A., Young, G.D., Xu, H., Sharma, R., McMiller, T.L., Chen, S., Klein, A.P., Pardoll, D.M., Topalian, S.L., et al. (2012). Colocalization of inflammatory response with B7-h1 expression in human melanocytic lesions supports an adaptive resistance mechanism of immune escape. Sci. Transl. Med. 4, 127ra37.

Topalian, S.L., Hodi, F.S., Brahmer, J.R., Gettinger, S.N., Smith, D.C., McDermott, D.F., Powderly, J.D., Carvajal, R.D., Sosman, J.A., Atkins, M.B., et al. (2012). Safety, activity, and immune correlates of anti-PD-1 antibody in cancer. N. Engl. J. Med. 366, 2443-2454.

Zaretsky, J.M., Garcia-Diaz, A., Shin, D.S., Escuin-Ordinas, H., Hugo, W., Hu-Lieskovan, S., Torrejon, D.Y., Abril-Rodriguez, G., Sandoval, S., Barthly, L., et al. (2016). Mutations Associated with Acquired Resistance to PD-1 Blockade in Melanoma. N. Engl. J. Med. 375, 819-829.

Zitvogel, L., Tesniere, A., and Kroemer, G. (2006). Cancer despite immunosurveillance: immunoselection and immunosubversion. Nat. Rev. Immunol. 6, 715-727.X

**Claims**

1. A method for *in vitro* determining if an individual having a renal cell cancer (RCC) is likely to respond to a treatment with an immune checkpoint inhibitor (ICI)-based therapy, comprising the following steps:

   (i) from a fecal sample of said individual, obtaining an abundances pattern based on the relative abundances of a set of bacterial species comprising at least 8 bacterial species selected from the group consisting of:

| CAG number | Bacterial species |
|---|---|
| CAG00008 | Clostridium_bolteae_ATCC_BAA_613 |
| CAG00013 | Clostridiales_bacterium_1_7_47FAA |
| CAG00037 | Bacteroides_faecis_MAJ27 |
| CAG00048_1 | Clostridium_sp__CAG_226 |
| CAG00063 | Barnesiella_viscericola_DSM_18177 |
| CAG00122 | Coprococcus_catus_GD_7 |
| CAG00140 | Subdoligranulum_sp__4_3_54A2FAA |
| CAG00142 | Bacteroides_stercoris_ATCC_43183 |
| CAG00211 | Firmicutes_bacterium_CAG_227 |
| CAG00218 | Bacteroides_sp__CAG_20 |
| CAG00243 | Lachnospiraceae_bacterium_1_1_57FAA |
| CAG00300 | Prevotella_sp__CAG_891 |
| CAG00317 | Clostridium_sp__CAG_230 |
| CAG00327 | Faecalibacterium_sp__CAG_74 |
| CAG00341 | Eubacterium_sp__CAG_115 |
| CAG00346 | Eubacterium_rectale_M104_1 |
| CAG00357 | Bacteroides_ovatus_V975 |
| CAG00413 | Bacteroides_sp__CAG_144 |
| CAG00473 | Prevotella_sp__CAG_617 |
| CAG00474 | Sutterella_wadsworthensis_2_1_59BFAA |
| CAG00487 | Prevotella_sp__CAG_279 |
| CAG00508 | Alistipes_obesi |
| CAG00530 | Prevotella_sp__CAG_617 |
| CAG00557 | Ruminococcus_callidus_ATCC_27760 |
| CAG00580 | Clostridium_sp__CAG_62 |
| CAG00601 | uncultured_Faecalibacterium_sp_ |
| CAG00607 | Eubacterium_sp__CAG_251 |
| CAG00610 | Hungatella_hathewayi_12489931 |
| CAG00624 | Firmicutes_bacterium_CAG_552 |
| CAG00646 | Alistipes_sp__CAG_268 |
| CAG00650 | Dorea_formicigenerans_ATCC_27755 |
| CAG00668 | Azospirillum_sp__CAG_239 |
| CAG00669 | Firmicutes_bacterium_CAG_103 |

(continued)

| CAG number | Bacterial species |
|---|---|
| CAG00676 | Firmicutes_bacterium_CAG_176 |
| CAG00698 | Ruminococcus_sp__CAG_177 |
| CAG00713 | Firmicutes_bacterium_CAG_270 |
| CAG00720 | Anaerotruncus_colihominis_DSM_17241 |
| CAG00727 | Eggerthella_lenta_DSM_2243 |
| CAG00766 | Firmicutes_bacterium_CAG_176 |
| CAG00771 | Clostridium_sp__CAG_413 |
| CAG00782 | Eubacterium_rectale_CAG_36 |
| CAG00861 | Oscillibacter_sp__CAG_241 |
| CAG00873 | Butyricimonas_virosa_DSM_23226 |
| CAG00880 | Subdoligranulum_sp__CAG_314 |
| CAG00886 | Sutterella_sp__CAG_351 |
| CAG00889 | Megasphaera_elsdenii_14_14 |
| CAG00897 | Firmicutes_bacterium_CAG_83 |
| CAG00919 | Clostridium_methylpentosum_DSM_5476 |
| CAG00928 | Acidiphilium_sp__CAG_727 |
| CAG00937 | Clostridium_sp__CAG_7 |
| CAG00963 | Clostridium_sp__CAG_524 |
| CAG01039 | Faecalibacterium_cf_prausnitzii_KLE1255 |
| CAG01141 | Holdemanella_biformis_DSM_3989 |
| CAG01144 | Lactobacillus_vaginalis_DSM_5837_ATCC_49540 |
| CAG01158 | Pseudoflavonifractor_capillosus_ATCC_29799 |
| CAG01197 | Dialister_succinatiphilus_YIT_11850 |
| CAG01208 | Coprococcus_catus_GD_7 |
| CAG01263 | Clostridium_clostridioforme_2_1_49FAA |
| CAG01321 | Faecalibacterium_prausnitzii_SL3_3 |

(ii) using one or several pre-defined equations each corresponding to a model obtained for at least 8 bacterial species from said set of bacterial species, calculating the probability that said individual responds to the treatment ($P_R$) or the probability that said individual resists to the treatment ($P_{NR}$) with an ICI-based therapy.

2. The method of claim 1, wherein in step (ii), at least one equation corresponds to a model for a set of at least 8 bacterial species which are present in the fecal sample of said individual.

3. The method of claim 1 or claim 2, wherein the model(s) used in step (ii) lead to an individual probability percentage of at least 75% and/or to a predictability of at least 65% and a success rate of at least 70%.

4. The method of any of claims 1 to 3, which further comprises a step (iii) of assessing, in an animal model, whether the individual is likely to be a good responder to the treatment with an ICI-based therapy, wherein step (iii) comprises (iiia) performing a fecal microbial transplantation (FMT) of feces from the individual into a germ free (GF) model animal; (iiib) at least 7 to 14 days after step (iiia), inoculating said animal with a transplantable tumor model; (iiic) treating the inoculated animal with the ICI-based therapy; and (iiid) measuring the tumor size in the treated animals, wherein the results of step (iiid) are illustrative of the response that can be expected for said individual to said treatment.

5. The method of any of claims 1 to 4, wherein the individual's antibiotic regimen exposure during the last two months is unknown and wherein, in step (i), the set of bacterial species comprises at least 8 bacterial species selected from the group consisting of:

| CAG number | Bacterial species |
|---|---|
| CAG00008 | Clostridium_bolteae_ATCC_BAA_613 |
| CAG00037 | Bacteroides_faecis_MAJ27 |
| CAG00063 | Barnesiella_ viscericola_DSM_18177 |
| CAG00122 | Coprococcus_catus_GD_7 |
| CAG00140 | Subdoligranulum_sp__4_3_54A2FAA |
| CAG00243 | Lachnospiraceae_bacterium_1_1_57FAA |
| CAG00317 | Clostridium_sp__CAG_230 |
| CAG00327 | Faecalibacterium_sp__CAG_74 |
| CAG00357 | Bacteroides_ovatus_V975 |
| CAG00413 | Bacteroides_sp__CAG_144 |
| CAG00473 | Prevotella_sp__CAG_617 |
| CAG00610 | Hungatella_hathewayi_12489931 |
| CAG00650 | Dorea_formicigenerans_ATCC_27755 |
| CAG00727 | Eggerthella_lenta_DSM_2243 |
| CAG00928 | Acidiphilium_sp__CAG_727 |
| CAG01039 | Faecalibacterium_cf_prausnitzii_KLE1255 |
| CAG01141 | Holdemanella_biformis_DSM_3989 |
| CAG01144 | Lactobacillus_vaginalis_DSM_5837_ATCC_49540 |
| CAG01263 | Clostridium_clostridioforme_2_1_49FAA |

6. The method of claim 5, wherein in step (ii), one, two or three equations are used, which correspond to models obtained for the following sets of bacterial species, identified by their CAG numbers:

| set for model 1.2 | set for model 2.2 | set for model 3.2 |
|---|---|---|
| CAG00008 | CAG00727 | CAG00243 |
| CAG01039 | CAG01141 | CAG00473 |
| CAG00473 | CAG00650 | CAG00327 |
| CAG00140 | CAG00928 | CAG01039 |
| CAG00610 | CAG00473 | CAG00140 |
| CAG01141 | CAG00122 | CAG01263 |
| CAG00413 | CAG01144 | CAG00037 |
| CAG00317 | CAG00063 | CAG00357 |

and wherein preferably at least one equation used corresponds to a model obtained with a set of bacteria which are all present in the individual's sample.

7. The method of claim 6, wherein the equations for calculating the probability that said individual responds to the treatment ($P_R$) are as follows:

$$[B] \quad P_R = 1 / [1 + \exp\text{\textasciicircum}-(\sum_{j=1}^{8} \beta_j X_j)]$$

wherein $X_j$ ($j$ = 1 to 8) are the relative abundances of the bacterial species measured in the individual's sample and $\beta_j$ ($j$ = 1 to 8) are the following regression coefficients:

| Model 1.2 | | Model 2.2 | | Model 3.2 | |
|---|---|---|---|---|---|
| bacterial species | R coefficients | bacterial species | R coefficients | bacterial species | R coefficients |
| CAG00008 | -1.0323 | CAG00727 | -1.0791 | CAG00243 | -1.1735 |
| CAG01039 | -1.084 | CAG01141 | -1.1164 | CAG00473 | -1.027 |
| CAG00473 | -0.9723 | CAG00650 | -1.2839 | CAG00327 | -0.9968 |
| CAG00140 | -0.9907 | CAG00928 | -1.1156 | CAG01039 | -1.0043 |
| CAG00610 | -0.6234 | CAG00473 | -1.014 | CAG00140 | -0.7456 |
| CAG01141 | -0.8468 | CAG00122 | -0.9394 | CAG01263 | -0.8736 |
| CAG00413 | 0.8994 | CAG01144 | -0.8085 | CAG00037 | 0.7032 |
| CAG00317 | 0.6041 | CAG00063 | -0.7679 | CAG00357 | -0.6073 |

8. The method of any of claims 1 to 4, wherein the individual did not take any antibiotic during the last two months and wherein, in step (i), the set of bacterial species comprises at least 8 bacterial species selected from the group consisting of:

| CAG number | Bacterial species |
|---|---|
| CAG00008 | Clostridium_bolteae_ATCC_BAA_613 |
| CAG00048_1 | Clostridium_sp__CAG_226 |
| CAG00140 | Subdoligranulum_sp__4_3_54A2FAA |
| CAG00243 | Lachnospiraceae_bacterium_1_1_57FAA |
| CAG00300 | Prevotella_sp__CAG_891 |
| CAG00327 | Faecalibacterium_sp__CAG_74 |
| CAG00413 | Bacteroides_sp__CAG_144 |
| CAG00473 | Prevotella_sp__CAG_617 |
| CAG00487 | Prevotella_sp__CAG_279 |
| CAG00650 | Dorea_formicigenerans_ATCC_27755 |
| CAG00698 | Ruminococcus_sp__CAG_177 |
| CAG00720 | Anaerotruncus_colihominis_DSM_17241 |
| CAG00727 | Eggerthella_lenta_DSM_2243 |
| CAG00766 | Firmicutes_bacterium_CAG_176 |
| CAG00897 | Firmicutes_bacterium_CAG_83 |
| CAG00963 | Clostridium_sp__CAG_524 |

(continued)

| CAG number | Bacterial species |
|---|---|
| CAG01039 | Faecalibacterium_cf_prausnitzii_KLE1255 |
| CAG01141 | Holdemanella_biformis_DSM_3989 |
| CAG01208 | Coprococcus_catus_GD_7 |

9. The method of claim 8, wherein in step (ii), one, two or three equations are used, which correspond to models obtained for the following sets of bacterial species, identified by their CAG numbers:

| set for model 4.2 | set for model 5.2 | set for model 6.2 |
|---|---|---|
| CAG00008 | CAG00048_1 | CAG00243 |
| CAG01039 | CAG00300 | CAG00008 |
| CAG00473 | CAG00473 | CAG00698 |
| CAG00487 | CAG00650 | CAG00473 |
| CAG00140 | CAG00720 | CAG00327 |
| CAG01141 | CAG00727 | CAG01039 |
| CAG01208 | CAG00963 | CAG00897 |
| CAG00413 | CAG01141 | CAG00766 |

and wherein preferably at least one equation used corresponds to a model obtained with a set of bacteria which are all present in the individual's sample.

10. The method of claim 9, wherein the equations for calculating the probability that said individual resists ($P_{NR}$) or responds ($P_R$) to the treatment are as follows:

$$[A]\ P_{NR} = 1 / [1 + exp^{\wedge}-(\sum_{j=1}^{8} \beta_j X_j)]\ \text{for models 4.2 and 5.2}$$

$$[B]\ P_R = 1 / [1 + exp^{\wedge}-(\sum_{j=1}^{8} \beta_j X_j)]\ \text{for model 6.2}$$

wherein $X_j$ ($j$ = 1 to 8) are the relative abundances of the bacterial species measured in the individual's sample and $\beta_j$ ($j$ = 1 to 8) are the following regression coefficients:

| Model 4.2 | | Model 5.2 | | Model 6.2 | |
|---|---|---|---|---|---|
| bacterial species | NR coefficients | bacterial species | NR coefficients | bacterial species | R coefficients |
| CAG00008 | 0.955 | CAG00048_1 | 0.7112 | CAG00243 | -1.2907 |
| CAG01039 | 0.8761 | CAG00300 | 0.8931 | CAG00008 | -0.8848 |
| CAG00473 | 0.9951 | CAG00473 | 1.0042 | CAG00698 | -0.9776 |
| CAG00487 | 0.7609 | CAG00650 | 1.1282 | CAG00473 | -0.9997 |
| CAG00140 | 1.1107 | CAG00720 | 1.2569 | CAG00327 | -1.0697 |
| CAG01141 | 0.9402 | CAG00727 | 1.2397 | CAG01039 | -0.7806 |

(continued)

| Model 4.2 | | Model 5.2 | | Model 6.2 | |
|---|---|---|---|---|---|
| bacterial species | NR coefficients | bacterial species | NR coefficients | bacterial species | R coefficients |
| CAG01208 | 0.7187 | CAG00963 | 0.7595 | CAG00897 | -0.8376 |
| CAG00413 | -0.5812 | CAG01141 | 1.0235 | CAG00766 | -0.7459 |

**11.** The method of any of claims 1 to 4, for determining if the individual is likely to have a long-term benefit from a treatment with an ICI-based therapy, wherein the individual's antibiotic regimen exposure during the last two months is unknown and wherein, in step (i), the set of bacterial species comprises at least 8 bacterial species selected from the group consisting of:

| CAG number | Bacterial species |
|---|---|
| CAG00013 | Clostridiales_bacterium_1_7_47FAA |
| CAG00211 | Firmicutes_bacterium_CAG_227 |
| CAG00474 | Sutterella_wadsworthensis_2_1_59BFAA |
| CAG00557 | Ruminococcus_callidus_ATCC_27760 |
| CAG00601 | uncultured_Faecalibacterium_sp_ |
| CAG00607 | Eubacterium_sp__CAG_251 |
| CAG00624 | Firmicutes_bacterium_CAG_552 |
| CAG00650 | Dorea_formicigenerans_ATCC_27755 |
| CAG00668 | Azospirillum_sp__CAG_239 |
| CAG00669 | Firmicutes_bacterium_CAG_103 |
| CAG00676 | Firmicutes_bacterium_CAG_176 |
| CAG00771 | Clostridium_sp__CAG_413 |
| CAG00782 | Eubacterium_rectale_CAG_36 |
| CAG00861 | Oscillibacter_sp__CAG_241 |
| CAG00873 | Butyricimonas_virosa_DSM_23226 |
| CAG00880 | Subdoligranulum_sp__CAG_314 |
| CAG00886 | Sutterella_sp__CAG_351 |
| CAG00889 | Megasphaera_elsdenii_14_14 |
| CAG00937 | Clostridium_sp__CAG_7 |
| CAG01141 | Holdemanella_biformis_DSM_3989 |
| CAG01197 | Dialister_succinatiphilus_YIT_11850 |
| CAG01321 | Faecalibacterium_prausnitzii_SL3_3 |

**12.** The method of claim 11, wherein in step (ii), one, two or three equations are used, which correspond to models obtained for the following sets of bacterial species, identified by their CAG numbers:

| set for model 7.2 | set for model 8.2 | set for model 9.2 |
|---|---|---|
| CAG00782 | CAG00211 | CAG00557 |
| CAG00013 | CAG00474 | CAG00601 |
| CAG00873 | CAG00624 | CAG00607 |

(continued)

| set for model 7.2 | set for model 8.2 | set for model 9.2 |
|---|---|---|
| CAG01141 | CAG00650 | CAG00669 |
| CAG00668 | CAG00676 | CAG00861 |
| CAG00669 | CAG00771 | CAG00880 |
| CAG00886 | CAG01197 | CAG00937 |
| CAG00889 | CAG01321 | CAG01321 |

and wherein preferably at least one equation used corresponds to a model obtained with a set of bacteria which are all present in the individual's sample.

13. The method of claim 12, wherein the equations for calculating the probability that said individual resists ($P_{NR}$) to the treatment are as follows:

$$[A] \quad P_{NR} = 1 / [1 + \exp{\textasciicircum}{-}(\sum_{j=1}^{8} \beta_j X_j)]$$

wherein $X_j$ ($j$ = 1 to 8) are the relative abundances of the bacterial species measured in the individual's sample and $\beta_j$ ($j$ = 1 to 8) are the following regression coefficients:

| Model 7.2 | | Model 8.2 | | Model 9.2 | |
|---|---|---|---|---|---|
| bacterial species | NR coefficients | bacterial species | NR coefficients | bacterial species | NR coefficients |
| CAG00782 | -0.2034 | CAG00211 | 0.6422 | CAG00557 | 0.9341 |
| CAG00013 | 0.4092 | CAG00474 | -1.0581 | CAG00601 | 0.8927 |
| CAG00873 | 0.2708 | CAG00624 | -0.8984 | CAG00607 | 0.7108 |
| CAG01141 | 0.7665 | CAG00650 | 1.3189 | CAG00669 | -1.2341 |
| CAG00668 | -0.5899 | CAG00676 | -0.7324 | CAG00861 | 1.0379 |
| CAG00669 | -0.5181 | CAG00771 | -0.6623 | CAG00880 | -1.0016 |
| CAG00886 | -0.8415 | CAG01197 | -1.1372 | CAG00937 | -0.872 |
| CAG00889 | -0.7339 | CAG01321 | -0.8525 | CAG01321 | -0.8362 |

14. The method of any of claims 1 to 4, for determining if the individual is likely to have a long-term benefit from a treatment with an ICI-based therapy, wherein the individual did not take any antibiotic during the last two months and wherein, in step (i), the set of bacterial species comprises at least 8 bacterial species selected from the group consisting of:

| CAG number | Bacterial species |
|---|---|
| CAG00013 | Clostridiales_bacterium_1_7_47FAA |
| CAG00142 | Bacteroides_stercoris_ATCC_43183 |
| CAG00218 | Bacteroides_sp__CAG_20 |
| CAG00341 | Eubacterium_sp__CAG_115 |
| CAG00346 | Eubacterium_rectale_M104_1 |

(continued)

| CAG number | Bacterial species |
|---|---|
| CAG00474 | Sutterella_wadsworthensis_2_1_59BFAA |
| CAG00508 | Alistipes_obesi |
| CAG00530 | Prevotella_sp__CAG_617 |
| CAG00580 | Clostridium_sp__CAG_62 |
| CAG00601 | uncultured_Faecalibacterium_sp_ |
| CAG00607 | Eubacterium_sp__CAG_251 |
| CAG00646 | Alistipes_sp__CAG_268 |
| CAG00668 | Azospirillum_sp__CAG_239 |
| CAG00669 | Firmicutes_bacterium_CAG_103 |
| CAG00713 | Firmicutes_bacterium_CAG_270 |
| CAG00873 | Butyricimonas_virosa_DSM_23226 |
| CAG00880 | Subdoligranulum_sp__CAG_314 |
| CAG00886 | Sutterella_sp__CAG_351 |
| CAG00889 | Megasphaera_elsdenii_14_14 |
| CAG00919 | Clostridium_methylpentosum_DSM_5476 |
| CAG01141 | Holdemanella_biformis_DSM_3989 |
| CAG01158 | Pseudoflavonifractor_capillosus_ATCC_29799 |
| CAG01263 | Clostridium_clostridioforme_2_1_49FAA |
| CAG01321 | Faecalibacterium_prausnitzii_SL3_3 |

15. The method of claim 14, wherein in step (ii), one, two or three equations are used, which correspond to models obtained for the following sets of bacterial species, identified by their CAG numbers:

| set for model 10.2 | set for model 11.2 | set for model 12.2 |
|---|---|---|
| CAG00580 | CAG00346 | CAG00142 |
| CAG00013 | CAG00530 | CAG00218 |
| CAG00873 | CAG00601 | CAG00341 |
| CAG01141 | CAG00607 | CAG00474 |
| CAG00668 | CAG00646 | CAG00508 |
| CAG00669 | CAG00713 | CAG00880 |
| CAG00886 | CAG00919 | CAG01263 |
| CAG00889 | CAG01158 | CAG01321 |

and wherein preferably at least one equation used corresponds to a model obtained with a set of bacteria which are all present in the individual's sample.

16. The method of claim 15, wherein the equations for calculating the probability that said individual resists to the treatment ($P_{NR}$) or responds ($P_R$) are as follows:

$$[A] \quad P_{NR} = 1 / [1 + exp^\wedge - (\sum_{j=1}^{8} \beta_j X_j)] \text{ for model 12.2}$$

$$[B] \quad P_{R} = 1 / [1 + exp^\wedge - (\sum_{j=1}^{8} \beta_j X_j)] \text{ for models 10.2 and 11.2}$$

wherein $X_j$ ($j$ = 1 to 8) are the relative abundances of the bacterial species measured in the individual's sample and $\beta_j$ ($j$ = 1 to 8) are the following regression coefficients:

| Model 10.2 | | Model 11.2 | | Model 12.2 | |
|---|---|---|---|---|---|
| bacterial species | R coefficients | bacterial species | R coefficients | bacterial species | NR coefficients |
| CAG00580 | -0.1456 | CAG00346 | -0.8463 | CAG00142 | 1.138 |
| CAG00013 | -0.5496 | CAG00530 | 1.13 | CAG00218 | 1.1396 |
| CAG00873 | -0.4646 | CAG00601 | -1.183 | CAG00341 | -1.0166 |
| CAG01141 | -0.5496 | CAG00607 | -0.9544 | CAG00474 | -0.8403 |
| CAG00668 | 0.5684 | CAG00646 | 0.8929 | CAG00508 | -0.7067 |
| CAG00669 | 0.5129 | CAG00713 | 1.0919 | CAG00880 | -1.185 |
| CAG00886 | 0.6623 | CAG00919 | 0.4881 | CAG01263 | 0.8466 |
| CAG00889 | 0.7102 | CAG01158 | 1.1606 | CAG01321 | -0.8895 |

17. The method of any of claims 1 to 16, wherein the fecal sample is obtained before the first administration of ICI.

18. The method of any of claims 1 to 17, wherein the ICI-based therapy is a treatment with an anti-PD1 antibody, an anti-PD-L1 antibody and/or an anti-PD-L2 antibody.

19. The method of claim 18, wherein the ICI-based therapy is a treatment with an anti-PD1 antibody.

20. A theranostic method for determining if a cancer patient needs a bacterial compensation before administration of an ICI-based therapy and/or during such a therapy, comprising assessing, by a method according to any of claims 1 to 19, whether the patient is likely to be a good responder to such a therapy, wherein if the patient is not identified as likely to be a good responder, the patient needs a bacterial compensation.

21. A nucleic acid microarray designed to perform the method according to any of claims 1 to 19, **characterized in that** it comprises nucleic acid probes specific for each of the microorganism species to be detected in said method.

22. A set of primers for performing the method according to any of claims 1 to 19, **characterized in that** it comprises primer pairs for amplifying sequences specific for each of the microorganism species to be detected in said method.

Figure 1A

Figure 1B

Figure 1C

Figure 1D

Figure 2A

Figure 2B

Figure 2C

Figure 2D

Lefse plot INRA_DF_ScExt_KIDNEY - TTP6 -

NR
R

Oscillibacter_sp___KLE_1745_6 -
Eubacterium_sp___CAG_115 -
Azospirillum_sp___CAG_239_3 -
Clostridium_sp___CAG_167 -
Clostridium_sp___CAG_302 -
Firmicutes_bacterium_CAG_176_12 -
Clostridium_sp___CAG_230 -

LDA score
0  1  2  3

Volcano plot INRA_DF_ScExt_KIDNEY - TTP6 -

Not Sig
Pvalue<5.0%

Eubacterium_sp__CAG_115
Clostridium_sp__CAG_230
Lactococcus_lactis_subsp_lactis_KLDS_4_0325
Dorea_sp__CAG_105
Clostridium_sp__CAG_245
Clostridium_sp__CAG_167
Clostridium_sp__CAG_302
Bacteroides_sp__CAG_598
Firmicutes_bacterium_CAG_552
Clostridium_sp__CAG_1024_3
Eggerthella_sp_CAG_298
Oscillibacter_sp_KLE_1745_6
Ruminococcus_torques_L2_14
Lachnospiraceae_bacterium_VE202_23_2

R
NR

$log_2(FoldChange)$
$-log_{10}(Pvalue)$

TTP6

Figure 3A

92

Figure 3B

Figure 3C

Figure 3D

Figure 4

Figure 4 (follow-up)

Figure 4 (follow-up)

Model 1.2
(8 species – **Pred. 79.71%**)

Optimal # of features : 8

$P_{R\,(JD)}$ = 1 / [1 + exp^−(-1.0323 * CAG00008 -1.084 * CAG01039 -0.9723 * CAG00473 -0.9907 * CAG00140 -0.6234 * CAG00610 -0.8468 * CAG01141 +0.8994 * CAG00413 +0.6041 * CAG00317)]

Model 2.2
(8 species – **Pred. 89.86%**)

Optimal # of features : 8

$P_{R\,(JD)}$ = 1 / [1 + exp^−(-1.0791 * CAG00727 -1.1164 * CAG01141 -1.2839 * CAG00650 -1.1156 * CAG00928 -1.014 * CAG00473 -0.9394 * CAG00122 -0.8085 * CAG01144 -0.7679 * CAG00063)]

Figure 5

Model 3.2
(8 species – **Pred. 85.51%**)

Optimal # of features : 8

$P_{R (JD)} = 1 / [1 + \exp\verb|^|-(-1.1735 * CAG00243 -1.027 * CAG00473 -0.9968 * CAG00327 -1.0043 * CAG01039 -0.7456 * CAG00140 -0.8736 * CAG01263 +0.7032 * CAG00037 -0.6073 * CAG00357)]$

Figure 5 (follow-up)

Model 4.2 (8 species – **Pred. 87.93%**)

$P_{NR\,(JD)}$ = 1 / [1 + exp^−(0.955 * CAG00008 +0.8761 * CAG01039 +0.9951 * CAG00473 +0.7609 * CAG00487 + 1.1107 * CAG00140 +0.9402 * CAG01141 +0.7187 * CAG01208 -0.5812 * CAG00413)]

Model 5.2 (8 species – **Pred. 93.1%**)

$P_{NR\,(JD)}$ = 1 / [1 + exp^−(0.7112 * CAG00048 +0.8931 * CAG00300 +1.0042 * CAG00473 +1.1282 * CAG00650 + 1.2569 * CAG00720 +1.2397 * CAG00727 +0.7595 * CAG00963 +1.0235 * CAG01141)]

Figure 6

EP 3 629 023 A1

Model 6.2
(8 species – **Pred. 84.48%**)

PR (JD) =  1 / [1 + exp^−(-1.2907 * CAG00243 -0.8848 * CAG00008 -0.9776 * CAG00698 -0.9997 * CAG00473 -1.0697 * CAG00327 -0.7806 * CAG01039 -0.8376 * CAG00897 -0.7459 * CAG00766)]

Figure 6 (follow-up)

Model 7.2
(8 species – **Pred. 66.67%**)

Optimal # of features : 6

AUC: 0.704
CV_AUC: 0.481

$P_{NR\,(JD)}$ = 1 / [1 + exp^−(-0.2034 * CAG00782 +0.4092 * CAG00013 +0.2708 * CAG00873 +0.7665 * CAG01141 -0.5899 * CAG00668 -0.5181 * CAG00669 -0.8415 * CAG00886 -0.7339 * CAG00889)]

Model 8.2
(8 species – **Pred. 84.41%**)

Optimal # of features : 8

AUC: 0.926
CV_AUC: 0.667

$P_{NR\,(JD)}$ = 1 / [1 + exp^−(0.6422 * CAG00211 -1.0581 * CAG00474 -0.8984 * CAG00624 +1.3189 * CAG00650 -0.7324 * CAG00676 -0.6623 * CAG00771 -1.1372 * CAG01197 -0.8525 * CAG01321)]

Figure 7

Model 9.2
(8 species – **Pred. 88.41%**)

Optimal # of features : 8

| | NR | R |
|---|---|---|
| NR | 0.89 | 0.11 |
| R | 0.22 | 0.78 |

AUC: 0.864
CV_AUC: 0.481

$P_{NR\,(JD)}$ = 1 / [1 + exp^−(0.9341 * CAG00557 +0.8927 * CAG00601 +0.7108 * CAG00607 -1.2341 * CAG00669 + 1.0379 * CAG00861 -1.0016 * CAG00880 -0.872 * CAG00937 -0.8362 * CAG01321)]

Figure 7 (follow-up)

EP 3 629 023 A1

Model 10.2
(8 species – **Pred. 68.97%**)

Optimal # of features : 8

$P_{R\,(JD)}$ = 1 / [1 + exp^−(-0.1456 * CAG00580 -0.5496 * CAG00013 -0.4646 * CAG00873 -0.5496 * CAG01141 + 0.5684 * CAG00668 +0.5129 * CAG00669 +0.6623 * CAG00886 +0.7102 * CAG00889)]

AUC: 0.981
CV_AUC: 0.556

Model 11.2
(8 species – **Pred. 91.38%**)

Optimal # of features : 8

$P_{R\,(JD)}$ = 1 / [1 + exp^−(-0.8463 * CAG00346 +1.13 * CAG00530 -1.183 * CAG00601 -0.9544 * CAG00607 + 0.8929 * CAG00646 +1.0919 * CAG00713 +0.4881 * CAG00919 +1.1606 * CAG01158)]

AUC: 0.981
CV_AUC: 1.000

Figure 8

Model 12.2
(8 species – **Pred. 89.66%**)

Optimal # of features : 8

AUC: 0.611
CV_AUC: 0.889

$P_{NR\,(JD)}$ = 1 / [1 + exp^−(1.138 * CAG00142 +1.1396 * CAG00218 -1.0166 * CAG00341 -0.8403 * CAG00474 -0.7067 * CAG00508 -1.185 * CAG00880 +0.8466 * CAG01263 -0.8895 * CAG01321)]

Figure 8 (follow-up)

Europäisches
Patentamt

European
Patent Office

Office européen
des brevets

# EUROPEAN SEARCH REPORT

**Application Number**

EP 18 30 6282

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LISA DEROSA ET AL: "Gut Microbiome Composition to Predict Resistance in Renal Cell Carcinoma Patients on Nivolumab", ASCO 2018, 1 June 2018 (2018-06-01), XP055554785, * abstract * | 1-20 | INV. G01N33/569 C12Q1/689 G01N33/574 |
| X | V. GOPALAKRISHNAN ET AL: "Gut microbiome modulates response to anti-PD-1 immunotherapy in melanoma patients", SCIENCE, vol. 359, no. 6371, 5 January 2018 (2018-01-05), pages 97-103, XP055554925, US ISSN: 0036-8075, DOI: 10.1126/science.aan4236 | 21,22 | |
| Y | * suppl. information; figure S4 * | 1-20 | |
| X | BERTRAND ROUTY ET AL: "Gut microbiome influences efficacy of PD-1-based immunotherapy against epithelial tumors", SCIENCE, vol. 359, no. 6371, 5 January 2018 (2018-01-05), pages 91-97, XP055554928, US ISSN: 0036-8075, DOI: 10.1126/science.aan3706 | 21,22 | **TECHNICAL FIELDS SEARCHED (IPC)** G01N C12Q |
| Y | * suppl. information * | 1-20 | |

The present search report has been drawn up for all claims

| Place of search | Date of completion of the search | Examiner |
|---|---|---|
| The Hague | 12 February 2019 | Rosin, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

**Patent documents cited in the description**

- WO 2016063263 A

- WO 2018115519 A


**Non-patent literature cited in the description**

- **ROUTY et al.** *Science,* 05 January 2018
- **ROUTY et al.** *Science,* 2018
- **J. LI.** *Nat. Biotechnol.,* 2014, vol. 32, 834-841
- **BORGHAEI, H. ; PAZ-ARES, L. ; HORN, L. ; SPIGEL, D.R. ; STEINS, M. ; READY, N.E. ; CHOW, L.Q. ; VOKES, E.E. ; FELIP, E. ; HOLGADO, E. et al.** Nivolumab versus Docetaxel in Advanced Nonsquamous Non-Small-Cell Lung Cancer. *N. Engl. J. Med.,* 2015, vol. 373, 1627-1639
- **GAO, J. ; SHI, L.Z. ; ZHAO, H. ; CHEN, J. ; XIONG, L. ; HE, Q. ; CHEN, T. ; ROSZIK, J. ; BERNATCHEZ, C. ; WOODMAN, S.E. et al.** Loss of IFN-γ Pathway Genes in Tumor Cells as a Mechanism of Resistance to Anti-CTLA-4 Therapy. *Cell,* 2016, vol. 167, 397-404
- **KOYAMA, S. ; AKBAY, E.A. ; LI, Y.Y. ; HERTER-SPRIE, G.S. ; BUCZKOWSKI, K.A. ; RICHARDS, W.G. ; GANDHI, L. ; REDIG, A.J. ; RODIG, S.J. ; ASAHINA, H. et al.** Adaptive resistance to therapeutic PD-1 blockade is associated with upregulation of alternative immune checkpoints. *Nat. Commun.,* 2016, vol. 7, 10501
- **LI J.1 ; JIA H. ; CAI X. ; ZHONG H. ; FENG Q. ; SUNAGAWA S. ; ARUMUGAM M. ; KULTIMA J.R. ; PRIFTI E. ; NIELSEN T. et al.** An integrated catalog of reference genes in the human gut microbiome. *Nat Biotechnol.,* August 2014, vol. 32 (8), 834-41
- **NGHIEM, P.T. ; BHATIA, S. ; LIPSON, E.J. ; KUDCHADKAR, R.R. ; MILLER, N.J. ; ANNAMALAI, L. ; BERRY, S. ; CHARTASH, E.K. ; DAUD, A. ; FLING, S.P. et al.** PD-1 Blockade with Pembrolizumab in Advanced Merkel-Cell Carcinoma. *N. Engl. J. Med.,* 2016, vol. 374, 2542-2552
- **PARDOLL, D.** Cancer and the Immune System: Basic Concepts and Targets for Intervention. *Semin. Oncol.,* 2015, vol. 42, 523-538
- **PARDOLL, D.M.** The blockade of immune checkpoints in cancer immunotherapy. *Nat. Rev. Cancer,* 2012, vol. 12, 252-264
- **RESTIFO, N.P. ; DUDLEY, M.E. ; ROSENBERG, S.A.** Adoptive immunotherapy for cancer: harnessing the T cell response. *Nat. Rev. Immunol.,* 2012, vol. 12, 269-281

- **RIAZ, N. ; HAVEL, J.J. ; KENDALL, S.M. ; MAKAROV, V. ; WALSH, L.A. ; DESRICHARD, A. ; WEINHOLD, N. ; CHAN, T.A.** Recurrent SERPINB3 and SERPINB4 mutations in patients who respond to anti-CTLA4 immunotherapy. *Nat. Genet.,* 2016, vol. 48, 1327-1329
- **RIBAS, A. ; HAMID, O. ; DAUD, A. ; HODI, F.S. ; WOLCHOK, J.D. ; KEFFORD, R. ; JOSHUA, A.M. ; PATNAIK, A. ; HWU, W.-J. ; WEBER, J.S. et al.** JAMA. Association of Pembrolizumab With Tumor Response and Survival Among Patients With Advanced Melanoma, 2016, vol. 315, 1600-1609
- **RIZVI, N.A. ; HELLMANN, M.D. ; SNYDER, A. ; KVISTBORG, P. ; MAKAROV, V. ; HAVEL, J.J. ; LEE, W. ; YUAN, J. ; WONG, P. ; HO, T.S. et al.** Cancer immunology. Mutational landscape determines sensitivity to PD-1 blockade in non-small cell lung cancer. *Science,* 2015, vol. 348, 124-128
- **ROBERT, C. ; THOMAS, L. ; BONDARENKO, I. ; O'DAY, S. ; WEBER, J. ; GARBE, C. ; LEBBE, C. ; BAURAIN, J.-F. ; TESTORI, A. ; GROB, J.-J. et al.** Ipilimumab plus dacarbazine for previously untreated metastatic melanoma. *N. Engl. J. Med.,* 2011, vol. 364, 2517-2526
- **ROSENBERG, J.E. ; HOFFMAN-CENSITS, J. ; POWLES, T. ; VAN DER HEIJDEN, M.S. ; BALAR, A.V. ; NECCHI, A. ; DAWSON, N. ; O'DONNELL, P.H. ; BALMANOUKIAN, A. ; LORIOT, Y. et al.** Atezolizumab in patients with locally advanced and metastatic urothelial carcinoma who have progressed following treatment with platinum-based chemotherapy: a single-arm, multicentre, phase 2 trial. *Lancet Lond. Engl.,* 2016, vol. 387, 1909-1920
- **SHARMA, P. ; ALLISON, J.P.** Immune checkpoint targeting in cancer therapy: toward combination strategies with curative potential. *Cell,* 2015, vol. 161, 205-214
- **SHARMA, P. ; ALLISON, J.P.** The future of immune checkpoint therapy. *Science,* 2015, vol. 348, 56-61

- **SMYTH, M.J. ; NGIOW, S.F. ; RIBAS, A. ; TENG, M.W.L.** Combination cancer immunotherapies tailored to the tumour microenvironment. *Nat. Rev. Clin. Oncol.,* 2016, vol. 13, 143-158
- **SPRANGER, S. ; BAO, R. ; GAJEWSKI, T.F.** Melanoma-intrinsic β-catenin signalling prevents anti-tumour immunity. *Nature,* 2015, vol. 523, 231-235
- **TAUBE, J.M. ; ANDERS, R.A. ; YOUNG, G.D. ; XU, H. ; SHARMA, R. ; MCMILLER, T.L. ; CHEN, S. ; KLEIN, A.P. ; PARDOLL, D.M. ; TOPALIAN, S.L. et al.** Colocalization of inflammatory response with B7-h1 expression in human melanocytic lesions supports an adaptive resistance mechanism of immune escape. *Sci. Transl. Med.,* 2012, vol. 4, 127ra37
- **TOPALIAN, S.L. ; HODI, F.S. ; BRAHMER, J.R. ; GETTINGER, S.N. ; SMITH, D.C. ; MCDERMOTT, D.F. ; POWDERLY, J.D. ; CARVAJAL, R.D. ; SOSMAN, J.A. ; ATKINS, M.B. et al.** Safety, activity, and immune correlates of anti-PD-1 antibody in cancer. *N. Engl. J. Med.,* 2012, vol. 366, 2443-2454
- **ZARETSKY, J.M. ; GARCIA-DIAZ, A. ; SHIN, D.S. ; ESCUIN-ORDINAS, H. ; HUGO, W. ; HU-LIESKOVAN, S. ; TORREJON, D.Y. ; ABRIL-RODRIGUEZ, G. ; SANDOVAL, S. ; BARTHLY, L. et al.** Mutations Associated with Acquired Resistance to PD-1 Blockade in Melanoma. *N. Engl. J. Med.,* 2016, vol. 375, 819-829
- **ZITVOGEL, L. ; TESNIERE, A. ; KROEMER, G.** Cancer despite immunosurveillance: immunoselection and immunosubversion. *Nat. Rev. Immunol.,* 2006, vol. 6, 715-727